# EUROPEAN PATENT APPLICATION

(11) **EP 4 684 803 A1**
(43) Date of publication of application: **28.01.2026**
(21) Application number: 24191008.2
(22) Date of filing: 25.07.2024
(51) Int. Cl.: A61K 47/55, A61K 47/68, A61P 35/00

(54) **ANTIBODY CONJUGATED CHEMICAL INDUCERS OF DEGRADATION OF RBM39 AND THERAPEUTIC USES THEREOF**

(71) Applicant: CeMM - Forschungszentrum für Molekulare Medizin GmbH, 1090 Wien (AT); St. Anna Kinderkrebsforschung GmbH, 1090 Vienna (AT)
(72) Inventor: Boztug, Kaan, 1170 Vienna (AT); Winter, Georg, 1140 Vienna (AT); Konc, Juraj, 1180 Vienna (AT)
(74) Representative: Vossius & Partner Patentanwälte Rechtsanwälte mbB

(57) **Abstract**

The present invention relates to immunoconjugates comprising a) an antibody binding to a tumor antigen displayed on the surface of a cancer cell; in particular an antibody binding GD2 and b) a compound according to formula (I): 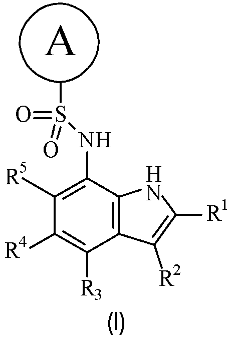 which is an RBM39 degrader. The instant immunoconjugates are particularly useful in treatment or prevention of cancer characterized by increased expression of RBM39, in particular cancer characterized by increased expression of GD2 or presence of GD2 antigen on the cell surface.

## Description

### Field of the invention

The present invention relates to immunoconjugates comprising a) an antibody binding to a tumor antigen displayed on the surface of a cancer cell; in particular an antibody binding GD2 disialoganglioside and b) a compound according to formula (I), which is an RBM39 degrader. The instant immunoconjugates are particularly useful in treatment or prevention of cancer characterized by increased expression of RBM39, in particular cancer characterized by increased expression of GD2 or presence of GD2 antigen on the cell surface.

### Background of the invention

Induction of targeted protein degradation (TPD) has recently emerged as a new therapeutic strategy that has expanded the druggable proteome for cancer treatment. TPD involves the use of small molecule degraders which hijack the endogenous ubiquitin-proteosome pathway and selectively induce degradation of a target protein of interest (POI). Unlike classic occupancy-based drugs, TPD eliminates the target and works catalytically, and so can be more efficacious and sustained, with potentially lower dose requirements.

Increasing amount of evidence shows that the pre-mRNA splicing factor RNA-binding motif protein 39 (RBM39) is overexpressed in several types of cancer and is as a proto-oncogene involved the tumor development and progression via regulation of tumor-related gene expression, alternative pre-mRNA splicing and protein translation.¹

Expression of RBM39 is upregulated in a panel of adult and pediatric malignancies and it has been shown that the loss of RBM39 disrupts spliceosome and induces significant genome-wide splicing errors which are lethal to several types of cancers, including neuroblastoma, Ewing sarcoma, osteosarcoma, rhabdomyosarcoma, melanoma, acute myeloid leukemia (AML), breast cancer, lung cancer, ovarian cancer or colorectal cancer.¹

Therefore, RBM39 has emerged as a promising therapeutic target for cancer treatment,¹ however its pharmacological targeting had been challenging until the recent discovery of a class of anti-cancer aryl sulfonamides such as indisulam (E7070), E7820, chloroquinoxaline sulfonamide (CQS) and tasisulam (LY-573636) which act as molecular glue degraders and selectively induce proteasomal degradation of RBM39 via recruitment of the DCAF15 E3 ubiquitin ligase, leading to cancer cell death and tumor regression.²³

Importantly, pre-mRNA splicing has been reported as a potential therapeutic vulnerability in MYC-driven cancers, such as MYC-driven high-risk neuroblastoma. Neuroblastoma is the most common extracranial childhood solid tumor characterized by one of the lowest survival rates among all pediatric malignancies. Amplification of the *MYCN* oncogene is present in about 25% of all neuroblastomas (~40% of high-risk neuroblastomas cases) and is an extremely adverse prognostic factor with -50% five-year overall survival rate.

The exceptional sensitivity towards TPD of RBM39 is the result of the combination of the dependency on RBM39-mediated RNA splicing and overexpression of the DCAF15, making RBM39 degradation a promising therapeutic strategy for high-risk neuroblastoma. Subsequently, the efficacy of some RBM39 small molecule degraders was successfully validated in *in vitro* and *in vivo* preclinical models of high-risk neuroblastoma.^{4,5}

Furthermore, high-risk neuroblastoma was the first pediatric cancer that received authorization for the clinical use of immunotherapy. The target of the antibody-based immunotherapy is GD2, a disialoganglioside tumor-associated antigen (TAA), that is present in high density on the surface of most of neuroblastoma tumor cells. Incorporation of anti-GD2 therapeutic antibodies, administered either alone or in combination with immunostimulatory agents, into standard of care treatment protocols has significantly improved survival rates of high-risk neuroblastoma patients.⁶ However, about 40% patients still do not respond or relapse after the treatment⁶⁻⁸ indicating that some of the neuroblastoma tumor cells might be able to escape anti-GD2 therapy. This suggests that efficacy of the anti-GD2 antibody-based immunotherapy needs to be improved so that more patients with high-risk neuroblastoma (including those with refractory or relapsed disease) can benefit from this treatment approach.

Various GD2-directed therapeutic approaches, e.g., chimeric antigen receptor (CAR) T, CAR-natural killer (NK) T cells or bispecific trifunctional antibodies (trAb) have been developed and underwent either preclinical or clinical testing. Anti-GD2 CAR T cells for the treatment of relapsed or refractory high-risk neuroblastoma were tested in a phase 1-2 academic trial (NCT03373097)⁹ but no significant increase in the 3-year overall survival rate (60%) or event-free survival rate (36%) was observed in comparison to the long-term, continuous single-agent dinutuximab beta therapy (NCT02743429)¹⁰ (66% and 31%, respectively) with cytokine release syndrome, a not negligible side-effect, occurring in 74% patients. CAR-NKT cells were also tested in a phase 1 trial for relapsed or refractory high-risk neuroblastoma (NCT03294954)¹¹ and 25% objective response rate was observed with this type of therapy which is comparable to the dinutuximab beta antibody (26%) therapy but lower compared to objective responses observed with anti-GD2 CAR T cell (63%) therapy. Recently, anti-GD2/anti-CD3 bispecific trifunctional antibodies (trAbs) have demonstrated reduction or complete elimination of neuroblastoma metastases in a syngeneic mouse model with superior anti-tumor activity compared to dinutuximab beta antibody,¹² however their efficacy has not yet been evaluated in a clinical trial.

One strategy to increase the efficacy of the antibody-based immunotherapy is through their chemical modification with small molecule cytotoxic drugs in a concept known as immunoconjugates or, in other words, antibody-drug conjugates (ADCs). Accordingly, ADCs have emerged as an effective anti-cancer biological drug class that combines the benefits of highly specific targeting ability and potent cytotoxic effect to achieve accurate and efficient killing of cancer cells. The therapeutic concept of ADCs uses an antibody, such as monoclonal antibody as a vehicle targeting a tumor-associated or -specific antigen to deliver highly potent (cytotoxic) payload selectively to the tumor cells, thus enhancing the therapeutic index and minimizing the unwanted side effect of anti-cancer agents. In addition, ADCs can also interact with both cancer and immune cells by eliciting mechanisms such as immunogenic cell death or antibody-dependent cell-mediated cytotoxicity (ADCC), ultimately providing potential synergism with immunotherapy which further increase the treatment efficacy. Importantly, ADC biotherapeutic platform has already shown proof-of-concept therapeutic potential for the treatment of various types of cancers with about half of the ADCs being currently clinically approved for the treatment of solid tumors.

Despite the recent addition of the anti-GD2 antibody-based immunotherapy¹³ to the standard of care for high-risk neuroblastoma and the strong clinical potential of ADCs which have demonstrated substantial improvement over available therapies and unmet medical needs for the treatment of solid tumors, the anti-GD2 ADC biotherapeutic platform remains surprisingly underexplored, especially in the context of high-risk neuroblastoma therapy.^{14,15} ADCs targeting different surface markers on neuroblastoma cells, e.g., ALK,¹⁶ GPC2,¹⁷⁻¹⁹ B7-H3²⁰ have been developed and *in vivo* preclinical studies provided rationale for their clinical development. However, therapeutic efficacy or potential side effects of any of these ADC constructs have so far not been evaluated in high-risk neuroblastoma clinical trials, pointing towards the need and space for new, more potent and safer strategies for targeting neuroblastoma tumors.

Document WO 2023/172968 discloses certain anti-GD2 antibody-based conjugates, in particular this document covers M3554, linking an exatecan payload with an anti-GD2 antibody. This potentially first-in-class ADC will enter its first-in-human study later in 2024. M3554 will be evaluated in patients with solid tumors with high GD2 prevalence, such as neuroblastoma, where GD2 has been validated as a target for a naked antibody therapy, as well as soft tissue sarcoma, glioblastoma, and osteosarcoma.

The reason for this might be the use of highly cytotoxic anti-cancer drug payloads, e.g., tubulin inhibitors, DNA damaging agents or DNA topoisomerase I inhibitors. Premature ADC payload release in the extratumoral compartments, non-specific endocytosis of the intact ADC, off-target receptor-mediated uptake of ADCs or on-target off-tumor toxicities due to the expression of the ADC target antigens in healthy tissue may lead to unacceptable toxicity profiles and premature termination of the clinical development of such ADC constructs. As an example, a higher incidence of peripheral neuropathy has been reported in ADCs containing monomethyl auristatin E (MMAE)²¹ which is a cytotoxic drug payload frequently used in the development of ADC biotherapeutics. A solution to avoid such unwanted and life-threatening toxicities could be the utilization of payloads anti-cancer activity and efficacy of which (unlike the above-mentioned inhibitors) does not solely depend on the expression and activity of the target POI, but also on a range of additional proteins, including E3 ligases and associated factors. Such requirements are fulfilled by molecular glue degraders which enable efficient tissue-specific removal of disease-causing proteins with decreased risk of off-target toxicity compared to conventional inhibitor drugs.

As it is the case for conventional cytotoxic ADC payloads, the cancer cell specificity, efficacy and safety profile of molecular glue degrader payloads can be potentially improved through the precise delivery to cancer cells using antibodies targeting TAAs. Merging the power of TPD with the precision of antibody-mediated delivery technology should overcome the limitations of each modality.

Moreover, it has been suggested that RBM39 plays an important role in cancer immunity.²² RBM39 expression could be associated with the less permissive tumor microenvironment and lower immune cell infiltration. In addition, it has also been shown that pharmacologic modulation of splicing via aryl sulfonamide RBM39 molecular glue degraders lead to the generation of novel neoantigens presented on tumor MHC class I and enhanced anti-tumor immunity.²³ This indicates a potential synergistic effect of TPD of RBM39 and antibody-based immunotherapy in provoking anti-tumor responses especially for so-called immunologically 'cold' tumors.

There is a range of unwanted side effects associated with both above mentioned therapeutic approaches, especially (but not only) allodynia, neuropathy and acute neuropathic pain. These unwanted side effects have been described especially in context of anti-GD2 monoclonal antibody (mAb) infusions. This issue limits the dosage and efficacy of the anti-GD2 immunotherapy for high-risk neuroblastoma. Furthermore, based on the collated results from Phase I and II clinical trials, the most common side effects reported for RBM39 degraders E7820 and indisulam (E7070) include fatigue, constipation, diarrhea, nausea, anemia, neutropenia and thrombocytopenia.

Accordingly, there is a need for novel therapeutic approaches, devoid of, or minimizing, these side effects.

### Summary of the invention

Particularly desirable are cancer therapies with high efficacy, at the same time causing limited side effects. It is further desirable to be able to target immunologically "cold" tumors, such as pediatric malignancies, including pediatric neuroblastoma.

Thus, it is a technical problem of the present invention to provide an improved therapy against cancer, in particular neuroblastoma, such as pediatric neuroblastoma.

The problem is solved by the embodiments provided herein and as characterized by the claims.

In particular, the invention provides immunoconjugates comprising the degraders of RBM39 oncoprotein. More specifically, this invention provides antibody-drug conjugates of increased potency, efficacy and safety of both targeted protein degradation of oncoprotein RBM39 and (anti-GD2) antibody-based immunotherapy through the precise antibody-mediated cancer cell-specific delivery of small molecule glue degraders of RBM39 RNA splicing factor in the form of RBM39. Whereas molecular glue degraders are typically small molecules that indiscriminately enter both healthy and diseased cells throughout the body, attachment of the degrader payloads to antibodies drives localization to specific sites and cells. In the form of immunoconjugates involving a degrader, or in other words degrader-antibody conjugates (DACs), such a biotherapeutic approach results in increased potency, improved pharmacokinetic and pharmacodynamic profiles and potentially reduced likelihood of systemic exposure and toxicity of small molecule degrader drugs.

Therefore, it was surprisingly found and demonstrated herein, that combining a (anti-GD2) mAb targeting a TAA present in high copy numbers on the surface of cancer cells with a potent non-neurotoxic molecular glue degrader payload targeting an oncoprotein RBM39 that is overexpressed in cancer cells, such as neuroblastoma cells (and expressed only in low levels in the healthy tissue) and required for the growth and survival of these cancer cells and anti-cancer activity of which is dependent on an E3 ligase DCAF15 that is also overexpressed in these particular cancer cells (and has only low expression in the healthy tissue), in an immunoconjugate, may more selectively deliver the cytotoxic payload to malignant cells (to kill/eliminate them) without triggering the unwanted toxicities. In particular, the peripheral neuropathies, as well as hematological toxicities, which otherwise limit dosing and therapeutic efficacy, could be avoided.

The invention is summarized in the following embodiments.

In a first embodiment, the present invention relates to an immunoconjugate comprising
a) an antibody binding to a tumor antigen displayed on the surface of a cancer cell; and
b) a compound according to formula (I): 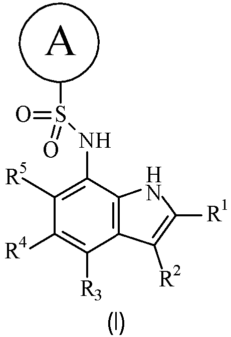 wherein
   R¹ is selected from -H, C₁₋₅ alkyl, -OH, -CN and -NO₂;
   R² is selected from -H, halogen, C₁₋₅ alkyl, C₁₋₅ haloalkyl, -OH, -CN, -NO₂, -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and - SO₂N(C₁₋₅ alkyl)(C₁₋₅ alkyl);
   R³ is selected from -H, halogen, C₁₋₅ alkyl, aryl, -OH, -O(C₁₋₅ alkyl), -CN, -NO₂, -NH₂, -NH(C₁₋₅ alkyl), - N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
   R⁴ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, - SO₂NH(C₁₋₅ alkyl) and -SO₂N(C₁₋₅ alkyl)(C₁₋₅ alkyl);
   R⁵ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, - SO₂NH(C₁₋₅ alkyl) and -SO₂N(C₁₋₅ alkyl)(C₁₋₅ alkyl);
   A is a phenyl or five- or six-membered heteroaryl, optionally substituted with one or more groups selected from R^{S}; and
   each R^{S} is independently selected from halogen, C₁₋₅ alkyl, C₁₋₅ haloalkyl, -OH, -O(C₁₋₅ alkyl), -(C₁₋₅ alkylene)-OH, -CN, -NH₂, -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -O-(C₁₋₅ alkylene)-NH₂, -O-(C₂₋₅ alkylene)-NH(C₁₋₅ alkyl), -O-(C₂₋₅ alkylene)-N(C₁₋₅ alkyl)(C₁₋₅ alkyl), -NO₂, -NH(C₁₋₅ alkyl), -N(C₁₋₅ alkyl)(C₁₋₅ alkyl), -CHO, -COOH, -COO(C₁₋₅ alkyl), - CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

In a second embodiment, the present invention relates to a pharmaceutical composition comprising the immunoconjugate of the present invention, and a pharmaceutically acceptable carrier.

In a third embodiment, the present invention relates to an immunoconjugate of the present invention or a pharmaceutical composition of the present invention for use as a medicament.

In a fourth embodiment, the present invention relates to an immunoconjugate of the present invention or a pharmaceutical composition of the present invention for use in treatment or prevention of cancer.

In a fifth embodiment, the present invention relates to use of the immunoconjugate of the present invention in manufacture of a medicament for the treatment or prevention or cancer.

In a sixth embodiment, the present invention relates to a method of treatment of cancer, the method comprising administering the immunoconjugate of the present invention to an individual in need thereof. It is to be assumed that a therapeutically effective dose of immunoconjugate is to be administered.

In a seventh embodiment, the present invention relates to a compound of formula:

Z_{Z}-L₁-L_{P}-L₂-X-Y (III)

or a pharmaceutically acceptable salt thereof, wherein
Zz is Z₁ or Z₂.,
Z₁ is selected from 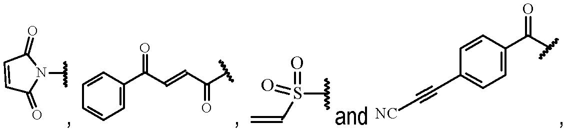 or Z₁ is selected from 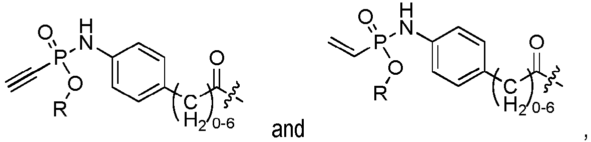 wherein R is -(CH₂CH₂O)₁₋₂₃-CH₂CH₂OH or -(CH₂CH₂O)₁₋₂₃-CH₂CF₃;
Z₂ is selected from 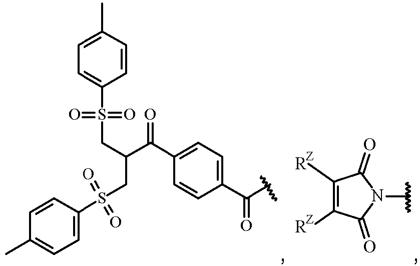 wherein each R^{Z} independently is Hal
(such as Cl, Br or I) or -S-aryl (such as -S-phenyl), and 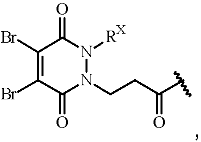 wherein R^{X} is C₁₋₅ alkyl (such as methyl or ethyl), C₂₋₅ alkenyl or C₂₋₅ alkynyl (such as propargyl);
L₁, L_{P}, L₂, and X are as defined for formula (II); and
Y is the moiety of formula (Ia), or the moiety of formula (Ib).

### Brief description of figures

The invention is illustrated with the following figures, which are not to be construed as limiting the scope of the invention in any way, which is defined by the hereto appended claims.

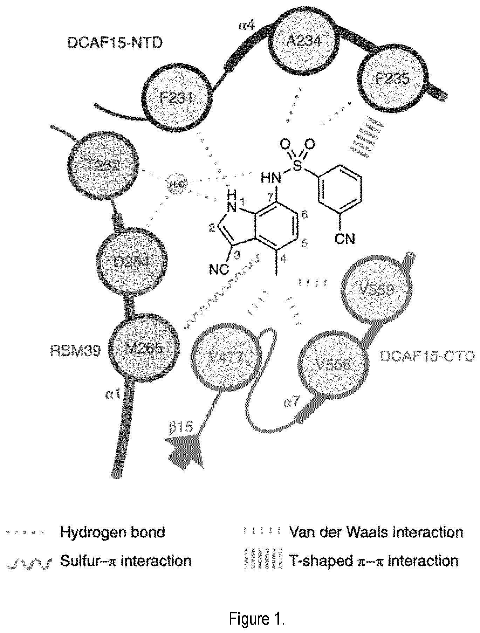

- **Fig. 1**: shows schematic illustration of E7820 and its interactions with DCAF15 and RBM39, highlighting the key amino acid residues and functional groups important for the biological activity of E7820. Figure copied from reference²⁵.

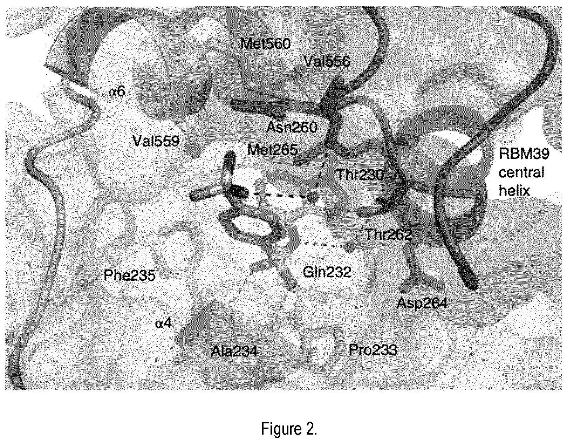

- **Fig. 2**: shows Indisulam-mediated interactions between DCAF15 and RBM39. Indisulam bridges the structure of DCAF15 and RBM39 by forming several direct or water-mediated interactions with both DCAF15 and RBM39 leading to increased complementarity between the two surfaces. Hydrogen bonds are shown as dotted lines and the surfaces of both DCAF15 and RBM39 are shown with key residues labeled. Figure copied from reference²⁶.

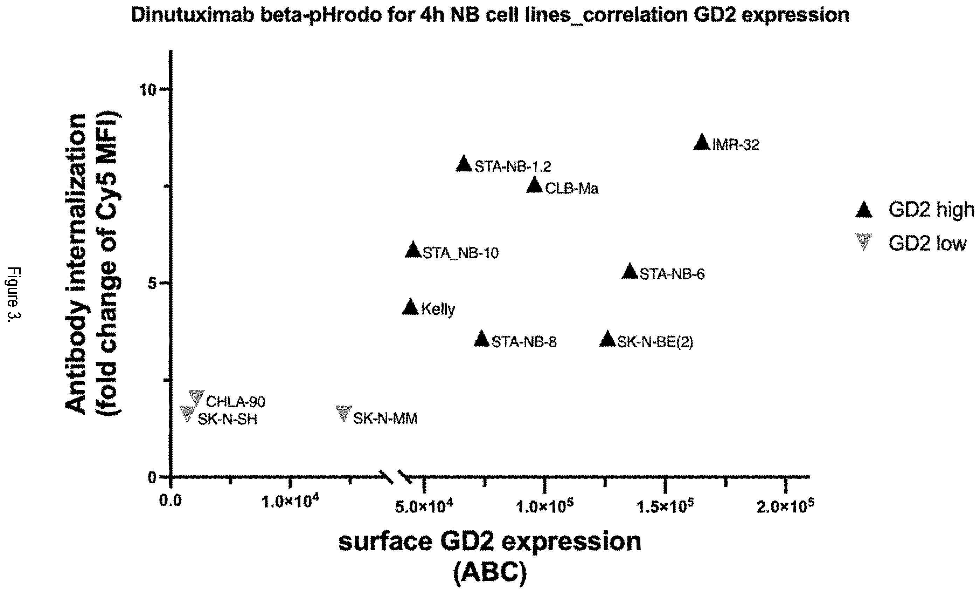

- **Fig. 3**: presents dinutuximab beta antibody internalization rates plotted against cell surface present GD2 assessed by flow cytometry on a test panel of human neuroblastoma cell lines. Error bars represent SD. This experiment was performed twice, in technical triplicates, with similar results obtained each time. Representative data from one experiment are shown.

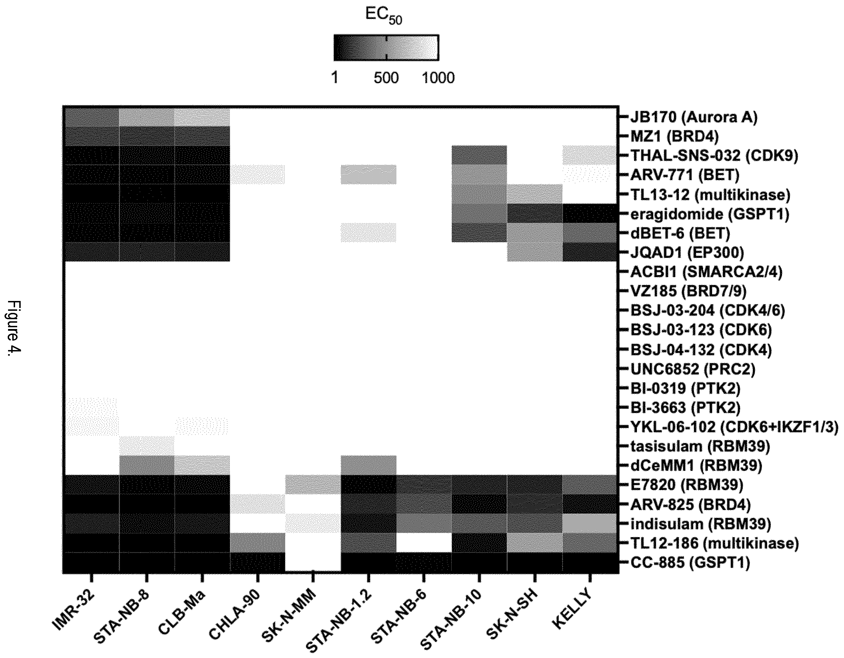

- **Fig. 4**: shows the results of the cell viability-based degrader payload screen evaluated by CellTiter-Blue^{®} cell viability assay after 72 h treatment and plotted as EC₅₀ values. Percentages of the viable neuroblastoma cells normalized to vehicle-treated controls were plotted and EC₅₀ values were calculated using log(inhibitor) vs. response -- variable slope equation in GraphPad Prism 9 software. This experiment was performed once, in technical triplicates and results were validated in the follow-up experiments. RBM39 molecular glue degrader E7820 displayed <150 nM anti-cancer activities in 7 out of 10 tested neuroblastoma cell lines, 5 of which bear *MYCN* oncogene amplification.

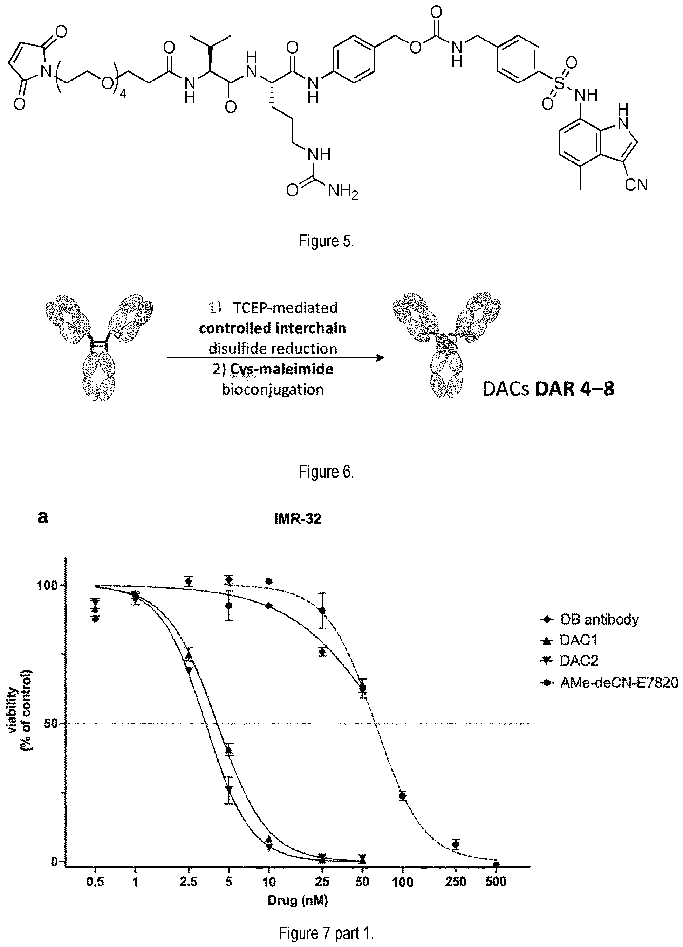

- **Fig. 5**: presents structure of the maleimide-containing chemical modifier (JKW-68) with AMe-deCN-E7820 (JKW-48) as the RBM39 degrader payload used for the preparation of the first generation anti-GD2/RBM39 DACs (DAR 4-8).
- **Fig. 6**: shows schematic illustration of the preparation of anti-GD2 DACs comprising of a controlled interchain disulfide reduction using tris(2-carboxyethyl)phosphine (TCEP) followed by a site-specific modification of the reduced Cys thiol groups using maleimide-containing chemical linkers. Reaction conditions such as temperature, reaction time, reaction buffer, amount of the TCEP reagent and finally amount of the maleimide-containing chemical modifier were optimized to obtain DACs with average DAR ranging from 4 to 8.

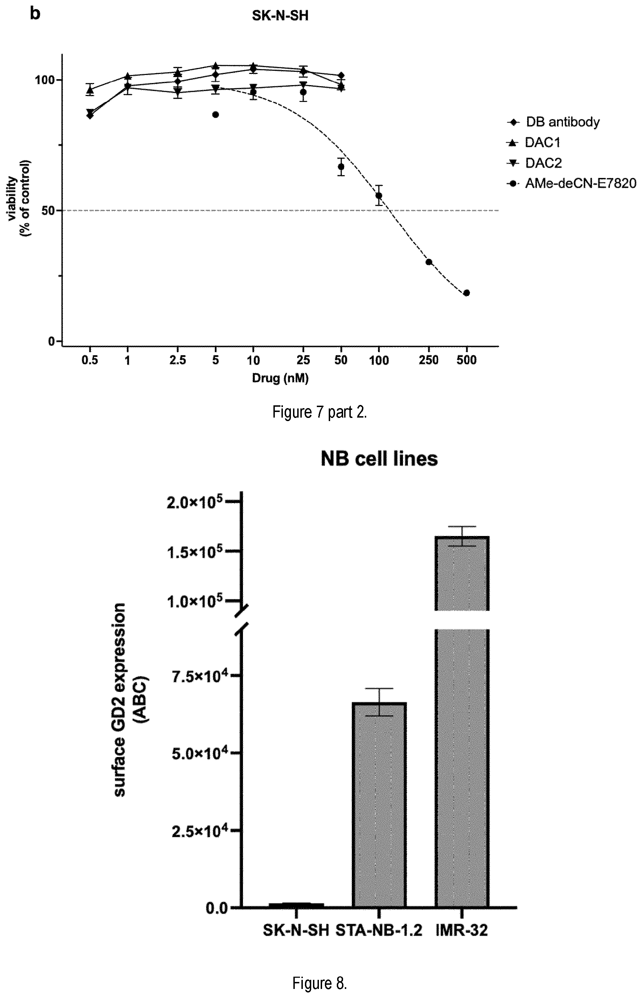

- **Fig. 7**: presents data on *in vitro* anti-cancer activity of the first generation anti-GD2/RBM39 DACs in **(a)** IMR-32 (GD2⁺) and **(b)** SK-N-SH (GD2) neuroblastoma cell line evaluated by CellTiter-Blue^{®} cell viability assay after 72 h treatment. DAC1 corresponds to an anti-GD2/RBM39 DAC with an average DAR ~4 whilst DAC2 to an anti-GD2/RBM39 DAC with an average DAR ~6. Percentages of the viable neuroblastoma cells normalized to vehicle-treated controls were plotted. Error bars represent SD. This experiment was performed twice, in technical triplicates, with similar results obtained each time. Representative data from one experiment are shown. EC₅₀ values were calculated using log(inhibitor) vs. response -- variable slope equation in GraphPad Prism 9 software.
- **Fig. 8**: shows surface GD2 expression in the selected neuroblastoma cell lines used in the anti-GD2/RBM39 DAC *in vitro* biological activity evaluation experiments assessed by flow cytometry. Results show that IMR-32 cells contain ~2.5 times higher number of surface GD2 disialoganglioside TAA compared to STA-NB-1.2 cells, whereas only low amount of GD2 can be detected on the surface of SK-N-SH cells. Viable neuroblastoma cells were selected using a FACS gating strategy comprising of FSC-A/SSC-A selection of neuroblastoma cells, FSC-A/FSC-H exclusion of doublets and finally selection of FSC-A/PI⁻ viable neuroblastoma cells. Geometric mean fluorescence intensity was determined for every sample, BD Quantibrite^{™} Phycoerythrin (PE) Fluorescence Quantitation Kit (BD Biosciences 340495) were analyzed according to the manufacturer's instructions with every run. This was used to generate a standard curve from which the PE geometric mean for each sample was converted into the number of antibodies bound per cell (ABC) representing surface antigen expression. Error bars represent SD. This experiment was performed three times, in technical triplicates, with similar results obtained each time. Representative data from one experiment are shown.

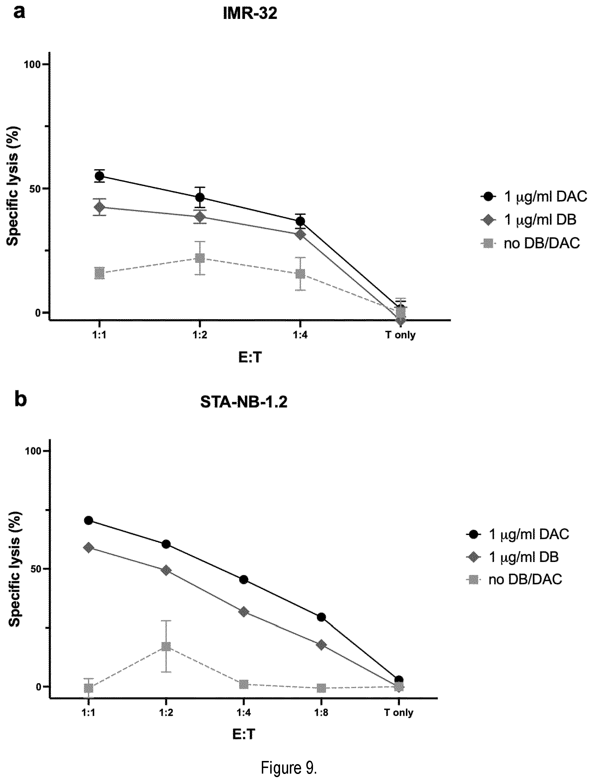

- **Fig. 9**: presents *in vitro* anti-cancer activity of the first generation anti-GD2/RBM39 DAC or DB antibody in 22 h co-culture assay with freshly isolated human HD NK cells in GD2⁺ **(a)** IMR-32 and **(b)** STA-NB-1.2 neuroblastoma cell line evaluated by flow cytometry-based assay. Percentage of the viable neuroblastoma cells normalized to vehicle-treated controls containing target viable neuroblastoma cells only were plotted. Viable neuroblastoma cells were selected using a FACS gating strategy comprising of the selection of VPD450⁺ cells, then FSC-A/SSC-A selection of neuroblastoma cells, FSC-A/FSC-H exclusion of doublets and finally selection of VPD450⁺/7-AAD⁻ viable neuroblastoma cells. E:T represents effector (NK cells) to target (neuroblastoma cells) ratio. Error bars represent SD. This experiment was performed twice, in technical triplicates, with similar results obtained each time. Representative data from one experiment are shown.

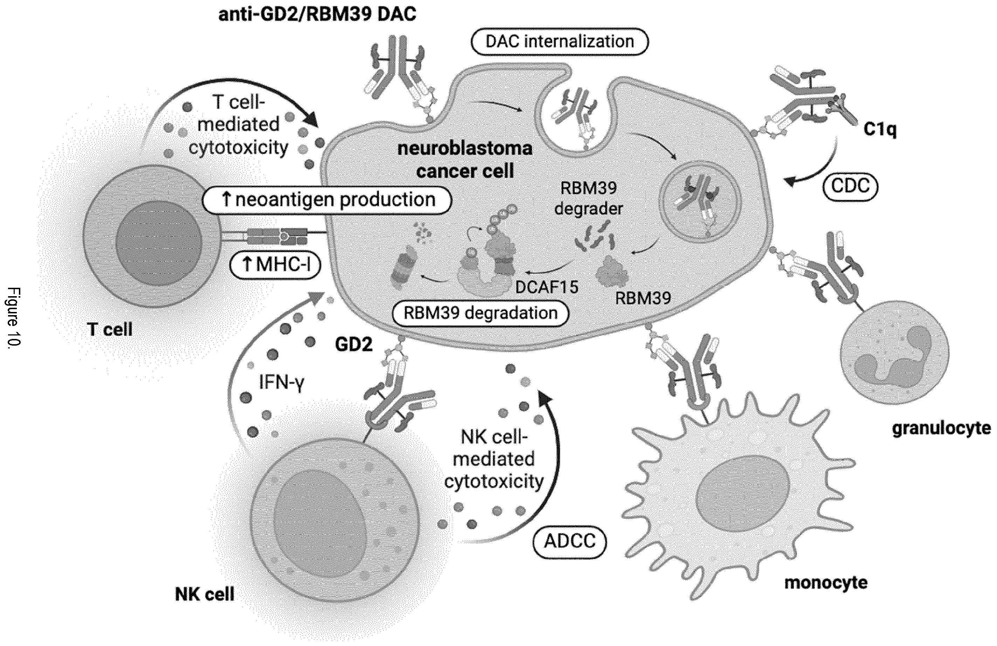

- **Fig. 10**: discusses proposed novel and unique mechanism of action of the anti-GD2/RBM39 DACs leading to increased anti-cancer efficacy against neuroblastoma tumor cells.

### Detailed description of the invention

As mentioned before, in one embodiment, the present invention relates to an immunoconjugate comprising
a) an antibody binding to a tumor antigen displayed on the surface of a cancer cell; and
b) a compound according to formula (I): 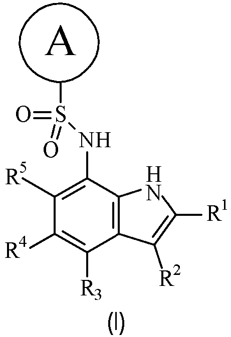 wherein
   R¹ is selected from -H, C₁₋₅ alkyl, -OH, -CN and -NO₂;
   R² is selected from -H, halogen, C₁₋₅ alkyl, C₁₋₅ haloalkyl, -OH, -CN, -NO₂, -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and - SO₂N(C₁₋₅ alkyl)(C₁₋₅ alkyl);
   R³ is selected from -H, halogen, C₁₋₅ alkyl, aryl, -OH, -O(C₁₋₅ alkyl), -CN, -NO₂, -NH₂, -NH(C₁₋₅ alkyl), - N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
   R⁴ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, - SO₂NH(C₁₋₅ alkyl) and -SO₂N(C₁₋₅ alkyl)(C₁₋₅ alkyl);
   R⁵ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, - SO₂NH(C₁₋₅ alkyl) and -SO₂N(C₁₋₅ alkyl)(C₁₋₅ alkyl);
   A is a phenyl or five- or six-membered heteroaryl, optionally substituted with one or more groups selected from R^{S}; and
   each R^{S} is independently selected from halogen, C₁₋₅ alkyl, C₁₋₅ haloalkyl, -OH, -O(C₁₋₅ alkyl), -(C₁₋₅ alkylene)-OH, -CN, -NH₂, -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -O-(C₁₋₅ alkylene)-NH₂, -O-(C₂₋₅ alkylene)-NH(C₁₋₅ alkyl), -O-(C₂₋₅ alkylene)-N(C₁₋₅ alkyl)(C₁₋₅ alkyl), -NO₂, -NH(C₁₋₅ alkyl), -N(C₁₋₅ alkyl)(C₁₋₅ alkyl), -CHO, -COOH, -COO(C₁₋₅ alkyl), - CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

It has surprisingly been found in the appended examples that the present invention is in particular characterized by an improved cancer therapy, in particular an improved neuroblastoma/pediatric neuroblastoma therapy. Such an improvement comprises enhancement of treatment efficacy when antibody therapy, in particular anti-GD2 immunotherapy, is employed. As was shown herein, the specifically claimed therapeutic approaches employing immunoconjugate of the invention can, *inter alia,* lead to the preferred use of minimal and reduced dosages of (in particular) therapeutically active antibodies, like anti-GD2 antibodies. Since such antibodies are known to induce undesired side effects, one of the gists of the present invention is that reduced amount of these antibodies can be used *in vivo* which, in turn, enables the skilled artisan to achieve successful medical interventions.

Accordingly, as previously mentioned, the immunoconjugate of the invention comprises a) an antibody binding to a tumor antigen (such as GD2) displayed on the surface of a cancer cell.

As understood herein, the term "binding to" as used in the context of the present invention defines a binding (interaction) of at least two "antigen-interaction-sites" with each other. The term "antigen-interaction-site" defines, in accordance with the present invention, a motif of a polypeptide, i.e., a part of the antibody of the present invention, which shows the capacity of specific interaction with a specific antigen or a specific group of antigens displayed on the surface of the cancer cell. Preferably, said binding is to be understood as specific binding.

As understood herein, the notion that an antibody "specifically binds" to a certain target antigen (or an epitope thereof) is well-known in the art. In particular, an antibody can be said to "specifically bind" to a certain target antigen (or epitope) if it binds to said target antigen (or epitope) with greater affinity, avidity, more readily, and/or with greater duration (preferably with greater affinity) than it binds to other alternative antigens. Moreover, an antibody that "specifically binds" to a certain epitope (of an antigen) may be an antibody that binds to this epitope with greater affinity, avidity, more readily, and/or with greater duration (preferably with greater affinity) than it binds to other epitopes of the same antigen. It will be understood that "specific binding" does not necessarily require exclusive binding to the corresponding target, although such exclusive (or nearly exclusive) binding is generally desirable. Accordingly, an antibody that specifically binds to a first antigen may or may not specifically bind to a second antigen (which is different from the first antigen). In some embodiments, an antibody that "specifically binds" to a target antigen does not (or does not significantly) bind to other antigens (or, analogously, an antibody that "specifically binds" to a certain epitope may not, or may not significantly, bind to other epitopes in the same antigen), which may be reflected, e.g., in that only baseline binding activity can be detected for other antigens (or other epitopes).

As used herein, the term "epitope" refers to the site on a target antigen that is recognized and bound by an antibody. An epitope may be linear and, in that case, may typically have a length of 6 to 15 amino acid residues. Alternatively, an epitope can be conformational. The epitope to which an antibody (or an antigen-binding fragment) binds can be determined by routine methods, e.g., by epitope mapping methods, as also described further below.

As used herein, "tumor antigen" refers to a molecule that is present in cancer cells, but absent from normal (non-cancerous) cells. Particularly relevant in the context of the present invention are tumor antigens that are displayed on the surface of the cancer cell. Particularly preferred such tumor-specific antigens are described in the following. The tumor antigen is preferably a protein. However, the tumor antigen is not limited to protein, and tumor antigens that are e.g. sugar molecules, are also known.

As mentioned before, the tumor antigen (like GD2) is preferably displayed on a surface of a cancer cell. As referred to herein, the cancer cell is not particularly limited and the antibody comprised in the conjugate of the invention may be targeting any cancer cell. It is however preferred that the cancer cell, as referred to herein, is selected from a neuroblastoma cell, an Ewing sarcoma cell, an osteosarcoma cell, a leukemia cell (such as AML cell), a rhabdomyosarcoma cell, a melanoma cell, a breast cancer cell, a triple negative breast cancer cell, a lung cancer cell, a colorectal cancer cell (such as colorectal adenoma cell), an ovarian cancer cell, a multiple myeloma cell, a hepatocellular carcinoma cell, pancreatic cancer cell, brain cancer cell (such as glioma cell), a medulloblastoma cell, prostate cancer cell, bladder cancer cell, urethral cancer cell, cervical cancer cell, a soft tissue sarcoma cell and a lymphoma cell. Preferably, the cancer cell is selected from a neuroblastoma cell, an Ewing sarcoma cell, an osteosarcoma cell, a rhabdomyosarcoma cell, a melanoma cell and a triple negative breast cancer cell. As referred to herein, the colorectal cancer cell is preferably a colorectal adenoma cell. As further referred to herein, a brain cancer cell is not particularly limited and may refer to any brain cancer. It is however particularly preferred that the brain cancer cell is a glioma cell.

Particularly preferred is a cancer cell expressing RBM39. As referred to herein, RBM39 refers to the pre-mRNA splicing factor RNA-binding motif protein 39 (RBM39). This protein is known to the skilled person. Its sequence can be retrieved in the UniProt database under UniProt code Q14498. This protein is an RNA binding protein and possible splicing factor. The RBM39 protein is found in the nucleus, where it colocalizes with core spliceosomal proteins. Studies of a mouse protein with high sequence similarity to this protein suggest that this protein may act as a transcriptional coactivator for JUN/AP-1 and estrogen receptors. Multiple transcript variants encoding different isoforms have been observed for this gene

Several cancers have been shown to be dependent on RBM39, which have been summarized in Table 1. In each of the cancers referred to in Table 1, RBM39 is overexpressed. Particularly suitable target surface antigens characteristics to these cancers are provided.

**Table 1. Role of RBM39 in selected cancers (adapted from C. Xu et al.¹) with a selection of relevant surface antigen targets for the development of RBM39 DACs.**

| **Cancer** | **Function** | **Target surface antigen** | | |
|---|---|---|---|---|
| **Neuroblastoma** | **Required for neuroblastoma tumor cell growth and survival** | **GD2*^{b}*** | **B7-H3*^{c}*** | GPC2 |
| **Ewing sarcoma** | **Regulates proliferation and protein synthesis pathways** | **GD2*^{b}*** | B7-H3*^{c}* | STEAP-1 |
| **Osteosarcoma** | **Promotes cancer cell invasion and metastasis** | **GD2*^{b}*** | B7-H3*^{c}* | HER2*^{a}* |
| **AML** | **Causes changes in the splicing of HOXA9 target genes** | **CD33*^{a}*** | CLL-1 | CD123*^{c}* |
| Rhabdomyosarcoma | Promotes alternative splicing of the insulin receptor (IR) to IR-A isoform | GD2*^{b}* | B7-H3*^{c}* | HER2*^{a}* |
| Melanoma | Regulation of genes involved in mitochondrial energy metabolism | GD2*^{b}* | B7-H3*^{c}* | HER3*^{c}* |
| Breast | Mediates VEGF alternative splicing | HER2*^{a}* | B7-H3*^{c}* | Trop-2*^{a}* |
| Triple-negative breast cancer (TNBC) | Regulates DNA repair, cell apoptosis and cell cycle | GD2*^{b}* | B7-H3*^{c}* | Trop-2*^{a}* |
| Lung | Promotes proliferation and migration | GD2*^{b}* | HER2*^{a}* | B7-H3*^{c}* |
| Colorectal adenoma | Mediates cell viability Affects cell survival and viability | HER2*^{a}* | MUC16 | EGFR*^{b}* |
| Ovarian | Regulates mRNA splicing of DNA damage repair genes | GD2*^{b}* | FR-α*^{a}* | Trop-2*^{a}* |
| Multiple myeloma | Regulates mTOR signaling, promotes cell proliferation | BCMA^{a} | CD74*^{b}* | CD38*^{b}* |
| Hepatocellular carcinoma (HCC) | Related to the appearance of microvessels | GPC3 | EGFR*^{b}* | FR-α*^{a}* |

^{a}ADC-based targeted therapy approved by FDA or EMA. *^{b}*Antibody-based immunotherapy approved by FDA or EMA. *^{c}*ADC-based targeted therapy in a clinical trial.

Accordingly, in the immunoconjugate of the present invention, (a) is an antibody binding to a tumor antigen displayed on the surface of a cancer cell that expresses RBM39. Preferably, such cell expressing RBM39 is a cell overexpressing RBM39. It is to be understood that said antibody binds to a tumor antigen on surface of such cell. Such tumor antigens are disclosed.

Thus, according to the present invention, the tumor antigen is selected from GD2, O-acetyl-GD2 (such as 7-*O*-acetyl-GD2 or 9-*O*-acetyl-GD2), B7-H3, GPC2, ALK, HER2, HER3, Trop-2, CD33, CLL-1, c-KIT, CD123, EGFR, FR-a, MUC16 and STEAP1.

Further known tumor antigens include ROR1, ROR2, CEACAM5, CEACAM6, Nectin-4, CD19, CD22, CD30, CD37, CD79b, CD142, CD166, B7-H4, BCMA, 5T4, PSMA, NaPi2b, c-Met, claudin 18.2, claudin 3, claudin 4, claudin 6, integrin beta-6, mesothelin, AXL, LIV-1, FLT3, CA9, DLL3 and PD-L1.

As understood herein, GD2 is a disialoganglioside, also referred to as ganglioside GD2. It is expressed on tumors of neuroectodermal origin, including human neuroblastoma and melanoma, with highly restricted expression on normal tissues, principally to the cerebellum and peripheral nerves in humans. The relatively tumor-specific expression of GD2 makes it a suitable target for immunotherapy with monoclonal antibodies or with artificial T cell receptors. The term GD2 may refer to GD2 or its *O*-acetylated form, which can also be referred to as *O*-acetyl-GD2. Preferably, unless indicated to the contrary, the term GD2 refers to non-acetylated form of GD2, as defined in the following.

Accordingly, GD2 may refer to a non-acetylated form of said ganglioside, which can be defined by its chemical name: (2*R*,4*R*,5*S*,6*S*)-2-[3-[(2*S*,3*S*,4*R*,6*S*)-6-[(2*S*,3*R*,4*R*,5*S*,6*R*)-5-[(2*S*,3*R*,4*R*,5*R*,6*R*)-3-acetamido-4,5-dihydroxy-6-(hydroxymethyl)oxan-2-yl]oxy-2-[(2*R*,3*S*,4*R*,5*R*,6*R*)-4,5-dihydroxy-2-(hydroxymethyl)-6-[(*E*)-3-hydroxy-2-(octadecanoylamino)octadec-4-enoxy]oxan-3-yl]oxy-3-hydroxy-6-(hydroxymethyl)oxan-4-yl]oxy-3-amino-6-carboxy-4-hydroxyoxan-2-yl]-2,3-dihydroxypropoxy]-5-amino-4-hydroxy-6-(1,2,3-trihydroxypropyl)oxane-2-carboxylic acid. Said GD2 may also be defined structurally, as being according to formula:

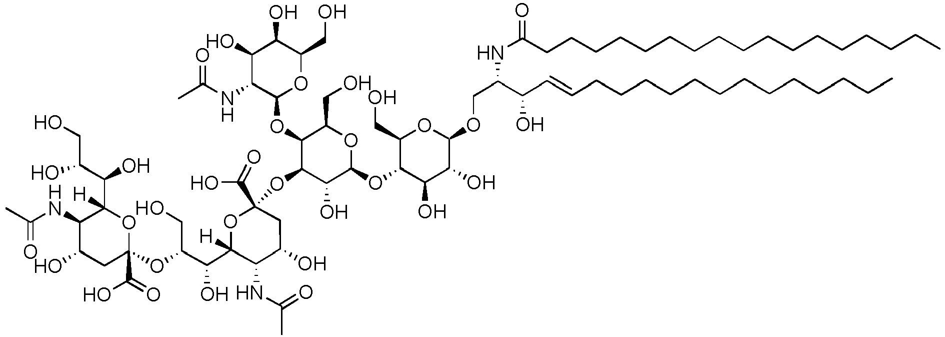

Alternatively, the term GD2 may also refer to acetylated version of GD2, also referred to as *O*-acetyl-GD2. Different types of *O*-acetyl modification of GD2 exist and include 7-*O*-acetyl-GD2 and 9-*O*-acetyl-GD2. As understood herein, 9-*O*-acetyl-GD2 is a compound according to formula:

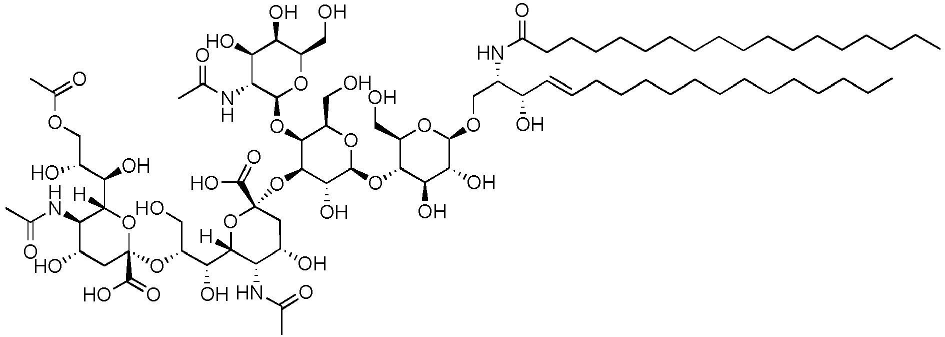

As further understood herein, 7-*O*-acetyl-GD2 is a compound according to formula:

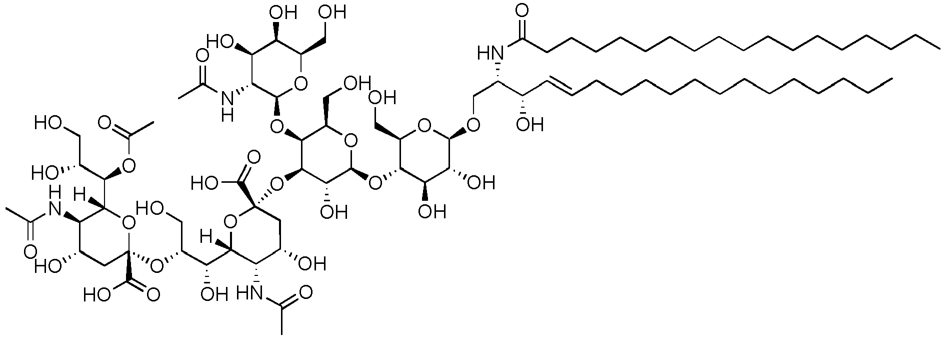

If reference is made to *O*-acetyl-GD2 or acetylated form of GD2, a reference is thereby made to 7-*O-*acetyl-GD2 and/or 9-*O*-acetyl-GD2. Such reference may also be understood as a reference made individually to 7-*O*-acetyl-GD2, or a reference made individually to 9-*O*-acetyl-GD2.

Alternatively, the tumor antigen may be a protein. Such tumor antigen is preferably selected from B7-H3, GPC2, ALK, HER2, HER3, Trop-2, CD33, CLL-1, c-KIT, CD123, EGFR, FR-a, MUC16 and STEAP1.

Accordingly, said tumor antigen may be B7-H3. As apparent to the skilled person, B7-H3 is type I transmembrane protein, existing in two isoforms determined by its extracellular domain. In humans, the extracellular domain consists of one pair (2Ig-B7-H3) or two identical pairs (4Ig-B7-H3) due to exon duplication. B7-H3 mRNA is expressed in most normal tissues. In contrast, B7-H3 protein has a very limited expression on normal tissues because of its post-transcriptional regulation by microRNAs. However, B7-H3 protein is expressed at high frequency on many different cancer types (60% of all cancers). The 4Ig-B7-H3 isoform is predominant in cancer. Protein B7-H3 is a protein characterized by an amino acid sequence according to SEQ ID NO.: 1.

B7-H3 (SEQ ID NO.: 1):
MLRRRGSPGMGVHVGAALGALWFCLTGALEVQVPEDPVVALVGTDATLCCSFSPEPGFSLAQLNLIWQLTDTKQL
VHSFAEGQDQGSAYANRTALFPDLLAQGNASLRLQRVRVADEGSFTCFVSIRDFGSAAVSLQVAAPYSKPSMTLE
PNKDLRPGDTVTITCSSYQGYPEAEVFWQDGQGVPLTGNVTTSQMANEQGLFDVHSILRVVLGANGTYSCLVRNP
VLQQDAHSSVTITPQRSPTGAVEVQVPEDPVVALVGTDATLRCSFSPEPGFSLAQLNLIWQLTDTKQLVHSFTEG
RDQGSAYANRTALFPDLLAQGNASLRLQRVRVADEGSFTCFVSIRDFGSAAVSLQVAAPYSKPSMTLEPNKDLRP
GDTVTITCSSYRGYPEAEVFWQDGQGVPLTGNVTTSQMANEQGLFDVHSVLRVVLGANGTYSCLVRNPVLQQDAH
GSVTITGQPMTFPPEALWVTVGLSVCLIALLVALAFVCWRKIKQSCEEENAGAEDQDGEGEGSKTALQPLKHSDS
KEDDGQEIA

Said tumour antigen may also be glypican 2 (GPC2), also known cerebroglycan. Cerebroglycan is a glycophosphatidylinositol-linked integral membrane heparan sulfate proteoglycan found in the developing nervous system. Cerebroglycan participates in cell adhesion and is thought to regulate the growth and guidance of axons. Cerebroglycan has especially high affinity for laminin-1. GPC2 has been identified as a therapeutic target in neuroblastoma in two independent studies published by Mitchell Ho's lab at the NCI and John Maris's lab at the University of Pennsylvania in 2017. GPC2 is highly expressed in about half of neuroblastoma cases and that high GPC2 expression correlates with poor overall survival. Protein GPC2 is characterized by an amino acid sequence according to SEQ ID NO.: 2.

GPC2 (SEQ ID NO.: 2):
MSALRPLLLLLLPLCPGPGPGPGSEAKVTRSCAETRQVLGARGYSLNLIPPALISGEHLRVCPQEYTCCSSETEQ
RLIRETEATFRGLVEDSGSFLVHTLAARHRKFDEFFLEMLSVAQHSLTQLFSHSYGRLYAQHALIFNGLFSRLRD
FYGESGEGLDDTLADFWAQLLERVFPLLHPQYSFPPDYLLCLSRLASSTDGSLQPFGDSPRRLRLQITRTLVAAR
AFVQGLETGRNVVSEALKVPVSEGCSQALMRLIGCPLCRGVPSLMPCQGFCLNVVRGCLSSRGLEPDWGNYLDGL
LILADKLQGPFSFELTAESIGVKISEGLMYLQENSAKVSAQVFQECGPPDPVPARNRRAPPPREEAGRLWSMVTE
EERPTTAAGTNLHRLVWELRERLARMRGFWARLSLTVCGDSRMAADASLEAAPCWTGAGRGRYLPPVVGGSPAEQ
VNNPELKVDASGPDVPTRRRRLQLRAATARMKTAALGHDLDGQDADEDASGSGGGQQYADDWMAGAVAPPARPPR
PPYPPRRDGSGGKGGGGSARYNQGRSRSGGASIGFHTQTILILSLSALALLGPR

Anaplastic lymphoma kinase (ALK) is also known as ALK tyrosine kinase receptor or CD246 (cluster of differentiation 246). Anaplastic lymphoma kinase (ALK) was originally discovered in 1994 in anaplastic large-cell lymphoma (ALCL) cells. ALCL is caused by a (2;5)(p23:q35) chromosomal translocation that generates the fusion protein NPM-ALK, in which the kinase domain of ALK is fused to the amino-terminal part of the nucleophosmin (NPM) protein. Dimerization of NPM constitutively activates the ALK kinase domain. The full-length protein ALK was identified in 1997. The deduced amino acid sequences revealed that ALK was a novel receptor tyrosine kinase (RTK), having an extracellular ligand-binding domain, a transmembrane domain, and an intracellular tyrosine kinase domain. While the tyrosine kinase domain of human ALK shares a high degree of similarity with that of the insulin receptor, its extracellular domain is unique among the RTK family in containing two MAM domains (meprin, A5 protein and receptor protein tyrosine phosphatase mu), an LDLa domain (low-density lipoprotein receptor class A) and a glycine-rich region. Based on overall homology, ALK is closely related to the leukocyte receptor tyrosine kinase (LTK) and, together with the insulin receptor, forms a subgroup in the RTK superfamily. The receptor ALK plays a pivotal role in cellular communication and in the normal development and function of the nervous system. This observation is based on the extensive expression of ALK messenger RNA (mRNA) throughout the nervous system during mouse embryogenesis. In vitro functional studies have demonstrated that ALK activation promotes neuronal differentiation of PC12 or neuroblastoma cell lines. Protein Alk is characterized by an amino acid sequence according to SEQ ID NO.: 3.

ALK (SEQ ID NO.: 3):
MGAIGLLWLLPLLLSTAAVGSGMGTGQRAGSPAAGPPLQPREPLSYSRLQRKSLAVDFVVPSLFRVYARDLLLPP
SSSELKAGRPEARGSLALDCAPLLRLLGPAPGVSWTAGSPAPAEARTLSRVLKGGSVRKLRRAKQLVLELGEEAI
LEGCVGPPGEAAVGLLQFNLSELFSWWIRQGEGRLRIRLMPEKKASEVGREGRLSAAIRASQPRLLFQIFGTGHS
SLESPTNMPSPSPDYFTWNLTWIMKDSFPFLSHRSRYGLECSFDFPCELEYSPPLHDLRNQSWSWRRIPSEEASQ
MDLLDGPGAERSKEMPRGSFLLLNTSADSKHTILSPWMRSSSEHCTLAVSVHRHLQPSGRYIAQLLPHNEAAREI
LLMPTPGKHGWTVLQGRIGRPDNPFRVALEYISSGNRSLSAVDFFALKNCSEGTSPGSKMALQSSFTCWNGTVLQ
LGQACDFHQDCAQGEDESQMCRKLPVGFYCNFEDGFCGWTQGTLSPHTPQWQVRTLKDARFQDHQDHALLLSTTD
VPASESATVTSATFPAPIKSSPCELRMSWLIRGVLRGNVSLVLVENKTGKEQGRMVWHVAAYEGLSLWQWMVLPL
LDVSDRFWLQMVAWWGQGSRAIVAFDNISISLDCYLTISGEDKILQNTAPKSRNLFERNPNKELKPGENSPRQTP
IFDPTVHWLFTTCGASGPHGPTQAQCNNAYQNSNLSVEVGSEGPLKGIQIWKVPATDTYSISGYGAAGGKGGKNT
MMRSHGVSVLGIFNLEKDDMLYILVGQQGEDACPSTNQLIQKVCIGENNVIEEEIRVNRSVHEWAGGGGGGGAT
YVFKMKDGVPVPLIIAAGGGGRAYGAKTDTFHPERLENNSSVLGLNGNSGAAGGGGGWNDNTSLLWAGKSLQEGA
TGGHSCPQAMKKWGWETRGGFGGGGGGCSSGGGGGGYIGGNAASNNDPEMDGEDGVSFISPLGILYTPALKVMEG
HGEVNIKHYLNCSHCEVDECHMDPESHKVICFCDHGTVLAEDGVSCIVSPTPEPHLPLSLILSVVTSALVAALVL
AFSGIMIVYRRKHQELQAMQMELQSPEYKLSKLRTSTIMTDYNPNYCFAGKTSSISDLKEVPRKNITLIRGLGHG
AFGEVYEGQVSGMPNDPSPLQVAVKTLPEVCSEQDELDFLMEALIISKFNHQNIVRCIGVSLQSLPRFILLELMA
GGDLKSFLRETRPRPSQPSSLAMLDLLHVARDIACGCQYLEENHFIHRDIAARNCLLTCPGPGRVAKIGDFGMAR
DIYRASYYRKGGCAMLPVKWMPPEAFMEGIFTSKTDTWSFGVLLWEIFSLGYMPYPSKSNQEVLEFVTSGGRMDP
PKNCPGPVYRIMTQCWQHQPEDRPNFAIILERIEYCTQDPDVINTALPIEYGPLVEEEEKVPVRPKDPEGVPPLL
VSQQAKREEERSPAAPPPLPTTSSGKAAKKPTAAEISVRVPRGPAVEGGHVNMAFSQSNPPSELHKVHGSRNKPT
SLWNPTYGSWFTEKPTKKNNPIAKKEPHDRGNLGLEGSCTVPPNVATGRLPGASLLLEPSSLTANMKEVPLFRLR
HFPCGNVNYGYQQQGLPLEAATAPGAGHYEDTILKSKNSMNQPGP

Including most common mutations occurring at three hotspots within the tyrosine kinase domain: F1174L (or alternatively to C, S, V or I), F1245V (or alternatively to I, C, Y or L) and R1275Q (or alternatively to L). These mutations are the most frequent mutations occurring in neuroblastoma. Further mutations commonly occurring in other malignancies include G1202R (or deletion), L1196M, L1152R, C1156Y, G1269A and I1171N (or alternatively to S or T).

Receptor tyrosine-protein kinase erbB-2 is a protein that normally resides in the membranes of cells and is encoded by the ERBB2 gene. ERBB is abbreviated from erythroblastic oncogene B, a gene originally isolated from the avian genome. The human protein is also frequently referred to as HER2 (human epidermal growth factor receptor 2) or CD340 (cluster of differentiation 340). HER2 is a member of the human epidermal growth factor receptor (HER/EGFR/ERBB) family. But contrary to other members of the ERBB family, HER2 does not directly bind ligand. HER2 activation results from heterodimerization with another ERBB member or by homodimerization when HER2 concentration are high, for instance in cancer. Amplification or over-expression of this oncogene has been shown to play an important role in the development and progression of certain aggressive types of breast cancer. In recent years the protein has become an important biomarker and target of therapy for approximately 30% of breast cancer patients. Protein HER2 is characterized by an amino acid sequence according to SEQ ID NO.: 4.

HER2 (SEQ ID NO.: 4):


















Receptor tyrosine-protein kinase erbB-3, also known as HER3 (human epidermal growth factor receptor 3), is a membrane bound protein that in humans is encoded by the ERBB3 gene.ErbB3 is a member of the epidermal growth factor receptor (EGFR/ERBB) family of receptor tyrosine kinases. The kinase-impaired ErbB3 is known to form active heterodimers with other members of the ErbB family, most notably the ligand binding-impaired ErbB2. Protein HER3 is characterized by an amino acid sequence according to SEQ ID NO.: 5.

HER3 (SEQ ID NO.: 5):
























Tumor-associated calcium signal transducer 2, also known as Trop-2 and as epithelial glycoprotein-1 antigen (EGP-1) is a protein that in humans is encoded by the TACSTD2 gene. This intronless gene encodes a carcinoma-associated antigen defined by the monoclonal antibody GA733. This antigen is a member of a family including at least two type I membrane proteins. It transduces an intracellular calcium signal and acts as a cell surface receptor. Mutations of this gene result in gelatinous drop-like corneal dystrophy, an autosomal recessive disorder characterized by severe corneal amyloidosis leading to blindness. Trop-2 expression was originally described in trophoblasts (placenta) and fetal tissues (e.g., lung). Later, its expression was also described in the normal stratified squamous epithelium of the skin, uterine cervix, esophagus, and tonsillar crypts. Trop-2 plays a role in tumor progression by actively interacting with several key molecular signaling pathways traditionally associated with cancer development and progression. Aberrant overexpression of Trop-2 has been described in several solid cancers, such as colorectal, renal, lung, and breast cancers. Trop-2 expression has also been described in some rare and aggressive malignancies, e.g., salivary duct, anaplastic thyroid, uterine/ovarian, and neuroendocrine prostate cancers. Protein Trop-2 is characterized by an amino acid sequence according to SEQ ID NO.: 6.

Trop-2 (SEQ ID NO.: 6):
MARGPGLAPPPLRLPLLLLVLAAVTGHTAAQDNCTCPTNKMTVCSPDGPGGRCQCRALGSGMAVDCSTLTSKCLL
LKARMSAPKNARTLVRPSEHALVDNDGLYDPDCDPEGRFKARQCNQTSVCWCVNSVGVRRTDKGDLSLRCDELVR
THHILIDLRHRPTAGAFNHSDLDAELRRLFRERYRLHPKFVAAVHYEQPTIQIELRQNTSQKAAGDVDIGDAAYY
FERDIKGESLFQGRGGLDLRVRGEPLQVERTLIYYLDEIPPKFSMKRLTAGLIAVIVVVVVALVAGMAVLVITNR
RKSGKYKKVEIKELGELRKEPSL

CD33 or Siglec-3 (sialic acid binding Ig-like lectin 3, SIGLEC3, SIGLEC-3, gp67, p67) is a transmembrane receptor expressed on cells of myeloid lineage. It is usually considered myeloid-specific, but it can also be found on some lymphoid cells. It binds sialic acids, therefore is a member of the SIGLEC family of lectins. The extracellular portion of this receptor contains two immunoglobulin domains (one IgV and one IgC2 domain), placing CD33 within the immunoglobulin superfamily. The intracellular portion of CD33 contains immunoreceptor tyrosine-based inhibitory motifs (ITIMs) that are implicated in inhibition of cellular activity. CD33 can be stimulated by any molecule with sialic acid residues such as glycoproteins or glycolipids. Upon binding, the immunoreceptor tyrosine-based inhibition motif (ITIM) of CD33, present on the cytosolic portion of the protein, is phosphorylated and acts as a docking site for Src homology 2 (SH2) domain-containing proteins like SHP phosphatases. This results in a cascade that inhibits phagocytosis in the cell CD33 is the target of gemtuzumab ozogamicin (trade name: Mylotarg^{®}; Pfizer/Wyeth-Ayerst Laboratories), an antibody-drug conjugate (ADC) for the treatment of patients with acute myeloid leukemia. The drug is a recombinant, humanized anti-CD33 monoclonal antibody (IgG4 κ antibody hP67.6) covalently attached to the cytotoxic antitumor antibiotic calicheamicin (*N*-acetyl-γ-calicheamicin) via a bifunctional linker (4-(4-acetylphenoxy)butanoic acid).[14] Several mechanisms of resistance to gemtuzumab ozogamicin have been elucidated. On September 1, 2017, the FDA approved Pfizer's Mylotarg. Gemtuzumab ozogamicin was initially approved by the U.S. Food and Drug Administration in 2000. However, during post marketing clinical trials researchers noticed a greater number of deaths in the group of patients who received gemtuzumab ozogamicin compared with those receiving chemotherapy alone. Based on these results, Pfizer voluntarily withdrew gemtuzumab ozogamicin from the market in mid-2010, but was reintroduced to the market in 2017. CD33 is also the target in Vadastuximab talirine (SGN-CD33A), a novel antibody-drug conjugate being developed by Seattle Genetics, utilizing this company's ADC technology. Protein CD33 is characterized by an amino acid sequence according to SEQ ID NO.: 7. 
CD33 (SEQ ID NO.: 7):
MPLLLLLPLLWAGALAMDPNFWLQVQESVTVQEGLCVLVPCTFFHPIPYYDKNSPVHGYWFREGAIISRDSPVAT
NKLDQEVQEETQGRFRLLGDPSRNNCSLSIVDARRRDNGSYFFRMERGSTKYSYKSPQLSVHVTDLTHRPKILIP
GTLEPGHSKNLTCSVSWACEQGTPPIFSWLSAAPTSLGPRTTHSSVLIITPRPQDHGTNLTCQVKFAGAGVTTER
TIQLNVTYVPQNPTTGIFPGDGSGKQETRAGVVHGAIGGAGVTALLALCLCLIFFIVKTHRRKAARTAVGRNDTH
PTTGSASPKHQKKSKLHGPTETSSCSGAAPTVEMDEELHYASLNFHGMNPSKDTSTEYSEVRTQ
 CLL-1 stands for C-type lectin domain family 12 member A. In the immunotherapy of acute myeloid leukemia (AML), CLL-1 becomes one of the target due to its high expression in AML cells while being absent in normal hematopoietic stem cells. CLL-1 is also expressed on the surface of leukemic stem cells (LSC), which possesses the ability to indefinitely self-renew, produce plenty of leukemic cells and are associated with leukemia relapses. Protein CLL-1 is characterized by an amino acid sequence according to SEQ ID NO.: 8.

CLL-1 (SEQ ID NO.: 8):
MSEEVTYADLQFQNSSEMEKIPEIGKFGEKAPPAPSHVWRPAALFLTLLCLLLLIGLGVLASMFHVTLKIEMKKM
NKLQNISEELQRNISLQLMSNMNISNKIRNLSTTLQTIATKLCRELYSKEQEHKCKPCPRRWIWHKDSCYFLSDD
VQTWQESKMACAAQNASLLKINNKNALEFIKSQSRSYDYWLGLSPEEDSTRGMRVDNIINSSAWVIRNAPDLNNM
YCGYINRLYVQYYHCTYKKRMICEKMANPVQLGSTYFREA

Proto-oncogene c-KIT is the gene encoding the receptor tyrosine kinase protein known as tyrosine-protein kinase KIT, CD117 (cluster of differentiation 117) or mast/stem cell growth factor receptor (SCFR). Multiple transcript variants encoding different isoforms have been found for this gene. KIT was first described by the German biochemist Axel Ullrich in 1987 as the cellular homolog of the feline sarcoma viral oncogene v-kit. KIT is a cytokine receptor expressed on the surface of hematopoietic stem cells as well as other cell types. Altered forms of this receptor may be associated with some types of cancer.[9] KIT is a receptor tyrosine kinase type III, which binds to stem cell factor, also known as "steel factor" or "c-kit ligand". When this receptor binds to stem cell factor (SCF) it forms a dimer that activates its intrinsic tyrosine kinase activity, that in turn phosphorylates and activates signal transduction molecules that propagate the signal in the cell. After activation, the receptor is ubiquitinated to mark it for transport to a lysosome and eventual destruction. Signaling through KIT plays a role in cell survival, proliferation, and differentiation. For instance, KIT signaling is required for melanocyte survival, and it is also involved in haematopoiesis and gametogenesis. Cluster of differentiation (CD) molecules are markers on the cell surface, as recognized by specific sets of antibodies, used to identify the cell type, stage of differentiation and activity of a cell. KIT is an important cell surface marker used to identify certain types of hematopoietic (blood) progenitors in the bone marrow. To be specific, hematopoietic stem cells (HSC), multipotent progenitors (MPP), and common myeloid progenitors (CMP) express high levels of KIT. Common lymphoid progenitors (CLP) express low surface levels of KIT. KIT also identifies the earliest thymocyte progenitors in the thymus-early T lineage progenitors (ETP/DN1) and DN2 thymocytes express high levels of c-Kit. It is also a marker for mouse prostate stem cells. In addition, mast cells, melanocytes in the skin, and interstitial cells of Cajal in the digestive tract express KIT. In humans, expression of c-kit in helper-like innate lymphoid cells (ILCs) which lack the expression of CRTH2 (CD294) is used to mark the ILC3 population. CD117/c-KIT is expressed not only by bone marrow-derived stem cells, but also by those found in other adult organs, such as the prostate, liver, and heart, suggesting that SCF/c-KIT signaling pathways may contribute to stemness in some organs. Additionally, c-KIT has been associated with numerous biological processes in other cell types. For example, c-KIT signaling, has been shown to regulate oogenesis, folliculogenesis, and spermatogenesis, playing important roles in female and male fertility. Activating mutations in this gene are associated with gastrointestinal stromal tumors, testicular seminoma, mast cell disease, melanoma, acute myeloid leukemia, while inactivating mutations are associated with the genetic defect piebaldism. c-KIT plays an important role in regulating many mechanisms leading to tumor formation and progression of carcinomas. c-KIT has been proposed as a regulator of stemness in several cancers. Its expression has been linked to cancer stemness in ovarian cancer cells, colon cancer cells, non-small cell lung cancer cells, and prostate cancer cells. c-KIT has also been linked to the epithelial-mesenchymal transition (EMT), which is important for tumor aggressiveness and metastatic potential. Ectopic expression of c-KIT and EMT have been linked in denoid cystic carcinoma of the salivary gland, thymic carcinomas, ovarian cancer cells, and prostate cancer cells. Several lines of evidence suggest that SCF/c-KIT signaling plays an important role in the tumor microenvironment. For example, in mice high levels of c-KIT in mast cells as well as its presence in the tumor microenvironment promote angiogenesis, leading to increased tumor growth and metastasis. Protein c-KIT is characterized by an amino acid sequence according to SEQ ID NO.: 9.

c-KIT (SEQ ID NO.: 9):















The interleukin-3 receptor (CD123) is a molecule found on cells which helps transmit the signal of interleukin-3, a soluble cytokine important in the immune system. The gene coding for the receptor is located in the pseudoautosomal region of the X and Y chromosomes. The receptor belongs to the type I cytokine receptor family and is a heterodimer with a unique alpha chain paired with the common beta (beta c or CD131) subunit. The gene for the alpha subunit is 40 kilobases long and has 12 exons. The receptor, found on pluripotent progenitor cells, induces tyrosine phosphorylation within the cell and promotes proliferation and differentiation within the hematopoietic cell lines. It can be found on basophils and pDCs as well as some cDCs among peripheral blood mononuclear cells. CD123 is expressed across acute myeloid leukemia (AML) subtypes, including leukemic stem cells. Protein CD123 is characterized by an amino acid sequence according to SEQ ID NO.: 10.

CD123 (SEQ ID NO.: 10):
MKKEHVLHCQFSAWYPFFRGVTIKSVILPLPQNVKDYLLDDGTLVVSGRDDPPTHSQPDSDDEAEEIQWSDDENT
ATLTAPEFPEFATKVQEAINSLGGSVFPKLNWSAPRDAYWIAMNSSLKCKTLSDIFLLFKSSDFITRDFTQPFIH
CTDDSPDPCIEYELVLRKWCELIPGAEFRCFVKENKLIGISQRDYTQYYDHISKQKEEIRRCIQDFFKKHIQYKF
LDEDFVFDIYRDSRGKVWLIDFNPFGEVTDSLLFTWEELISENNLNGDFSEVDAQEQDSPAFRCTNSEVTVQPSP
YLSYRLPKDFVDLSTGEDAHKLIDFLKLKRNQQEDD

The epidermal growth factor receptor (EGFR; ErbB-1; HER1 in humans) is a transmembrane protein that is a receptor for members of the epidermal growth factor family (EGF family) of extracellular protein ligands. The epidermal growth factor receptor is a member of the ErbB family of receptors, a subfamily of four closely related receptor tyrosine kinases: EGFR (ErbB-1), HER2/neu (ErbB-2), Her 3 (ErbB-3) and Her 4 (ErbB-4). In many cancer types, mutations affecting EGFR expression or activity could result in cancer. Epidermal growth factor and its receptor was discovered by Stanley Cohen of Vanderbilt University. Cohen shared the 1986 Nobel Prize in Medicine with Rita Levi-Montalcini for their discovery of growth factors. Deficient signaling of the EGFR and other receptor tyrosine kinases in humans is associated with diseases such as Alzheimer's, while over-expression is associated with the development of a wide variety of tumors. Interruption of EGFR signalling, either by blocking EGFR binding sites on the extracellular domain of the receptor or by inhibiting intracellular tyrosine kinase activity, can prevent the growth of EGFR-expressing tumours and improve the patient's condition. Protein EGFR is characterized by an amino acid sequence according to SEQ ID NO.: 11.

EGFR (SEQ ID NO.: 11):



















Folate receptor 1 (Folate receptor alpha, FOLR1 or FR-o) is a protein that in humans is encoded by the FOLR1 gene. The protein encoded by this gene is a member of the folate receptor (FOLR) family. Members of this family have a high affinity for folic acid and for several reduced folic acid derivatives, and mediate delivery of 5-methyltetrahydrofolate to the interior of cells. This receptor is responsible for binding to folic acid and its derivatives, which becomes crucial during fetal development. By adding folate supplementation during pregnancy, neural tube defects in the fetus are prevented. Folate derivatives are necessary for important metabolic processes such as DNA, protein and lipid methylation. More importantly, folate plays a major role in DNA replication and cell division, which are common characteristics of rapid growth. Even though it is unclear how folate affects neural tube formation, scientists are certain that without appropriate folate levels, neural tube defects can develop through human and mice studies. Neural tube defects refer to the improper development of the neural tube by not being sealed correctly. This results in exencephaly or spina bifida, both nervous system abnormalities. This gene is composed of 7 exons; exons 1 through 4 encode the 5' UTR and exons 4 through 7 encode the open reading frame. Due to the presence of 2 promoters, multiple transcription start sites, and alternative splicing of exons, several transcript variants are derived from this gene. These variants differ in the lengths of 5' and 3' UTR, but they encode an identical amino acid sequence. FRo, due to its high expression in some tumors, is an attractive therapeutic target for the development of novel anti-cancer agents in order to limit toxic side-effects on off-target tissues. FRa can be overexpressed by a number of epithelial-derived tumors including ovarian, breast, renal, lung, colorectal, and brain. According to a review published in 2020, elevated expression of FRa was noted in mesotheliomas (72-100% of cases), triple-negative breast cancer (35-68% of cases) and epithelial ovarian cancer (76-89% of cases). Protein FR-o is characterized by an amino acid sequence according to SEQ ID NO.: 12.

FR-α (SEQ ID NO.: 12):

MAQRMTTQLLLLLVWVAVVGEAQTRIAWARTELLNVCMNAKHHKEKPGPEDKLHEQCRPWRKNACCSTNTSQEAH
KDVSYLYRFNWNHCGEMAPACKRHFIQDTCLYECSPNLGPWIQQVDQSWRKERVLNVPLCKEDCEQWWEDCRTSY
TCKSNWHKGWNWTSGFNKCAVGAACQPFHFYFPTPTVLCNEIWTHSYKVSNYSRGSGRCIQMWFDPAQGNPNEEV
ARFYAAAMSGAGPWAAWPFLLSLALMLLWLLS

Mucin-16 (MUC16) also known as Ovarian cancer-related tumor marker CA125 is a protein that in humans is encoded by the MUC16 gene. MUC16 is a member of the mucin family glycoproteins. MUC16 has found application as a tumor marker or biomarker that may be elevated in the blood of some patients with specific types of cancers, most notably ovarian cancer, or other conditions that are benign. Protein MUC16 is characterized by an amino acid sequence according to SEQ ID NO.: 13.

MUC16 (SEQ ID NO.: 13):
MLKPSGLPGSSSPTRSLMTGSRSTKATPEMDSGLTGATLSPKTSTGAIVVTEHTLPFTSPDKTLASPTSSVVGRT
TQSLGVMSSALPESTSRGMTHSEQRTSPSLSPQVNGTPSRNYPATSMVSGLSSPRTRTSSTEGNFTKEASTYTLT
VETTSGPVTEKYTVPTETSTTEGDSTETPWDTRYIPVKITSPMKTFADSTASKENAPVSMTPAETTVTDSHTPGR
TNPSFGTLYSSFLDLSPKGTPNSRGETSLELILSTTGYPFSSPEPGSAGHSRISTSAPLSSSASVLDNKISETSI
FSGQSLTSPLSPGVPEARASTMPNSAIPFSMTLSNAETSAERVRSTISSLGTPSISTKQTAETILTFHAFAETMD
IPSTHIAKTLASEWLGSPGTLGGTSTSALTTTSPSTTLVSEETNTHHSTSGKETEGTLNTSMTPLETSAPGEESE
MTATLVPTLGFTTLDSKIRSPSQVSSSHPTRELRTTGSTSGRQSSSTAAHGSSDILRATTSSTSKASSWTSESTA
QQFSEPQHTQWVETSPSMKTERPPASTSVAAPITTSVPSVVSGFTTLKTSSTKGIWLEETSADTLIGESTAGPTT
HQFAVPTGISMTGGSSTRGSQGTTHLLTRATASSETSADLTLATNGVPVSVSPAVSKTAAGSSPPGGTKPSYTMV
SSVIPETSSLQSSAFREGTSLGLTPLNTRHPFSSPEPDSAGHTKISTSIPLLSSASVLEDKVSATSTFSHHKATS
SITTGTPEISTKTKPSSAVLSSMTLSNAATSPERVRNATSPLTHPSPSGEETAGSVLTLSTSAETTDSPNIHPTG
TLTSESSESPSTLSLPSVSGVKTTFSSSTPSTHLFTSGEETEETSNPSVSQPETSVSRVRTTLASTSVPTPVFPT
MDTWPTRSAQFSSSHLVSELRATSSTSVTNSTGSALPKISHLTGTATMSQTNRDTFNDSAAPQSTTWPETSPRFK
TGLPSATTTVSTSATSLSATVMVSKFTSPATSSMEATSIREPSTTILTTETTNGPGSMAVASTNIPIGKGYITEG
RLDTSHLPIGTTASSETSMDFTMAKESVSMSVSPSQSMDAAGSSTPGRTSQFVDTFSDDVYHLTSREITIPRDGT
SSALTPQMTATHPPSPDPGSARSTWLGILSSSPSSPTPKVTMSSTFSTQRVTTSMIMDTVETSRWNMPNLPSTTS
LTPSNIPTSGAIGKSTLVPLDTPSPATSLEASEGGLPTLSTYPESTNTPSIHLGAHASSESPSTIKLTMASVVKP
GSYTPLTFPSIETHIHVSTARMAYSSGSSPEMTAPGETNTGSTWDPTTYITTTDPKDTSSAQVSTPHSVRTLRTT
ENHPKTESATPAAYSGSPKISSSPNLTSPATKAWTITDTTEHSTQLHYTKLAEKSSGFETQSAPGPVSVVIPTSP
TIGSSTLELTSDVPGEPLVLAPSEQTTITLPMATWLSTSLTEEMASTDLDISSPSSPMSTFAIFPPMSTPSHELS
KSEADTSAIRNTDSTTLDQHLGIRSLGRTGDLTTVPITPLTTTWTSVIEHSTQAQDTLSATMSPTHVTQSLKDQT
SIPASASPSHLTEVYPELGTQGRSSSEATTFWKPSTDTLSREIETGPTNIQSTPPMDNTTTGSSSSGVTLGIAHL
PIGTSSPAETSTNMALERRSSTATVSMAGTMGLLVTSAPGRSISQSLGRVSSVLSESTTEGVTDSSKGSSPRLNT
QGNTALSSSLEPSYAEGSQMSTSIPLTSSPTTPDVEFIGGSTFWTKEVTTVMTSDISKSSARTESSSATLMSTAL
GSTENTGKEKLRTASMDLPSPTPSMEVTPWISLTLSNAPNTTDSLDLSHGVHTSSAGTLATDRSLNTGVTRASRL
ENGSDTSSKSLSMGNSTHTSMTYTEKSEVSSSIHPRPETSAPGAETTLTSTPGNRAISLTLPFSSIPVEEVISTG
ITSGPDINSAPMTHSPITPPTIVWTSTGTIEQSTQPLHAVSSEKVSVQTQSTPYVNSVAVSASPTHENSVSSGSS
TSSPYSSASLESLDSTISRRNAITSWLWDLTTSLPTTTWPSTSLSEALSSGHSGVSNPSSTTTEFPLFSAASTSA

AKQRNPETETHGPQNTAASTLNTDASSVTGLSETPVGASISSEVPLPMAITSRSDVSGLTSESTANPSLGTASSA
GTKLTRTISLPTSESLVSFRMNKDPWTVSIPLGSHPTTNTETSIPVNSAGPPGLSTVASDVIDTPSDGAESIPTV
SFSPSPDTEVTTISHFPEKTTHSFRTISSLTHELTSRVTPIPGDWMSSAMSTKPTGASPSITLGERRTITSAAPT
TSPIVLTASFTETSTVSLDNETTVKTSDILDARKTNELPSDSSSSSDLINTSIASSTMDVTKTASISPTSISGMT
ASSSPSLFSSDRPQVPTSTTETNTATSPSVSSNTYSLDGGSNVGGTPSTLPPFTITHPVETSSALLAWSRPVRTF
STMVSTDTASGENPTSSNSVVTSVPAPGTWTSVGSTTDLPAMGFLKTSPAGEAHSLLASTIEPATAFTPHLSAAV
VTGSSATSEASLLTTSESKAIHSSPQTPTTPTSGANWETSATPESLLVVTETSDTTLTSKILVTDTILFSTVSTP
PSKFPSTGTLSGASFPTLLPDTPAIPLTATEPTSSLATSFDSTPLVTIASDSLGTVPETTLTMSETSNGDALVLK
TVSNPDRSIPGITIQGVTESPLHPSSTSPSKIVAPRNTTYEGSITVALSTLPAGTTGSLVFSQSSENSETTALVD
SSAGLERASVMPLTTGSQGMASSGGIRSGSTHSTGTKTFSSLPLTMNPGEVTAMSEITTNRLTATQSTAPKGIPV
KPTSAESGLLTPVSASSSPSKAFASLTTAPPTWGIPQSTLTFEFSEVPSLDTKSASLPTPGQSLNTIPDSDASTA
SSSLSKSPEKNPRARMMTSTKAISASSFQSTGFTETPEGSASPSMAGHEPRVPTSGTGDPRYASESMSYPDPSKA
SSAMTSTSLASKLTTLFSTGQAARSGSSSSPISLSTEKETSFLSPTASTSRKTSLFLGPSMARQPNILVHLQTSA
LTLSPTSTLNMSQEEPPELTSSQTIAEEEGTTAETQTLTFTPSETPTSLLPVSSPTEPTARRKSSPETWASSISV
PAKTSLVETTDGTLVTTIKMSSQAAQGNSTWPAPAEETGSSPAGTSPGSPEMSTTLKIMSSKEPSISPEIRSTVR
NSPWKTPETTVPMETTVEPVTLQSTALGSGSTSISHLPTGTTSPTKSPTENMLATERVSLSPSPPEAWTNLYSGT
PGGTRQSLATMSSVSLESPTARSITGTGQQSSPELVSKTTGMEFSMWHGSTGGTTGDTHVSLSTSSNILEDPVTS
PNSVSSLTDKSKHKTETWVSTTAIPSTVLNNKIMAAEQQTSRSVDEAYSSTSSWSDQTSGSDITLGASPDVTNTL
YITSTAQTTSLVSLPSGDQGITSLTNPSGGKTSSASSVTSPSIGLETLRANVSAVKSDIAPTAGHLSQTSSPAEV
SILDVTTAPTPGISTTITTMGTNSISTTTPNPEVGMSTMDSTPATERRTTSTEHPSTWSSTAASDSWTVTDMTSN
LKVARSPGTISTMHTTSFLASSTELDSMSTPHGRITVIGTSLVTPSSDASAVKTETSTSERTLSPSDTTASTPIS
TFSRVQRMSISVPDILSTSWTPSSTEAEDVPVSMVSTDHASTKTDPNTPLSTFLFDSLSTLDWDTGRSLSSATAT
TSAPQGATTPQELTLETMISPATSQLPFSIGHITSAVTPAAMARSSGVTFSRPDPTSKKAEQTSTQLPTTTSAHP
GQVPRSAATTLDVIPHTAKTPDATFQRQGQTALTTEARATSDSWNEKEKSTPSAPWITEMMNSVSEDTIKEVTSS
SSVLRTLNTLDINLESGTTSSPSWKSSPYERIAPSESTTDKEAIHPSTNTVETTGWVTSSEHASHSTIPAHSASS
KLTSPVVTTSTREQAIVSMSTTTWPESTRARTEPNSFLTIELRDVSPYMDTSSTTQTSIISSPGSTAITKGPRTE
ITSSKRISSSFLAQSMRSSDSPSEAITRLSNFPAMTESGGMILAMQTSPPGATSLSAPTLDTSATASWTGTPLAT
TQRFTYSEKTTLFSKGPEDTSQPSPPSVEETSSSSSLVPIHATTSPSNILLTSQGHSPSSTPPVTSVFLSETSGL
GKTTDMSRISLEPGTSLPPNLSSTAGEALSTYEASRDTKAIHHSADTAVTNMEATSSEYSPIPGHTKPSKATSPL
VTSHIMGDITSSTSVFGSSETTEIETVSSVNQGLQERSTSQVASSATETSTVITHVSSGDATTHVTKTQATFSSG
TSISSPHQFITSTNTFTDVSTNPSTSLIMTESSGVTITTQTGPTGAATQGPYLLDTSTMPYLTETPLAVTPDFMQ
SEKTTLISKGPKDVSWTSPPSVAETSYPSSLTPFLVTTIPPATSTLQGQHTSSPVSATSVLTSGLVKTTDMLNTS
MEPVTNSPQNLNNPSNEILATLAATTDIETIHPSINKAVTNMGTASSAHVLHSTLPVSSEPSTATSPMVPASSMG
DALASISIPGSETTDIEGEPTSSLTAGRKENSTLQEMNSTTESNIILSNVSVGAITEATKMEVPSFDATFIPTPA
QSTKFPDIFSVASSRLSNSPPMTISTHMTTTQTGSSGATSKIPLALDTSTLETSAGTPSVVTEGFAHSKITTAMN
NDVKDVSQTNPPFQDEASSPSSQAPVLVTTLPSSVAFTPQWHSTSSPVSMSSVLTSSLVKTAGKVDTSLETVTSS
PQSMSNTLDDISVTSAATTDIETTHPSINTVVTNVGTTGSAFESHSTVSAYPEPSKVTSPNVTTSTMEDTTISRS
IPKSSKTTRTETETTSSLTPKLRETSISQEITSSTETSTVPYKELTGATTEVSRTDVTSSSSTSFPGPDQSTVSL
DISTETNTRLSTSPIMTESAEITITTQTGPHGATSQDTFTMDPSNTTPQAGIHSAMTHGFSQLDVTTLMSRIPQD
VSWTSPPSVDKTSSPSSFLSSPAMTTPSLISSTLPEDKLSSPMTSLLTSGLVKITDILRTRLEPVTSSLPNFSST
SDKILATSKDSKDTKEIFPSINTEETNVKANNSGHESHSPALADSETPKATTQMVITTTVGDPAPSTSMPVHGSS

ETTNIKREPTYFLTPRLRETSTSQESSFPTDTSFLLSKVPTGTITEVSSTGVNSSSKISTPDHDKSTVPPDTFTG
EIPRVFTSSIKTKSAEMTITTQASPPESASHSTLPLDTSTTLSQGGTHSTVTQGFPYSEVTTLMGMGPGNVSWMT
TPPVEETSSVSSLMSSPAMTSPSPVSSTSPQSIPSSPLPVTALPTSVLVTTTDVLGTTSPESVTSSPPNLSSITH
ERPATYKDTAHTEAAMHHSTNTAVTNVGTSGSGHKSQSSVLADSETSKATPLMSTTSTLGDTSVSTSTPNISQTN
QIQTEPTASLSPRLRESSTSEKTSSTTETNTAFSYVPTGAITQASRTEISSSRTSISDLDRPTIAPDISTGMITR
LFTSPIMTKSAEMTVTTQTTTPGATSQGILPWDTSTTLFQGGTHSTVSQGFPHSEITTLRSRTPGDVSWMTTPPV
EETSSGFSLMSPSMTSPSPVSSTSPESIPSSPLPVTALLTSVLVTTTNVLGTTSPEPVTSSPPNLSSPTQERLTT
YKDTAHTEAMHASMHTNTAVANVGTSISGHESQSSVPADSHTSKATSPMGITFAMGDTSVSTSTPAFFETRIQTE
STSSLIPGLRDTRTSEEINTVTETSTVLSEVPTTTTTEVSRTEVITSSRTTISGPDHSKMSPYISTETITRLSTF
PFVTGSTEMAITNQTGPIGTISQATLTLDTSSTASWEGTHSPVTQRFPHSEETTTMSRSTKGVSWQSPPSVEETS
SPSSPVPLPAITSHSSLYSAVSGSSPTSALPVTSLLTSGRRKTIDMLDTHSELVTSSLPSASSFSGEILTSEAST
NTETIHFSENTAETNMGTTNSMHKLHSSVSIHSQPSGHTPPKVTGSMMEDAIVSTSTPGSPETKNVDRDSTSPLT
PELKEDSTALVMNSTTESNTVFSSVSLDAATEVSRAEVTYYDPTFMPASAQSTKSPDISPEASSSHSNSPPLTIS
THKTIATQTGPSGVTSLGQLTLDTSTIATSAGTPSARTQDFVDSETTSVMNNDLNDVLKTSPFSAEEANSLSSQA
PLLVTTSPSPVTSTLQEHSTSSLVSVTSVPTPTLAKITDMDTNLEPVTRSPQNLRNTLATSEATTDTHTMHPSIN
TAVANVGTTSSPNEFYFTVSPDSDPYKATSAVVITSTSGDSIVSTSMPRSSAMKKIESETTFSLIFRLRETSTSQ
KIGSSSDTSTVFDKAFTAATTEVSRTELTSSSRTSIQGTEKPTMSPDTSTRSVTMLSTFAGLTKSEERTIATQTG
PHRATSQGTLTWDTSITTSQAGTHSAMTHGFSQLDLSTLTSRVPEYISGTSPPSVEKTSSSSSLLSLPAITSPSP
VPTTLPESRPSSPVHLTSLPTSGLVKTTDMLASVASLPPNLGSTSHKIPTTSEDIKDTEKMYPSTNIAVTNVGTT
TSEKESYSSVPAYSEPPKVTSPMVTSFNIRDTIVSTSMPGSSEITRIEMESTFSLAHGLKGTSTSQDPIVSTEKS
AVLHKLTTGATETSRTEVASSRRTSIPGPDHSTESPDISTEVIPSLPISLGITESSNMTIITRTGPPLGSTSQGT
FTLDTPTTSSRAGTHSMATQEFPHSEMTTVMNKDPEILSWTIPPSIEKTSFSSSLMPSPAMTSPPVSSTLPKTIH
TTPSPMTSLLTPSLVMTTDTLGTSPEPTTSSPPNLSSTSHEILTTDEDTTAIEAMHPSTSTAATNVETTSSGHGS
QSSVLADSEKTKATAPMDTTSTMGHTTVSTSMSVSSETTKIKRESTYSLTPGLRETSISQNASFSTDTSIVLSEV
PTGTTAEVSRTEVTSSGRTSIPGPSQSTVLPEISTRTMTRLFASPTMTESAEMTIPTQTGPSGSTSQDTLTLDTS
TTKSQAKTHSTLTQRFPHSEMTTLMSRGPGDMSWQSSPSLENPSSLPSLLSLPATTSPPPISSTLPVTISSSPLP
VTSLLTSSPVTTTDMLHTSPELVTSSPPKLSHTSDERLTTGKDTTNTEAVHPSTNTAASNVEIPSSGHESPSSAL
ADSETSKATSPMFITSTQEDTTVAISTPHFLETSRIQKESISSLSPKLRETGSSVETSSAIETSAVLSEVSIGAT
TEISRTEVTSSSRTSISGSAESTMLPEISTTRKIIKFPTSPILAESSEMTIKTQTSPPGSTSESTFTLDTSTTPS
LVITHSTMTQRLPHSEITTLVSRGAGDVPRPSSLPVEETSPPSSQLSLSAMISPSPVSSTLPASSHSSSASVTSL
LTPGQVKTTEVLDASAEPETSSPPSLSSTSVEILATSEVTTDTEKIHPFSNTAVTKVGTSSSGHESPSSVLPDSE
TTKATSAMGTISIMGDTSVSTLTPALSNTRKIQSEPASSLTTRLRETSTSEETSLATEANTVLSKVSTGATTEVS
RTEAISFSRTSMSGPEQSTMSQDISIGTIPRISASSVLTESAKMTITTQTGPSESTLESTLNLNTATTPSWVETH
SIVIQGFPHPEMTTSMGRGPGGVSWPSPPFVKETSPPSSPLSLPAVTSPHPVSTTFLAHIPPSPLPVTSLLTSGP
ATTTDILGTSTEPGTSSSSSLSTTSHERLTTYKDTAHTEAVHPSTNTGGTNVATTSSGYKSQSSVLADSSPMCTT
STMGDTSVLTSTPAFLETRRIQTELASSLTPGLRESSGSEGTSSGTKMSTVLSKVPTGATTEISKEDVTSIPGPA
QSTISPDISTRTVSWFSTSPVMTESAEITMNTHTSPLGATTQGTSTLDTSSTTSLTMTHSTISQGFSHSQMSTLM
RRGPEDVSWMSPPLLEKTRPSFSLMSSPATTSPSPVSSTLPESISSSPLPVTSLLTSGLAKTTDMLHKSSEPVTN
SPANLSSTSVEILATSEVTTDTEKTHPSSNRTVTDVGTSSSGHESTSFVLADSQTSKVTSPMVITSTMEDTSVST
STPGFFETSRIQTEPTSSLTLGLRKTSSSEGTSLATEMSTVLSGVPTGATAEVSRTEVTSSSRTSISGFAQLTVS
PETSTETITRLPTSSIMTESAEMMIKTQTDPPGSTPESTHTVDISTTPNWVETHSTVTQRFSHSEMTTLVSRSPG

DMLWPSQSSVEETSSASSLLSLPATTSPSPVSSTLVEDFPSASLPVTSLLNPGLVITTDRMGISREPGTSSTSNL
SSTSHERLTTLEDTVDTEDMQPSTHTAVTNVRTSISGHESQSSVLSDSETPKATSPMGTTYTMGETSVSISTSDF
FETSRIQIEPTSSLTSGLRETSSSERISSATEGSTVLSEVPSGATTEVSRTEVISSRGTSMSGPDQFTISPDIST
EAITRLSTSPIMTESAESAITIETGSPGATSEGTLTLDTSTTTFWSGTHSTASPGFSHSEMTTLMSRTPGDVPWP
SLPSVEEASSVSSSLSSPAMTSTSFFSTLPESISSSPHPVTALLTLGPVKTTDMLRTSSEPETSSPPNLSSTSAE
ILATSEVTKDREKIHPSSNTPVVNVGTVIYKHLSPSSVLADLVTTKPTSPMATTSTLGNTSVSTSTPAFPETMMT
QPTSSLTSGLREISTSQETSSATERSASLSGMPTGATTKVSRTEALSLGRTSTPGPAQSTISPEISTETITRIST
PLTTTGSAEMTITPKTGHSGASSQGTFTLDTSSRASWPGTHSAATHRSPHSGMTTPMSRGPEDVSWPSRPSVEKT
SPPSSLVSLSAVTSPSPLYSTPSESSHSSPLRVTSLFTPVMMKTTDMLDTSLEPVTTSPPSMNITSDESLATSKA
TMETEAIQLSENTAVTQMGTISARQEFYSSYPGLPEPSKVTSPVVTSSTIKDIVSTTIPASSEITRIEMESTSTL
TPTPRETSTSQEIHSATKPSTVPYKALTSATIEDSMTQVMSSSRGPSPDQSTMSQDISTEVITRLSTSPIKTEST
EMTITTQTGSPGATSRGTLTLDTSTTFMSGTHSTASQGFSHSQMTALMSRTPGDVPWLSHPSVEEASSASFSLSS
PVMTSSSPVSSTLPDSIHSSSLPVTSLLTSGLVKTTELLGTSSEPETSSPPNLSSTSAEILAITEVTTDTEKLEM
TNVVTSGYTHESPSSVLADSVTTKATSSMGITYPTGDTNVLTSTPAFSDTSRIQTKSKLSLTPGLMETSISEETS
SATEKSTVLSSVPTGATTEVSRTEAISSSRTSIPGPAQSTMSSDTSMETITRISTPLTRKESTDMAITPKTGPSG
ATSQGTFTLDSSSTASWPGTHSATTQRFPQSVVTTPMSRGPEDVSWPSPLSVEKNSPPSSLVSSSSVTSPSPLYS
TPSGSSHSSPVPVTSLFTSIMMKATDMLDASLEPETTSAPNMNITSDESLAASKATTETEAIHVFENTAASHVET
TSATEELYSSSPGFSEPTKVISPVVTSSSIRDNMVSTTMPGSSGITRIEIESMSSLTPGLRETRTSQDITSSTET
STVLYKMPSGATPEVSRTEVMPSSRTSIPGPAQSTMSLDISDEVVTRLSTSPIMTESAEITITTQTGYSLATSQV
TLPLGTSMTFLSGTHSTMSQGLSHSEMTNLMSRGPESLSWTSPRFVETTRSSSSLTSLPLTTSLSPVSSTLLDSS
PSSPLPVTSLILPGLVKTTEVLDTSSEPKTSSSPNLSSTSVEIPATSEIMTDTEKIHPSSNTAVAKVRTSSSVHE
SHSSVLADSETTITIPSMGITSAVDDTTVFTSNPAFSETRRIPTEPTFSLTPGFRETSTSEETTSITETSAVLYG
VPTSATTEVSMTEIMSSNRIHIPDSDQSTMSPDIITEVITRLSSSSMMSESTQMTITTQKSSPGATAQSTLTLAT
TTAPLARTHSTVPPRFLHSEMTTLMSRSPENPSWKSSLFVEKTSSSSSLLSLPVTTSPSVSSTLPQSIPSSSFSV
TSLLTPGMVKTTDTSTEPGTSLSPNLSGTSVEILAASEVTTDTEKIHPSSSMAVTNVGTTSSGHELYSSVSIHSE
PSKATYPVGTPSSMAETSISTSMPANFETTGFEAEPFSHLTSGFRKTNMSLDTSSVTPTNTPSSPGSTHLLQSSK
TDFTSSAKTSSPDWPPASQYTEIPVDIITPFNASPSITESTGITSFPESRFTMSVTESTHHLSTDLLPSAETIST
GTVMPSLSEAMTSFATTGVPRAISGSGSPFSRTESGPGDATLSTIAESLPSSTPVPFSSSTFTTTDSSTIPALHE
ITSSSATPYRVDTSLGTESSTTEGRLVMVSTLDTSSQPGRTSSSPILDTRMTESVELGTVTSAYQVPSLSTRLTR
TDGIMEHITKIPNEAAHRGTIRPVKGPQTSTSPASPKGLHTGGTKRMETTTTALKTTTTALKTTSRATLTTSVYT
PTLGTLTPLNASMQMASTIPTEMMITTPYVFPDVPETTSSLATSLGAETSTALPRTTPSVFNRESETTASLVSRS
GAERSPVIQTLDVSSSEPDTTASWVIHPAETIPTVSKTTPNFFHSELDTVSSTATSHGADVSSAIPTNISPSELD
ALTPLVTISGTDTSTTFPTLTKSPHETETRTTWLTHPAETSSTIPRTIPNFSHHESDATPSIATSPGAETSSAIP
IMTVSPGAEDLVTSQVTSSGTDRNMTIPTLTLSPGEPKTIASLVTHPEAQTSSAIPTSTISPAVSRLVTSMVTSL
AAKTSTTNRALTNSPGEPATTVSLVTHPAQTSPTVPWTTSIFFHSKSDTTPSMTTSHGAESSSAVPTPTVSTEVP
GVVTPLVTSSRAVISTTIPILTLSPGEPETTPSMATSHGEEASSAIPTPTVSPGVPGVVTSLVTSSRAVTSTTIP
ILTFSLGEPETTPSMATSHGTEAGSAVPTVLPEVPGMVTSLVASSRAVTSTTLPTLTLSPGEPETTPSMATSHGA
EASSTVPTVSPEVPGVVTSLVTSSSGVNSTSIPTLILSPGELETTPSMATSHGAEASSAVPTPTVSPGVSGVVTP
LVTSSRAVTSTTIPILTLSSSEPETTPSMATSHGVEASSAVLTVSPEVPGMVTSLVTSSRAVTSTTIPTLTISSD
EPETTTSLVTHSEAKMISAIPTLAVSPTVQGLVTSLVTSSGSETSAFSNLTVASSQPETIDSWVAHPGTEASSVV
PTLTVSTGEPFTNISLVTHPAESSSTLPRTTSRFSHSELDTMPSTVTSPEAESSSAISTTISPGIPGVLTSLVTS

SGRDISATFPTVPESPHESEATASWVTHPAVTSTTVPRTTPNYSHSEPDTTPSIATSPGAEATSDFPTITVSPDV
PDMVTSQVTSSGTDTSITIPTLTLSSGEPETTTSFITYSETHTSSAIPTLPVSPGASKMLTSLVISSGTDSTTTF
PTLTETPYEPETTAIQLIHPAETNTMVPRTTPKFSHSKSDTTLPVAITSPGPEASSAVSTTTISPDMSDLVTSLV
PSSGTDTSTTFPTLSETPYEPETTATWLTHPAETSTTVSGTIPNFSHRGSDTAPSMVTSPGVDTRSGVPTTTIPP
SIPGVVTSQVTSSATDTSTAIPTLTPSPGEPETTASSATHPGTQTGFTVPIRTVPSSEPDTMASWVTHPPQTSTP
VSRTTSSFSHSSPDATPVMATSPRTEASSAVLTTISPGAPEMVTSQITSSGAATSTTVPTLTHSPGMPETTALLS
THPRTETSKTFPASTVFPQVSETTASLTIRPGAETSTALPTQTTSSLFTLLVTGTSRVDLSPTASPGVSAKTAPL
STHPGTETSTMIPTSTLSLGLLETTGLLATSSSAETSTSTLTLTVSPAVSGLSSASITTDKPQTVTSWNTETSPS
VTSVGPPEFSRTVTGTTMTLIPSEMPTPPKTSHGEGVSPTTILRTTMVEATNLATTGSSPTVAKTTTTFNTLAGS
LFTPLTTPGMSTLASESVTSRTSYNHRSWISTTSSYNRRYWTPATSTPVTSTFSPGISTSSIPSSTAATVPFMVP
FTLNFTITNLQYEEDMRHPGSRKFNATERELQGLLKPLFRNSSLEYLYSGCRLASLRPEKDSSATAVDAICTHRP
DPEDLGLDRERLYWELSNLTNGIQELGPYTLDRNSLYVNGFTHRSSMPTTSTPGTSTVDVGTSGTPSSSPSPTTA
GPLLMPFTLNFTITNLQYEEDMRRTGSRKFNTMESVLQGLLKPLFKNTSVGPLYSGCRLTLLRPEKDGAATGVDA
ICTHRLDPKSPGLNREQLYWELSKLTNDIEELGPYTLDRNSLYVNGFTHQSSVSTTSTPGTSTVDLRTSGTPSSL
SSPTIMAAGPLLVPFTLNFTITNLQYGEDMGHPGSRKFNTTERVLQGLLGPIFKNTSVGPLYSGCRLTSLRSEKD
GAATGVDAICIHHLDPKSPGLNRERLYWELSQLTNGIKELGPYTLDRNSLYVNGFTHRTSVPTSSTPGTSTVDLG
TSGTPFSLPSPATAGPLLVLFTLNFTITNLKYEEDMHRPGSRKFNTTERVLQTLLGPMFKNTSVGLLYSGCRLTL
LRSEKDGAATGVDAICTHRLDPKSPGVDREQLYWELSQLTNGIKELGPYTLDRNSLYVNGFTHWIPVPTSSTPGT
STVDLGSGTPSSLPSPTTAGPLLVPFTLNFTITNLKYEEDMHCPGSRKFNTTERVLQSLLGPMFKNTSVGPLYSG
CRLTLLRSEKDGAATGVDAICTHRLDPKSPGVDREQLYWELSQLTNGIKELGPYTLDRNSLYVNGFTHQTSAPNT
STPGTSTVDLGTSGTPSSLPSPTSAGPLLVPFTLNFTITNLQYEEDMHHPGSRKFNTTERVLQGLLGPMFKNTSV
GLLYSGCRLTLLRPEKNGAATGMDAICSHRLDPKSPGLNREQLYWELSQLTHGIKELGPYTLDRNSLYVNGFTHR
SSVAPTSTPGTSTVDLGTSGTPSSLPSPTTAVPLLVPFTLNFTITNLQYGEDMRHPGSRKFNTTERVLQGLLGPL
FKNSSVGPLYSGCRLISLRSEKDGAATGVDAICTHHLNPQSPGLDREQLYWQLSQMTNGIKELGPYTLDRNSLYV
NGFTHRSSGLTTSTPWTSTVDLGTSGTPSPVPSPTTTGPLLVPFTLNFTITNLQYEENMGHPGSRKFNITESVLQ
GLLKPLFKSTSVGPLYSGCRLTLLRPEKDGVATRVDAICTHRPDPKIPGLDRQQLYWELSQLTHSITELGPYTLD
RDSLYVNGFTQRSSVPTTSTPGTFTVQPETSETPSSLPGPTATGPVLLPFTLNFTITNLQYEEDMRRPGSRKFNT
TERVLQGLLMPLFKNTSVSSLYSGCRLTLLRPEKDGAATRVDAVCTHRPDPKSPGLDRERLYWKLSQLTHGITEL
GPYTLDRHSLYVNGFTHQSSMTTTRTPDTSTMHLATSRTPASLSGPMTASPLLVLFTINFTITNLRYEENMHHPG
SRKFNTTERVLQGLLRPVFKNTSVGPLYSGCRLTLLRPKKDGAATKVDAICTYRPDPKSPGLDREQLYWELSQLT
HSITELGPYTLDRDSLYVNGFTQRSSVPTTSIPGTPTVDLGTSGTPVSKPGPSAASPLLVLFTLNFTITNLRYEE
NMQHPGSRKFNTTERVLQGLLRSLFKSTSVGPLYSGCRLTLLRPEKDGTATGVDAICTHHPDPKSPRLDREQLYW
ELSQLTHNITELGPYALDNDSLFVNGFTHRSSVSTTSTPGTPTVYLGASKTPASIFGPSAASHLLILFTLNFTIT
NLRYEENMWPGSRKFNTTERVLQGLLRPLFKNTSVGPLYSGCRLTLLRPEKDGEATGVDAICTHRPDPTGPGLDR
EQLYLELSQLTHSITELGPYTLDRDSLYVNGFTHRSSVPTTSTGVVSEEPFTLNFTINNLRYMADMGQPGSLKFN
ITDNVMQHLLSPLFQRSSLGARYTGCRVIALRSVKNGAETRVDLLCTYLQPLSGPGLPIKQVFHELSQQTHGITR
LGPYSLDKDSLYLNGYNEPGPDEPPTTPKPATTFLPPLSEATTAMGYHLKTLTLNFTISNLQYSPDMGKGSATFN
STEGVLQHLLRPLFQKSSMGPFYLGCQLISLRPEKDGAATGVDTTCTYHPDPVGPGLDIQQLYWELSQLTHGVTQ
LGFYVLDRDSLFINGYAPQNLSIRGEYQINFHIVNWNLSNPDPTSSEYITLLRDIQDKVTTLYKGSQLHDTFRFC
LVTNLTMDSVLVTVKALFSSNLDPSLVEQVFLDKTLNASFHWLGSTYQLVDIHVTEMESSVYQPTSSSSTQHFYL
NFTITNLPYSQDKAQPGTTNYQRNKRNIEDALNQLFRNSSIKSYFSDCQVSTFRSVPNRHHTGVDSLCNFSPLAR

RVDRVAIYEEFLRMTRNGTQLQNFTLDRSSVLVDGYSPNRNEPLTGNSDLPFWAVILIGLAGLLGVITCLICGVL
VTTRRRKKEGEYNVQQQCPGYYQSHLDLEDLQ

Metalloreductase STEAP1 is an enzyme that in humans is encoded by the STEAP1 gene. This gene is predominantly expressed in prostate tissue, and is found to be upregulated in multiple cancer cell lines. The gene product is predicted to be a six-transmembrane protein, and was shown to be a cell surface antigen significantly expressed at cell-cell junctions. Protein STEAP16 is characterized by an amino acid sequence according to SEQ ID NO.: 14.

STEAP1 (SEQ ID NO.: 14):
MESRKDITNQEELWKMKPRRNLEEDDYLHKDTGETSMLKRPVLLHLHQTAHADEFDCPSELQHTQELFPQWHLPI
KIAAIIASLTFLYTLLREVIHPLATSHQQYFYKIPILVINKVLPMVSITLLALVYLPGVIAAIVQLHNGTKYKKF
PHWLDKWMLTRKQFGLLSFFFAVLHAIYSLSYPMRRSYRYKLLNWAYQQVQQNKEDAWIEHDVWRMEIYVSLGIV
GLAILALLAVTSIPSVSDSLTWREFHYIQSKLGIVSLLLGTIHALIFAWNKWIDIKQFVWYTPPTFMIAVFLPIV
VLIFKSILFLPCLRKKILKIRHGWEDVTKINKTEICSQL

Preferably, the tumor antigen is selected from GD2, *O*-acetyl-GD2, B7-H3, GPC2, and ALK.

More preferably, the tumour antigen is selected from GD2, *O*-acetyl-GD2, and B7-H3.

Even more preferably, the tumour antigen is GD2, or *O*-acetyl-GD2 (such as 7-*O*-acetyl-GD2 or 9-*O-*acetyl-GD2).

In a particularly preferred embodiment of the present invention, the tumor antigen is GD2.

Accordingly, as provided by the present invention particularly preferred is antibody that specifically binds GD2.

As preferably referred to herein, an antibody is defined in the following.

In general, the term "antibody" is used herein in the broadest sense and encompasses various antibody structures, including but not limited to monoclonal antibodies, polyclonal antibodies, multispecific antibodies (*e.g.*, bispecific antibodies), fully-human antibodies and antibody constructs so long as they exhibit the desired antigen-binding activity. Unless explicitly indicated to the contrary, when discussing the antibody or the properties thereof, whenever a reference is made to an antibody, any form of the antibody as apparent to the skilled person (such as a form selected from monoclonal antibodies, polyclonal antibodies, multispecific antibodies *(e.g*., bispecific antibodies), and fully-human antibodies) is meant.

As apparent to the skilled person, the term "antibody" may also refer to an antigen-binding fragment thereof. As preferably referred to herein, an "antigen-binding fragment" of an antibody refers to a molecule other than an intact antibody that comprises a portion of an intact antibody and that binds the antigen to which the intact antibody binds. Examples of antibody fragments include but are not limited to Fv, Fab, Fab', Fab' -SH, F(ab')2; diabodies; linear antibodies; single-chain antibody molecules *(e*.*g*. scFv); single-domain antibodies (also known as nanobodies) and multispecific antibodies formed from antibody fragments.

Preferably, the antibody, as referred to herein, is not antigen-binding fragment of an antibody.

Preferably, the antibody is a monoclonal antibody, a chimeric antibody, a recombinant antibody, a single chain antibody, a humanized antibody, a bispecific antibody, or a multi-specific antibody.

The term "monoclonal antibody" as used herein, refers to an antibody obtained from a population of substantially homogeneous antibodies, *i.e*., the individual antibodies comprising the population are identical except for possible naturally occurring mutations that may be present in minor amounts. Monoclonal antibodies are highly specific, being directed against a single antigenic site. Monoclonal antibodies are advantageous in that they may be synthesized by a hybridoma culture, essentially uncontaminated by other immunoglobulins. The modified "monoclonal" indicates the character of the antibody as being amongst a substantially homogeneous population of antibodies, and is not to be construed as requiring production of the antibody by any particular method. As mentioned above, the monoclonal antibodies to be used in accordance with the present invention may be made by the hybridoma method described by Kohler, Nature 256 (1975), 495.

The term "chimeric antibodies", refers to an antibody which comprises a variable region of the present invention fused or chimerized with an antibody region (*e*.*g*., constant region) from another, human or non-human species (*e*.*g*., mouse, horse, rabbit, dog, cow, chicken).

The term "recombinant antibody" includes all antibodies that are prepared, expressed, created or isolated by recombinant means, such as antibodies isolated from an animal (e.g. a mouse) that is transgenic for human immunoglobulin genes, antibodies expressed using a recombinant expression vector transfected into a host cell, antibodies isolated from a recombinant, combinatorial human antibody library, or antibodies prepared, expressed, created or isolated by any other means that involves splicing of human immunoglobulin gene sequences to other DNA sequences. Accordingly, the term antibody also relates to recombinant human antibodies, heterologous antibodies and heterohybrid antibodies. Such recombinant human antibodies have variable and constant regions (if present) derived from human germline immunoglobulin sequences. Such antibodies can, however, be subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

A "heterologous antibody" is defined in relation to the transgenic non-human organism producing such an antibody. This term refers to an antibody having an amino acid sequence or an encoding nucleic acid sequence corresponding to that found in an organism not consisting of the transgenic non-human animal, and generally from a species other than that of the transgenic non-human animal.

The term "heterohybrid antibody" refers to an antibody having light and heavy chains of different organismal origins. For example, an antibody having a human heavy chain associated with a murine light chain is a heterohybrid antibody. Examples of heterohybrid antibodies include chimeric and humanized antibodies.

The term antibody also relates to humanized antibodies. "Humanized" forms of non-human (e.g. murine or rabbit) antibodies are chimeric immunoglobulins, immunoglobulin chains or fragments thereof (such as Fv, Fab, Fab', F(ab')2 or other antigen-binding subsequences of antibodies) which contain minimal sequence derived from non-human immunoglobulin. Often, humanized antibodies are human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues, which are found neither in the recipient antibody nor in the imported CDR or framework sequences. These modifications are made to further refine and optimize antibody performance. In general, the humanized antibody will comprise substantially all of at least one, and typically two variable domains, in which all or substantially all of the CDR regions correspond to those of a non-human immunoglobulin and all or substantially all of the FR regions are those of a human immunoglobulin consensus sequence. The humanized antibody may also comprise at least a portion of an immunoglobulin constant region (Fc), typically that of a human immunoglobulin. For further details, see: JonesNature 321 (1986), 522-525; Reichmann Nature 332 (1998), 323-327 and Presta Curr Op Struct Biol 2 (1992), 593-596.

Accordingly, in context of the present invention, the term "antibody" relates to full immunoglobulin molecules as well as to parts of such immunoglobulin molecules (i.e., "antigen-binding fragment thereof'). Furthermore, the term relates, as discussed above, to modified and/or altered antibody molecules. The term also relates to recombinantly or synthetically generated/synthesized antibodies. The term preferably relates to intact antibodies. However, the term may also relate to antibody fragments thereof, like, separated light and heavy chains, Fab, Fv, Fab', Fab'-SH, F(ab')2. The term antibody also comprises but is not limited to fully-human antibodies, chimeric antibodies, humanized antibodies, CDR-grafted antibodies and antibody constructs, like single chain Fvs (scFv) or antibody-fusion proteins.

A single chain antibody, i.e., "single-chain Fv" or "scFv" antibody fragments have, in the context of the invention, the V_{H} and V_{L} domains of an antibody, wherein these domains are present in a single polypeptide chain. Generally, the scFv polypeptide further comprises a polypeptide linker between the V_{H} and V_{L} domains which enables the scFv to form the desired structure for antigen binding. Techniques described for the production of single chain antibodies are described, e.g., in Plückthun in The Pharmacology of Monoclonal Antibodies, Rosenburg and Moore eds. Springer-Verlag, N.Y. (1994), 269-315.

A "Fab fragment" as used herein is comprised of one light chain and the C_{H}1 and variable regions of one heavy chain. The heavy chain of a Fab molecule cannot form a disulfide bond with another heavy chain molecule.

An "Fc" region contains two heavy chain fragments comprising the C_{H}2 and C_{H}3 domains of an antibody. The two heavy chain fragments are held together by two or more disulfide bonds and by hydrophobic interactions of the C_{H}3 domains.

A "Fab' fragment" contains one light chain and a portion of one heavy chain that contains the V_{H} domain and the C _{H}1 domain and also the region between the C_{H}1 and C _{H}2 domains, such that an interchain disulfide bond can be formed between the two heavy chains of two Fab' fragments to form a F(ab')₂ molecule.

A "F(ab')₂ fragment" contains two light chains and two heavy chains containing a portion of the constant region between the C_{H}1 and C_{H}2 domains, such that an interchain disulfide bond is formed between the two heavy chains. A F(ab')₂ fragment thus is composed of two Fab' fragments that are held together by a disulfide bond between the two heavy chains.

The "Fv region" comprises the variable regions from both the heavy and light chains, but lacks the constant regions.

A bispecific antibody, as referred to herein, is an antibody that can simultaneously bind to two different types of antigen, or to two different epitopes of the same antigen. Upon development, bispecific antibodies can be manufactured in several structural formats, which are known to the skilled person.

A multi-specific antibody, as referred to herein, is an antibody that can simultaneously bind to more than two different types of antigen, or to more than two different epitopes of the same antigen.

Antibodies, antibody constructs, antibody fragments, antibody derivatives (all being Ig-derived) to be employed in accordance with the invention or their corresponding immunoglobulin chain(s) can be further modified using conventional techniques known in the art, for example, by using amino acid deletion(s), insertion(s), substitution(s), addition(s), and/or recombination(s) and/or any other modification(s) known in the art either alone or in combination. Methods for introducing such modifications in the DNA sequence underlying the amino acid sequence of an immunoglobulin chain are well known to the person skilled in the art; see, e.g., Sambrook (1989), loc. cit. The term "Ig-derived domain" particularly relates to (poly) peptide constructs comprising at least one CDR. Fragments or derivatives of the recited Ig-derived domains define (poly) peptides which are parts of the above antibody molecules and/or which are modified by chemical/biochemical or molecular biological methods. Corresponding methods are known in the art and described inter alia in laboratory manuals (see Sambrook et al., Molecular Cloning: A Laboratory Manual; Cold Spring Harbor Laboratory Press, 2nd edition (1989) and 3rd edition (2001); Gerhardt et al., Methods for General and Molecular Bacteriology ASM Press (1994); Lefkovits, Immunology Methods Manual: The Comprehensive Sourcebook of Techniques; Academic Press (1997); Golemis, Protein-Protein Interactions: A Molecular Cloning Manual Cold Spring Harbor Laboratory Press (2002)).

More preferably, the antibody as referred to herein is a monoclonal antibody.

The antibody as referred to herein may be an IgG1, IgG2a or IgG2b, IgG3, IgG4, IgM, lgA1, IgA2, IgAsec, IgD, IgE. As used herein, "isotype" refers to the antibody class (e.g., IgM or IgG1) that is encoded by heavy chain constant region genes. The "class" of an antibody refers to the type of constant domain or constant region possessed by its heavy chain. There are five major classes of antibodies: IgA, IgD, IgE, IgG, and IgM, and several of these may be further divided into subclasses (isotypes), *e.g*., IgG1, IgG2, IgG3, IgG4, lgA1, and IgA2. The heavy chain constant domains that correspond to the different classes of immunoglobulins are called α, δ, ε, γ, and µ, respectively.

Preferably, the monoclonal antibody as described herein is an IgG1 antibody.

Accordingly, the antibodies of the present invention can be full length or can include only an antigen-binding fragment such as the antibody constant and/or variable domain of IgG1, IgG2, IgG3, IgG4, IgM, lgA1, IgA2, IgAsec, IgD or IgE or could consist of a Fab fragment, a F(ab')2 fragment and a Fv fragment.

Selection of a particular antibody depends on particular cancer cell to be targeted and a particular cell surface tumour antigen to be targeted. In the case of high-risk neuroblastoma, Ab is a full-length IgG monoclonal antibody (mAb) or antibody fragment targeting GD2, B7-H3 (CD276), GPC2 or ALK (CD246) which are well-established neuroblastoma tumor-associated antigens (TAAs) and have previously been used as cell surface targets for the development of ADCs for the targeted therapy of neuroblastoma.¹⁰⁻¹⁶

In addition to the abovementioned TAAs and for targeting other cancer types, additional targets for the Ab include HER2 and Trop-2 for targeting breast cancer cells; HER3 for targeting melanoma cancer cells; CD33, CLL-1 (CD371), c-KIT (CD117) or CD123 for targeting AML cancer cells; EGFR, or MUC16 for targeting colorectal cancer cells, FR-o or Trop-2 for targeting ovarian cancer cells and STEAP-1 for targeting Ewing sarcoma cancer cells.

Full-length IgG mAb targeting GD2 TAA can be a chimeric mouse-human dinutuximab (ch14.18, tradename Unituxin), dinutuximab beta (ch14.18/CHO, tradename Qarziba), humanized naxitamab (Hu3F8, tradename Danyelza) or humanized hu14.18K322A antibody (which is described in US 10,633,452 B2). Preferably, in the immunoconjugate of the present invention, (a) is dinutuximab, dinutuximab beta or naxitamab.

Accordingly, in one embodiment, (a) is dinutuximab or dinutuximab beta (chimeric anti-GD2). These both constructs are characterized by the same sequence, as outlined in the following.
dinutuximab or dinutuximab beta (chimeric anti-GD2) heavy chain (SEQ ID NO.: 15): 
EVQLLQSGPELEKPGASVMISCKASGSSFTGYNMNWVRQNIGKSLEWIGAIDPYYGGTSYNQKFKGRATLTVDKS
SSTAYMHLKSLTSEDSAVYYCVSGMEYWGQGTSVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKRVEPKSCDKTHTCPPC
PAPELLGGPSVFLFPPKPKDTLMISRTPEVTCVVVDVSHEDPEVKFNWYVDGVEVHNAKTKPREEQYNSTYRVVS
VLTVLHQDWLNGKEYKCKVSNKALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSLSPGK
dinutuximab or dinutuximab beta (chimeric anti-GD2) light chain (SEQ ID NO.: 16):

EIVMTQSPATLSVSPGERATLSCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC

In another embodiment, (a) is naxitamab (humanized anti-GD2), characterized by the sequence as outlined in the following.
naxitamab (humanized anti-GD2) heavy chain (SEQ ID NO.: 17): 
QVQLVESGPGVVQPGRSLRISCAVSGFSVTNYGVHWVRQPPGKGLEWLGVIWAGGITNYNSAFMSRLTISKDNSK
NTVYLQMNSLRAEDTAMYYCASRGGHYGYALDYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKRVEPKSCDKT
HTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHYTQKSLSLSPGK
naxitamab (humanized anti-GD2) light chain (SEQ ID NO.: 18): 
EIVMTQTPATLSVSAGERVTITCKASQSVSNDVTWYQQKPGQAPRLLIYSASNRYSGVPARFSGSGYGTEFTFTI
SSVQSEDFAVYFCQQDYSSFGQGTKLEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGEC
hu14.18K322A (humanized anti-GD2) heavy chain (SEQ ID NO.: 26): 
EVQLVQSGAEVEKPGASVKISCKASGSSFTGYNMNWVRQNIGKSLEWIGAIDPYYGGTSYNQKFKGRATLTVDKS
TSTAYMHLKSLRSEDTAVYYCVSGMEYWGQGTSVTVSSASTKGPSVFPLAPSSKSTSGGTAALGCLVKDYFPEPV
TVSWNSGALTSGVHTFPAVLQSSGLYSLSSVVTVPSSSLGTQTYICNVNHKPSNTKVDKRVEPKSCDKTHTCPPC
PAPELLGGPSVFLFPPKPKDTLMISRTPEVTCVVVDVSHEDPEVKFNWYVDGVEVHNAKTKPREEQYNSTYRVVS
 
VLTVLHQDWLNGKEYKCAVSNAALPAPIEKTISKAKGQPREPQVYTLPPSREEMTKNQVSLTCLVKGFYPSDIAV
EWESNGQPENNYKTTPPVLDSDGSFFLYSKLTVDKSRWQQGNVFSCSVMHEALHNHYTQKSLSLSPGK
hu14.18K322A (humanized anti-GD2) light chain (SEQ ID NO.: 27):

DVVMTQTPLSLPVTPGEPASISCRSSQSLVHRNGNTYLHWYLQKPGQSPKLLIHKVSNRFSGVPDRFSGSGSGTD
FTLKISRVEAEDLGVYFCSQSTHVPPLTFGAGTKLELKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC

In one embodiment, to enhance neuroblastoma tumor cell-specificity and reduce unwanted neurotoxicity, full-length IgG mAb can be a chimeric mouse-human c.8B6 antibody targeting O-acetyl-GD2 ganglioside (OAcGD2) TAA. Studies have shown that (neuroblastoma) tumor cells that express GD2 also express its O-acetyl derivative OAcGD2 and it was also found that OAcGD2 is not expressed by peripheral nerve fibers. The absence of OAcGD2 on nerve fibers leads to the reduction of adverse neurological side effects compared to the anti-GD2 antibody-based immunotherapy with preserved high anti-GD2 activity. Antibody c.8B6 is disclosed in document EP 3 068 802 B1.

Thus, in one embodiment, (a) is c.8B6 antibody, characterized by by the sequence as outlined in the following:
c.8B6 heavy chain (SEQ ID NO.: 22): 
EVKLVESGGGLVLPGDSLRLSCATSEFTFTDYYMTWVRQPPRKALEWLGFIRNRANGYTTEYNPSVKGRFTISRD
NSQSILYLQMNTLRTEDSATYYCARVSNWAFDYWGQGTTLTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFNWYVDGVEVHNAKTKPREEQYNS
TYRVVSVLTVLHQDWLNGKEYKCKVSNKALPAPIEKTISKAKGQPREPQVYTLPPSREEMTKNQVSLTCLVKGFY
PSDIAVEWESNGQPENNYKTTPPVLDSDGSFFLYSKLTVDKSRWQQGNVFSCSVMHEALHNHYTQKSLSLSPGK
c.8B6 light chain (SEQ ID NO.: 23):

DVVMTQTPLSLPVSLGDQASISCRSSQSLLKNNGNTFLHWYLQKSGQSPKLLIYKVSNRLSGVPDRFSGSGSGTY
FTLKISRVEAEDLGVYFCSQSTHIPYTFGGGTKLEIKRTVAAPSVFIFPPSDEQLKSGTASVVCLLNNFYPREAK
VQWKVDNALQSGNSQESVTEQDSKDSTYSLSSTLTLSKADYEKHKVYACEVTHQGLSSPVTKSFNRGEC

Additionally, to further enhance neuroblastoma tumor cell-specificity and reduce unwanted neurotoxicity, full-length IgG mAb is a bispecific antibody that targets a combination of two TAA- GD2 and B7-H3 (SNIPER genetically engineered bispecific Abs, Paul Sondel, Invenra). B7-H3 is overexpressed on the surface of multiple tumor cells, with only minimal expression on the healthy tissue and absent on nerve cells.

In one embodiment, (a) is Nivatrotamab (Hu3F8-BsAb), a humanized anti-GD2/CD3 bispecific (IgG(L)-scFv2) antibody. Nivatrotamab was in Phase I/II clinical development for the treatment of relapsed/refractory neuroblastoma, osteosarcoma and other GD2⁺ solid tumors- NCT03860207 (terminated) and relapsed/recurrent metastatic SCLC- NCT04750239 (terminated).

As understood herein, Nivatrotomab is characterized by the sequence as outlined in the following:
Nivatrotomab Heavy chain (SEQ ID NO.: 24): 
QVQLVESGPGVVQPGRSLRISCAVSGFSVTNYGVHWVRQPPGKGLEWLGVIWAGGITNYNSAFMSRLTISKDNSK
NTVYLQMNSLRAEDTAMYYCASRGGHYGYALDYWGQGTLVTVSSASTKGPSVFPLAPSSKSTSGGTAALGCLVKD
YFPEPVTVSWNSGALTSGVHTFPAVLQSSGLYSLSSVVTVPSSSLGTQTYICNVNHKPSNTKVDKRVEPKSCDKT
HTCPPCPAPELLGGPSVFLFPPKPKDTLMISRTPEVTCVVVDVSHEDPEVKFNWYVDGVEVHNAKTKPREEQYAS
TYRVVSVLTVLHQDWLNGKEYKCAVSNKALPAPIEKTISKAKGQPREPQVYTLPPSRDELTKNQVSLTCLVKGFY
PSDIAVEWESNGQPENNYKTTPPVLDSDGSFFLYSKLTVDKSRWQQGNVFSCSVMHEALHNHYTQKSLSLSPGK
Nivatrotomab Light chain fused to CD3-specific scFv via (G4S)3 linker (SEQ ID NO.: 25):

EIVMTQTPATLSVSAGERVTITCKASQSVSNDVTWYQQKPGQAPRLLIYSASNRYSGVPARFSGSGYGTEFTFTI
SSVQSEDFAVYFCQQDYSSFGQGTKLEIKRTVAAPSVFIFPPSDEQLKSGTASVVCLLNNFYPREAKVQWKVDNA
LQSGNSQESVTEQDSKDSTYSLSSTLTLSKADYEKHKVYACEVTHQGLSSPVTKSFNRGECTSGGGGSGGGGSGG
GGSQVQLVQSGGGVVQPGRSLRLSCKASGYTFTRYTMHWVRQAPGKCLEWIGYINPSRGYTNYNQKFKDRFTISR
DNSKNTAFLQMDSLRPEDTGVYFCARYYDDHYSLDYWGQGTPVTVSSGGGGSGGGGSGGGGSGGGGSGGGGSGGG
GSDIQMTQSPSSLSASVGDRVTITCSASSSVSYMNWYQQTPGKAPKRWIYDTSKLASGVPSRFSGSGSGTDYTFT
ISSLQPEDIATYYCQQWSSNPFTFGCGTKLQITR

In addition, to further enhance the efficacy of the anti-GD2 antibody-based therapy, full-length IgG mAb is a bispecific trifunctional antibody that targets a combination of GD2 and CD3 antigens (EKTOMUN, Trion Research). Such antibody can in addition to Fc-mediated cytotoxicity exert neuroblastoma cell killing by redirecting T cells towards GD2⁺ neuroblastoma tumors via its CD3 binding arm.

Thus, accordingly, in one embodiment, (a) is a bispecific antibody or antibody construct, which further binds to B7-H3 or to CD3. Preferably, (a) is a bispecific antibody or antibody construct, which further binds to B7-H3. Such exemplary antibodies include INV721 (which is described in US 2023/0018670 A1) or its afucosylated form INV724, and EKTOMUN (which is described in WO 2023/280880), which is a trifunctional antibody composed of a combination of mouse anti-GD2 IgG2a Me361 and rat anti-CD3 IgG2b 26II6 antibodies and produced using quadroma technology (see, e.g., Lindhofer H, Mocikat R,

Steipe B, Thierfelder S. Preferential speciesrestricted heavy/light chain pairing in rat/mouse quadromas: implications for a single-step purification of bispecific antibodies. J Immunol 1995;155:219 -25)

In one embodiment of the immunoconjugate of the present invention, the antibody is characterized by a point mutation K322A.

In one embodiment of the immunoconjugate of the present invention, the antibody is a deglycosylated antibody.

Another strategy for reducing the neuropathic pain which is likely mediated by antibody-dependent cell-mediated cytotoxicity (ADCC) or complement-dependent cytotoxicity (CDC) is via the decrease of antibody effector functions through Fc region engineering.

For example, Hu14.18K332A is a humanized anti-GD2 mAb with a single point mutation K322A significantly reduces the CDC effector functions and induces less pain (based on opioid requirements) than dinutuximab (ch14.18) non-engineered antibody. Moreover, Hu14.18K332A was produced in a YB2/0 rat myeloma cell line with decreased fucosylation activity and it has been previously shown that reduction of fucose content within the glycosylation site of antibody Fc domain results in higher mAb binding affinity for CD16 (FcyRIII) on NK cells and subsequent increase in ADCC activity.

It is noted that dinutuximab beta (ch14.18/CHO, tradename Qarziba) is expressed in a recombinant DHFR-deficient cell line with preserved fucosylation activity. Dinutuximab beta triggers CDC resulting in activation of the complement cascade and lysis of target cells which is implicated in the pain toxicity associated with anti-GD2 mAb immunotherapy

In certain embodiments, antibody variants having one or more amino acid substitutions are provided. Sites of interest for substitutional mutagenesis include the CDRs and FRs. Conservative substitutions are shown in Table D1 under the heading of "preferred substitutions." More substantial changes are provided in Table D1 under the heading of "exemplary substitutions," and as further described below in reference to amino acid side chain classes. Amino acid substitutions may be introduced into an antibody of interest and the products screened for a desired activity, e.g., retained/improved antigen binding, decreased immunogenicity, or improved ADCC or CDC.

**Table D1.**

| Original Residue | Exemplary Substitutions | Preferred Substitutions |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gin (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; Norleucine | Leu |

Amino acids may be grouped according to common side-chain properties:
(1) hydrophobic: Norleucine, Met, Ala, Val, Leu, Ile;
(2) neutral hydrophilic: Cys, Ser, Thr, Asn, Gln;
(3) acidic: Asp, Glu;
(4) basic: His, Lys, Arg;
(5) residues that influence chain orientation: Gly, Pro;
(6) aromatic: Trp, Tyr, Phe.

Non-conservative substitutions will entail exchanging a member of one of these classes for another class.

In certain embodiments, an antibody provided herein is altered to increase or decrease the extent to which the antibody is glycosylated. Addition or deletion of glycosylation sites to an antibody may be conveniently accomplished by altering the amino acid sequence such that one or more glycosylation sites is created or removed.

Where the antibody comprises an Fc region, the carbohydrate attached thereto may be altered. Native antibodies produced by mammalian cells typically comprise a branched, biantennary oligosaccharide that is generally attached by an N-linkage to Asn297 of the CH2 domain of the Fc region. See, *e.g.,* Wright et al. TIBTECH 15:26-32 (1997). The oligosaccharide may include various carbohydrates, *e*.*g*., mannose, N-acetyl glucosamine (GIcNAc), galactose, and sialic acid, as well as a fucose attached to a GIcNAc in the "stem" of the biantennary oligosaccharide structure. In some embodiments, modifications of the oligosaccharide in an antibody of the invention may be made in order to create antibody variants with certain improved properties.

In one embodiment, antibody variants are provided having a carbohydrate structure that lacks fucose attached (directly or indirectly) to an Fc region. For example, the amount of fucose in such antibody may be from 1% to 80%, from 1% to 65%, from 5% to 65% or from 20% to 40%. The amount of fucose is determined by calculating the average amount of fucose within the sugar chain at Asn297, relative to the sum of all glycostructures attached to Asn 297 (e. g. complex, hybrid and high mannose structures) as measured by MALDI-TOF mass spectrometry, as described in WO 2008/077546, for example. Asn297 refers to the asparagine residue located at about position 297 in the Fc region (Eu numbering of Fe region residues); however, Asn297 may also be located about ± 3 amino acids upstream or downstream of position 297, i.e., between positions 294 and 300, due to minor sequence variations in antibodies. Such fucosylation variants may have improved ADCC function. *See, e.g.,* US Patent Publication Nos. US 2003/0157108 (Presta, L.); US 2004/0093621 (Kyowa Hakko Kogyo Co., Ltd). Examples of publications related to "defucosylated" or "fucose deficient" antibody variants include: US 2003/0157108; WO 2000/61739; WO 2001/29246; US 2003/0115614; US 2002/0164328; US 2004/0093621; US 2004/0132140; US 2004/0110704; US 2004/0110282; US 2004/0109865; WO 2003/085119; WO 2003/084570; WO 2005/035586; WO 2005/035778; WO2005/053742; WO2002/031140; Okazaki et al. J. Mol. Biol. 336:1239-1249 (2004); Yamane-Ohnuki et al. Biotech. Bioeng. 87: 614 (2004). Examples of cell lines capable of producing defucosylated antibodies include Lec13 CHO cells deficient in protein fucosylation (Ripka et al. Arch. Biochem. Biophys. 249:533-545 (1986); US Pat Appl No US 2003/0157108 Al, Presta, L; and WO 2004/056312 Al, Adams et al.*,* especially at Example 11), and knockout cell lines, such as alpha-1,6-fucosyltransferase gene, *FUT8,* knockout CHO cells *(see, e.g.,* Yamane-Ohnuki et al. Bioteeh. Bioeng. 87: 614 (2004); Kanda, Y. et al., Bioteehnol. Bioeng., 94(4):680-688 (2006); and WO2003/085 I07).

Antibodies variants are further provided with bisected oligosaccharides, e.g., in which a biantennary oligosaccharide attached to the Fc region of the antibody is bisected by GlcNAc. Such antibody variants may have reduced fucosylation and/or improved ADCC function. Examples of such antibody variants are described, e.g., in WO 2003/011878 (Jean-Mairet et al.); US Patent No. 6,602,684 (Umana et al.); and US 2005/0123546 (Umana et al.*).* Antibody variants with at least one galactose residue in the oligosaccharide attached to the Fc region are also provided. Such antibody variants may have improved CDC function. Such antibody variants are described, e.g., in WO 1997/30087 (Patel et al.); WO 1998/58964 (Raju, S.); and WO 1999/22764 (Raju, S.).

In certain embodiments, one or more amino acid modifications may be introduced into the Fc region of an antibody provided herein, thereby generating an Fc region variant. The Fc region variant may comprise a human Fc region sequence (e.g., a human IgG1, IgG2, IgG3 or IgG4 Fc region) comprising an amino acid modification (e.g. a substitution) at one or more amino acid positions.

In certain embodiments, the invention contemplates an antibody variant that possesses some but not all effector functions, which make it a desirable candidate for applications in which the half-life of the antibody *in vivo* is important yet certain effector functions (such as complement and antibody-dependent cellular cytotoxicity) are unnecessary or deleterious. *In vitro* and/or *in vivo* cytotoxicity assays can be conducted to confirm the reduction/depletion of CDC (complement-dependent cytotoxicity) and/or ADCC activities. For example, Fc receptor (FcR) binding assays can be conducted to ensure that the antibody lacks FcyR binding (hence likely lacking ADCC activity), but retains FcRn binding ability. The primary cells for mediating ADCC, NK cells, express Fc(RIII only, whereas monocytes express Fc(RI, Fc(RII and Fc(RIII. FcR expression on hematopoietic cells is summarized in Table 3 on page 464 of Ravetch and Kinet, Annu. Rev. Immunol. 9:457-492 (1991). Non-limiting examples of *in vitro* assays to assess ADCC activity of a molecule of interest is described in U.S. Patent No. 5,500,362 *(see, e.g.* Hellstrom, I. et al. Proc. Nat'l Acad. Sci. USA 83:7059-7063 (1986)) and Hellstrom, I et al., Proc. Nat'l Acad. Sci. USA 82:1499-1502 (1985); 5,821,337 (see Bruggemann, M. et al., J. Exp. Med. 166:1351-1361 (1987)).

Accordingly and preferably, in the antibody or the antigen-binding fragment thereof (as far as applicable) according to the present invention, the heavy chain comprises at least one point mutation in Fc part that influences antibody-dependent cell-mediated cytotoxicity (ADCC), complement-dependent cytotoxicity (CDC), serum half-life and/or glycosylation status of the antibody. It is to be understood to the skilled person that only antigen-binding fragments of said antibody that comprise the heavy chain, or the fragment thereof, may be encompassed herein. The at least one point mutation as referred to herein is preferably selected from L234A, L234F, L235A, L235E, L235Q, G236A, M252Y, S254T, T256E, S267E, H268F, N297A, K322A, K322Q, S324T, P331S, and I332E. More preferably, the at least one point mutation is selected from L234A, L235A, P331S and N297A. It is to be understood herein that the at least one point mutation may refer to more than one mutation. For example, in one preferred embodiment of the present invention, the heavy chain comprises L234A, L235A, P331S and N297A point mutations. As it is preferably to be understood herein, the amino acid positions as recited herein refer to the residue numbering as in IgG1 isotype. Should any other antibody class or isotype be used, the skilled person will be in position to translate the amino acid positions to any other antibody class or isotype known in the art.

The skilled person may also envisage including at least one mutation that increases antibody-dependent cell-mediated cytotoxicity (ADCC) and/or complement-dependent cytotoxicity (CDC). One possible combination is at least one mutation selected from S267E/H268F/S324T/G236A/I332E (EFTAE modification), preferably the mutations S267E/H268F/S324T/G236A/I332E (EFTAE modification).

The skilled person may further envisage including at least one mutation that counters the negative effect of N297A mutation (which allows for the production of deglycosylated antibodies).

The antibody or an antigen binding thereof of the present invention, provided herein may be further modified to contain additional nonproteinaceous moieties that are known in the art and readily available. The moieties suitable for derivatization of the antibody include but are not limited to water soluble polymers. Nonlimiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-1,3,6-trioxane, ethylene/maleic anhydride copolymer, polyaminoacids (either homopolymers or random copolymers), and dextran or poly(n vinyl pyrrolidone)polyethylene glycol, propropylene glycol homopolymers, prolypropylene oxide/ethylene oxide co-polymers, polyoxyethylated polyols (*e.g*., glycerol), polyvinyl alcohol, and mixtures thereof. Polyethylene glycol propionaldehyde may have advantages in manufacturing due to its stability in water. The polymer may be of any molecular weight, and may be branched or unbranched. The number of polymers attached to the antibody may vary, and if more than one polymer are attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

Further modifications of the antibody are contemplated in the context of obtaining antibody conjugates with optimal *in vitro* and *in vivo* characteristics is via introduction of Cys residues at certain positions in the light or heavy chains of antibodies for site-specific bioconjugation that results in homogenous antibody labelling. THIOMAB^{™} thiol-engineering technology was developed by Genentech and has already been applied for the development of ADCs (DAR 2 or 4) via introduction of Cys at various positions, e.g., LC-S121C, LC-K149C, LC-V205C, HC-A114C, HC-A118C, HC-A121C, HC-S239C, HC-i239C, HC-S396C or HC-S442C. Further modifications of the antibody in order to prolong the serum half-life and increase internalization of therapeutic antibodies are also contemplated and encompassed by the present invention.

The antibody or an antigen binding thereof of the present invention, provided herein may also be further modified to contain additional moieties, thus yielding immunoconjugates comprising the antibody or the antigen-binding fragment thereof of the present invention. Said immunoconjugates are described herein. Antibodies may be produced using recombinant methods and compositions, *e.g*., as described in U.S. Patent No. 4,816,567. In one embodiment, isolated nucleic acid encoding an antibody described herein is provided. Such nucleic acid may encode an amino acid sequence comprising the VL and/or an amino acid sequence comprising the VH of the antibody (*e.g*., the light and/or heavy chains of the antibody).

An "isolated nucleic acid" refers to a nucleic acid molecule that has been separated from a component of its natural environment. An isolated nucleic acid includes a nucleic acid molecule contained in cells that ordinarily contain the nucleic acid molecule, but the nucleic acid molecule is present extra chromosomally or at a chromosomal location that is different from its natural chromosomal location.

Accordingly, for the production of the antibody, one or more vectors (*e.g.*, expression vectors) comprising such nucleic acid are provided. It is to be understood that, unless indicated to the contrary, the terms polynucleotide and (isolated) nucleic acids may be used interchangeably.

The term "vector," as used herein, refers to a nucleic acid molecule capable of propagating another nucleic acid to which it is linked. The term includes the vector as a self-replicating nucleic acid structure as well as the vector incorporated into the genome of a host cell into which it has been introduced. Certain vectors are capable of directing the expression of nucleic acids to which they are operatively linked. Such vectors are referred to herein as "expression vectors". Thus, further provided is a vector comprising the polynucleotide encoding at least one variable heavy chain sequence and/or at least one variable light chain sequence as described herein.

As disclosed herein, further provided is a host cell comprising such nucleic acid. The terms "host cell," "host cell line," and "host cell culture" are used interchangeably and refer to cells into which exogenous nucleic acid has been introduced, including the progeny of such cells. Host cells include "transformants" and "transformed cells," which include the primary transformed cell and progeny derived therefrom without regard to the number of passages. Progeny may not be completely identical in nucleic acid content to a parent cell, but may contain mutations. Mutant progeny that have the same function or biological activity as screened or selected for in the originally transformed cell are included herein.

As provided herein, a host cell comprises (*e.g*., has been transformed with): (1) a vector comprising a nucleic acid that encodes an amino acid sequence comprising the VL of the antibody and an amino acid sequence comprising the VH of the antibody, or (2) a first vector comprising a nucleic acid that encodes an amino acid sequence comprising the VL of the antibody and a second vector comprising a nucleic acid that encodes an amino acid sequence comprising the VH of the antibody. In one embodiment, the host cell is eukaryotic, *e.g*. a Chinese Hamster Ovary (CHO) cell or lymphoid cell (*e.g*., YO, NSO, Sp20). In one embodiment, a method of making an antibody of the present invention is provided, wherein the method comprises culturing a host cell comprising a nucleic acid encoding the antibody, as provided above, under conditions suitable for expression of the antibody, and optionally recovering the antibody from the host cell (or host cell culture medium).

For recombinant production of an antibody as described herein, nucleic acid encoding an antibody, *e.g*., as described above, is isolated and inserted into one or more vectors for further cloning and/or expression in a host cell. Such nucleic acid may be readily isolated and sequenced using conventional procedures (*e.g.*, by using oligonucleotide probes that are capable of binding specifically to genes encoding the heavy and light chains of the antibody).

Suitable host cells for cloning or expression of antibody-encoding vectors include prokaryotic or eukaryotic cells described herein. For example, antibodies may be produced in bacteria, in particular when glycosylation and Fc effector function are not needed. For expression of antibody fragments and polypeptides in bacteria, see, *e.g.,* U.S. Patent Nos. 5,648,237, 5,789,199, and 5,840,523. (See also Charlton, Methods in Molecular Biology, Val. 248 (B.K.C. Lo, ed., Humana Press, Totowa, NJ, 2003), pp. 245-254, describing expression of antibody fragments in *E. coli.)* After expression, the antibody may be isolated from the bacterial cell paste in a soluble fraction and can be further purified.

In addition to prokaryotes, eukaryotic microbes such as filamentous fungi or yeast are suitable cloning or expression hosts for antibody-encoding vectors, including fungi and yeast strains whose glycosylation pathways have been "humanized," resulting in the production of an antibody with a partially or fully human glycosylation pattern. See Gerngross, Nat. Biotech. 22:1409-1414 (2004), and Li et al., Nat. Biotech. 24:210-215 (2006).

Suitable host cells for the expression of glycosylated antibody are also derived from multicellular organisms (invertebrates and vertebrates). Examples of invertebrate cells include plant and insect cells. Numerous baculoviral strains have been identified which may be used in conjunction with insect cells, particularly for transfection of *Spodoptera frugiperda* cells.

Plant cell cultures can also be utilized as hosts. See, *e.g.,* US Patent Nos. 5,959,177, 6,040,498, 6,420,548, 7,125,978, and 6,417,429 (describing PLANTIBODIES^{™} technology for producing antibodies in transgenic plants).

Vertebrate cells may also be used as hosts. For example, mammalian cell lines that are adapted to grow in suspension may be useful. Other examples of useful mammalian host cell lines are macaque kidney CVI line transformed by SV40 (COS-7); human embryonic kidney line (293 or 293 cells as described, *e.g*., in Graham et al., J. Gen Viral. 36:59 (1977)); baby hamster kidney cells (BHK); mouse sertoli cells (TM4 cells as described, *e.g*., in Mather, Biol. Reprod. 23:243-251 (1980)); macaque kidney cells (CV I); African green macaque kidney cells (VER0-76); human cervical carcinoma cells (HELA); canine kidney cells (MDCK; buffalo rat liver cells (BRL 3A); human lung cells (WI38); human liver cells (Hep G2); mouse mammary tumor (MMT 060562); TRI cells, as described, *e.g*., in Mather et al., Annals M. Y Aead. Sei. 383:44-68 (1982); MRC 5 cells; and FS4 cells. Other useful mammalian host cell lines include Chinese hamster ovary (CHO) cells, including DHFR CHO cells (Urlaub et al., Proc. Natl. Acad. cii. USA 77:4216 (1980)); and myeloma cell lines such as YO, NSO and Sp2/0. For a review of certain mammalian host cell lines suitable for antibody production, see, *e.g.,* Yazaki and Wu, Methods in Molecular Biology, Val. 248 (B.K.C. Lo, ed., Humana Press, Totowa, NJ), pp. 255-268 (2003).

The antibody or the antigen-binding fragment thereof may in one embodiment include a further domain or a further amino acid sequence. For example, the antibody or the antigen-binding fragment thereof may include a localization sequence. Further examples of suitable include aldehyde tag and sortase recognition motif. The aldehyde tag is an artificial peptide tag recognized by the formylglycine-generating enzyme (FGE). A suitable example of an aldehyde tag is a tag according to sequence LCTPSR (SEQ ID NO.: 19), wherein upon FGE acting on said sequence, the cysteine residue is converted to formylglycine. The sortase recognition motif is according to sequence LPXTG (SEQ ID NO.: 20), wherein X can be any natural amino acid residue. The sortase enzyme, for example Staphylococcus aureus sortase, is a transpeptidase that attaches surface proteins to the cell wall; it cleaves between the Gly and Thr of the LPXTG motif and catalyses the formation of an amide bond between the carboxyl-group of threonine and the amino-group of the cell-wall peptidoglycan. As known to the skilled person, sortase recognition motif allows for attachment of further peptidic moieties.

As mentioned before, the immunoconjugate of the present invention comprises (b) a compound according to formula (I):

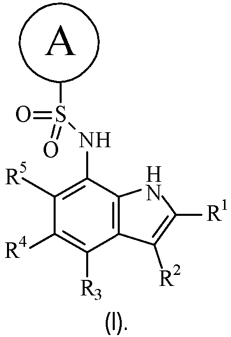

In formula (I), R¹ is selected from -H, C₁₋₅ alkyl, -OH, -CN and -NO₂. Preferably, R¹ is -H or -OH. More preferably, R¹ is -H.

In formula (I), R² is selected from -H, halogen, C₁₋₅ alkyl, C₁₋₅ haloalkyl, -OH, -CN, -NO₂, -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Preferably, R² is selected from -H, halogen, -OH, -CN, -CONH₂, - CONH(C₁₋₅ alkyl), and -CON(C₁₋₅ alkyl)(C₁₋₅ alkyl). More preferably R² is -H, halogen or -CN. Even more preferably, R² is - halogen or -CN. Again more preferably, R² is -CN. Alternatively, R² is halogen, such as -Cl..

In formula (I), R³ is selected from -H, halogen, C₁₋₅ alkyl, aryl, -OH, -O(C₁₋₅ alkyl), -CN, -NO₂, -NH₂, - NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Preferably, R³ is selected from -H, halogen, and C₁₋₅ alkyl. More preferably, R³ is selected from -H and C₁₋₅ alkyl (such as methyl). Even more preferably, R³ is C₁₋₅ alkyl (such as methyl). Again more preferably, R³ is methyl.

In formula (I), R⁴ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

In formula (I), R⁵ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Preferably, R⁴ is selected from -H and halogen. More preferably, R⁴ is -H.

In formula (I), A is a phenyl or five- or six-membered heteroaryl, optionally substituted with one or more groups selected from R^{S}. Preferably, A is a phenyl or six-membered nitrogen-containing heteroaryl, optionally substituted with one or more groups selected from R^{S}. More preferably, A is a phenyl, optionally substituted with one or more groups selected from R^{S}.

In formula (I), each R^{S} is independently selected from halogen, C₁₋₅ alkyl, C₁₋₅ haloalkyl, -OH, -O(C₁₋₅ alkyl), -(C₁₋₅ alkylene)-OH, -CN, -NH₂, -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -O-(C₁₋₅ alkylene)-NH₂, -O-(C₂₋₅ alkylene)-NH(C₁₋₅ alkyl), -O-(C₂₋₅ alkylene)-N(C₁₋₅ alkyl)(C₁₋₅ alkyl), -NO₂, -NH(C₁₋₅ alkyl), -N(C₁₋₅ alkyl)(C₁₋₅ alkyl), -CHO, -COOH, -COO(C₁₋₅ alkyl), - CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Preferably, each R^{S} is independently selected from halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, - CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Preferably, each R^{S} is independently selected from halogen, C₁₋₅ alkyl, -OH, -O(C₁₋₅ alkyl), -CN, and -NO₂. More preferably, each R^{S} is independently selected from halogen, C₁₋₅ alkyl, and -CN. Even more preferably, each R^{S} is independently selected from C₁₋₅ alkyl, and -CN. Again more preferably, each R^{S} is -CN. However, in one embodiment, each R^{S} is independently C₁₋₅ alkyl (such as methyl). In one embodiment, each R^{S} is independently halogen (such as -Cl or -F, preferably -F).

Preferably, in formula (I), A is a phenyl or five- or six-membered heteroaryl, optionally substituted with one or more groups selected from R^{S}, preferably one or more groups selected from halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), - CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

More preferably, A is a phenyl or six-membered heteroaryl, optionally substituted with one or more groups selected from R^{S}, preferably one or more groups selected from halogen, C₁₋₅ alkyl, -OH, -O(C₁₋₅ alkyl), - CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl). Said six-membered heteroaryl is preferably a six-membered nitrogen containing heteroaryl, such as pyridinyl. Thus, in one embodiment, A is six-membered nitrogen containing heteroaryl (preferably a pyridyl), optionally substituted with one or more groups selected from R^{S}, preferably one or more groups selected from halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, - COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

Even more preferably, A is phenyl optionally substituted with one or more groups selected from R^{S}, preferably one or more groups selected from -H, halogen, C₁₋₅ alkyl, -OH, -O(C₁₋₅ alkyl), -CN, -NO₂, - NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl), preferably a phenyl substituted with methyl or a phenyl substituted with -CN.

In one embodiment, A is a five-membered heteroaryl, optionally substituted with one or more groups selected from R^{S}, preferably one or more groups selected from halogen, C₁₋₅ alkyl, -OH, -O(C₁₋₅ alkyl), - CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl).

Particularly preferred A is selected from 3-cyanophenyl, 3-cyanopyrid-5-yl, 2-cyano-3-fluorophenyl, 3-cyano-4-hydroxyphenyl, 4-aminomethylphenyl, 4-N-methyl-aminomethylphenyl, and 5-bromothien-2-yl.

Particularly preferred are embodiments of the compound of formula (I), wherein A is as defined hereinabove, including any preferred definition of A and any individual and specific disclosed embodiment of A, including each and every particularly preferred A as recited above, and wherein:
R¹ is -H, R² is -CN, R³ is -H, R⁴, is H, and R⁵ is H;
R¹ is -H, R² is -CN, R³ is -methyl, R⁴, is H, and R⁵ is H;
R¹ is -H, R² is -Cl, R³ is -H, R⁴, is H, and R⁵ is H;
R¹ is -H, R² is -Cl, R³ is -methyl, R⁴, is H, and R⁵ is H;
R¹ is -H, R² is -Cl, R³ is -Cl, R⁴, is H, and R⁵ is H;

It is particularly preferred that the compound of formula (I) is selected from: 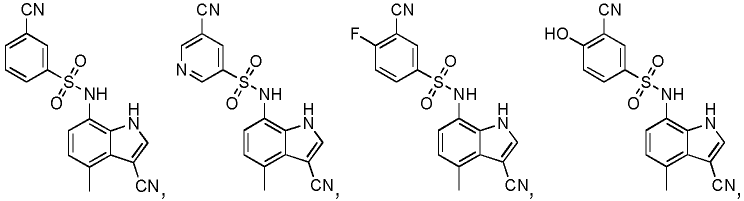 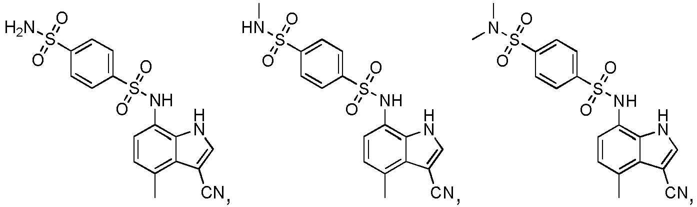 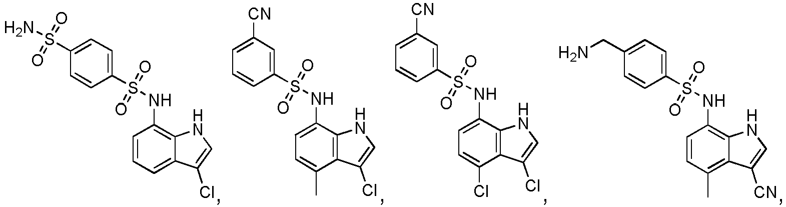 and

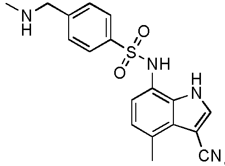

Further suitable compound of formula (I) is

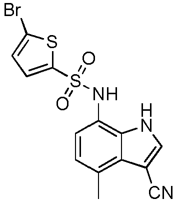

The compound of formula (I), as referred to herein, is capable of inducing degradation of RBM39, when contacted with RBM39 in the cell. Degradation here may refer to Ubiquitin induced degradation through proteasomal machinery, e.g. 26S proteasome, or degradation through any other mechanism that is apparent to the skilled person. The degradation of RBM39 manifests itself through reduced level of RBM39 in the cell, preferably to an extent that less than 10% of control level of RBM39 is present, more preferably to an extent that less than 1 % of control level of RBM39 is present, even more preferably to an extent that no RBM39 can be detected anymore. Accordingly, when reference is made to the biological or technical effect of RBM39, it means a reference to its ability to induce the degradation of RBM39. Further accordingly, when reference is made to interaction between the compound of formula (I) and RBM39, a reference is made to an effect of causing said degradation of RBM39. As it is apparent to the skilled person, and as it follows from the following, the interaction between the compound of formula (I) and RBM39 is preferably a ternary interaction involving the compound of formula (I), RBM39 and DCAF15, an E3 ubiquitin ligase.

Three recent publications have provided detailed structural evaluation of the binding mechanism of aryl sulfonamide drugs E7820^{24,25} and indisulam,²⁶ respectively and results and observations made in these studies are summarized below. According to one of these works (see Fig. 1), the two sulfonyl oxygens of E7820 form hydrogen bonds with the backbone amide nitrogens on Ala234 and Phe235 AA side chains of DCAF15, while the indole nitrogen and sulfonamide nitrogen form extensive water-mediated hydrogen bonds with the side chain oxygens on Thr262 and Asp264 of RBM39. Additional hydrogen bonds between the indole nitrogen and backbone carbonyl oxygen of DCAF15 Phe231 together with the hydrophobic interactions of the C4 methyl of E7820 with Val477 and Val556 AA residues of DCAF15 are crucial for the high affinity to DCAF15. Swapping the methyl for a hydrogen, as in indisulam or desmethyl-E7820, results in a substantial loss of DCAF15 binding. The phenyl ring forms a T-shaped π-π interaction with Phe235 of DCAF15 and is situated in a spacious pocket allowing for structural diversity as observed in indisulam.

As for indisulam (see Fig. 2), the chloro-indole group of indisulam binds in a hydrophobic pocket comprising of Thr230, Phe235 and Val559 of DCAF15, as well as Met265 of RBM39. The phenyl-sulfon amide is positioned between several aliphatic side chains, including Ala234, Thr262 of DCAF15 and Met265 side chain of RBM39. The central sulfonamide accepts two hydrogen bonds from the main chain amides of DCAF15 Ala234 and Phe235 and its nitrogen forms water-mediated hydrogen bonds with both Thr262 and Asp264 of RBM39.

A compound capable of inducing degradation of RBM39 is accordingly a compound, which contacted with RBM39 in the cell, induces degradation of RBM39, as described herein. Particularly preferred compounds that are RBM39 degraders are the compounds of formula (I), as referred to herein and as defined herein. However, skilled person will recognize that other degraders of RBM39 exist and may also be used in the immunoconjugate. For example, suitable RBM39 degrades are further disclosed in US 11,807,606 B2. Further suitable examples of RBM39 degraders are disclosed in WO 2024/039689. Further suitable RBM39 degraders are disclosed in WO 2022/169755 and WO 2024/129634.

It is to be understood that the compound of formula (I), as defined herein, including any individually disclosed compound of formula (I), are attached to (a), preferably to the antibody in (a). To this end, the skilled person will understand that formally the compound of formula (I) is to be transformed into a monovalent radical, or, in other words, into a monovalent group (or monovalent moiety), by detachment of an H atom from the compound. Particular selection of the attachment point depends on available chemistry for attachment to (a). Furthermore, particular selection of attachment point and applied chemistry must be tolerated from the point of view of interaction with RBM39.

In order to facilitate the chemical linker attachment and antibody bioconjugation, E7820, the most potent hit from the degrader payload screen was modified with an aminomethyl functionality according to the published procedure²⁶ to obtain compound AMe-deCN-E7820/JKW-48 and its cytotoxic activity was tested in neuroblastoma cell lines using cell viability assay. Such modification leads to decrease in the anti-cancer activity of JKW-48 compared to the parent molecule E7820.

Accordingly, to further improve the potency of the RBM39 degrader payload, based on the above observations and literature review, molecules with chemical structures consisting of the 7-amino-indole core of either E7820 or indisulam combined with substituted or modified benzenesulfonamide moieties based on either E7820 or indisulam (see Table 2 below) and methylated derivatives were proposed, designed and synthesized.

**Table 2. In vitro biological activity (EC₅₀ [nM]) of synthesized RBM39 SM degraders in selected neuroblastoma cell lines compared to investigational RBM39 degrader drugs E7820 or indisulam.**

| | **E7820** | **JKW-48** | **indisulam** |
|---|---|---|---|
| | 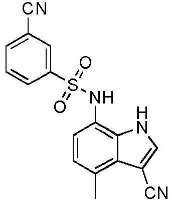 | 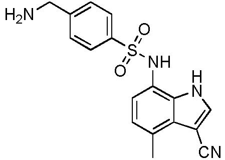 | 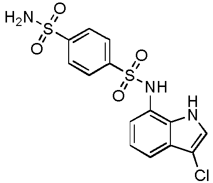 |
| SK-N-SH | 48.9 | 182.2 | 152.6 |
| STA-NB-1.2 | 28.7 | 51.9 | 79.6 |
| STA-NB-8 | 11.1 | 12.7 | 33.7 |
| IMR-32 | 45.9 | 99.7 | 96.1 |

The aim of this SAR study was to assess the effect of the indole moiety and aryl ring substitution on the biological/anticancer activity of this class of RBM39 molecular glue degraders. E7820 indole moiety was combined with the indisulam aryl moiety and vice versa (similar analogy was used for the bromo-thienyl portion of tasisulam, another reported RBM39 degrader). It appears that indole moieties bearing methyl and cyano group substituents are more favored. Investigated also was the effect of the methyl and chloride substitution on the indole ring. Methylation of the terminal sulfonamide on indisulam should lead to increased hydrophobicity of the molecule which might be beneficial for the degradation activity. Finally, cyano substituent on the phenyl ring seems to be important for the potency of the degrader and therefore it was retained whilst additional substituents which could potentially change the aromaticity of the phenyl ring (e.g., enhancing the T-shaped pi-pi interaction with F235 on DCAF15) or would allow for the formation of additional hydrogen bonds with the nearby amino acid residues were introduced.

Accordingly, and as demonstrated by the present inventors, the compound of formula (I) can be attached to (a), preferably to the antibody of (a), through N atom in the sulfonamide moiety of the compound of formula (I). Thus, the present invention relates to an embodiment, wherein the compound of formula (I) is present in the immunoconjugate as a moiety of formula (Ia): 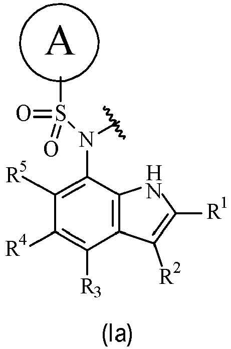 wherein R¹, R², R³, R⁴, R⁵ and A are as defined for formula (I).

In formula (Ia), it is preferred that R¹, and/or R⁴, and/or R⁵ are each independently -H. More preferably, R¹, R⁴, and R⁵ are each -H.

In formula (Ia), It is further preferred that R², is halogen (such as -CI) or -CN. More preferably, R² is -CN. Alternatively, R² is halogen (such as -CI), more preferably, R² is -Cl.

In formula (Ia), preferably R³ is C₁₋₅ alkyl (such as methyl). More preferably, R³ is methyl.

Thus, in one particularly preferred embodiment, the moiety of formula (Ia) is a moiety of formula (la-1):

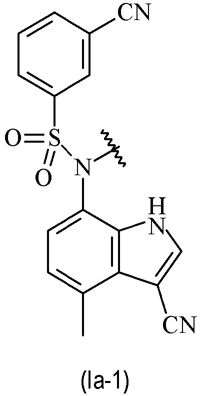

Alternatively, and as demonstrated by the present inventors, the compound of formula (I) can be attached to (a), preferably to the antibody of (a), through A ring, for example through amino group of the A moiety being (optionally substituted) 4-aminomethylphenyl. Thus, accordingly, the present invention relates to the immunoconjugate of the present invention, wherein the compound of formula (I) is present in the immunoconjugate as a moiety of formula (Ib):

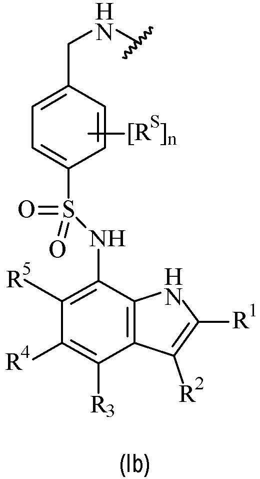

In formula (Ib) R¹, R², R³, R⁴, and R⁵ are as defined for formula (I), R^{S} is as defined for formula (I), preferably R^{S} selected from halogen, C₁₋₅ alkyl, C₁₋₅ haloalkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl), and n is an integer from 0 to 4.

Accordingly, and preferably, in formula (Ib), R¹, and/or R⁴, and/or R⁵ are each independently -H. More preferably, R¹, R⁴, and R⁵ are each -H.

In formula (Ib), It is further preferred that R², is halogen (such as -CI) or -CN. More preferably, R² is -CN. Alternatively, R² is halogen (such as -CI), more preferably, R² is -Cl.

In formula (Ib), preferably R³ is C₁₋₅ alkyl (such as methyl). More preferably, R³ is methyl.

In formula (Ib), preferably n is 0 or 1. More preferably, n is 0. Accordingly, the phenyl ring in A in formula (Ib) is not further substituted.

In a particularly preferred embodiment, the moiety of formula (Ib) is a moiety of formula (lb-1), as outlined in the following:

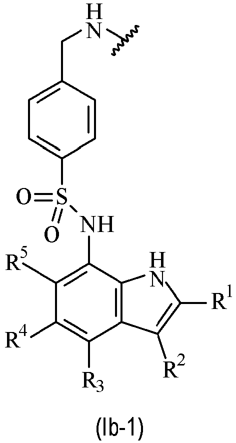

In formula (lb-1), R¹, R², R³, R⁴, and R⁵ are as for formula (Ib), and wherein n is an integer from 0 to 4.

Accordingly, and preferably, in formula (Ib), that R¹, and/or R⁴, and/or R⁵ are each independently -H. More preferably, R¹, R⁴, and R⁵ are each -H.

In formula (Ib), It is further preferred that R², is halogen (such as -CI) or -CN. More preferably, R² is -CN. Alternatively, R² is halogen (such as -CI), more preferably, R² is -Cl.

In formula (Ib), preferably R³ is C₁₋₅ alkyl (such as methyl). More preferably, R³ is methyl.

Thus, in a particularly preferred embodiment, the moiety of formula (lb-1) is a moiety of formula (lb-2), as outlined in the following

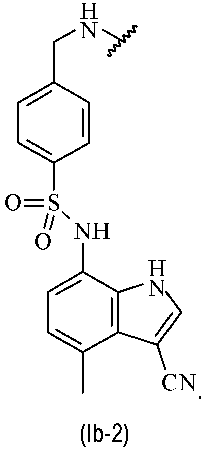

The attachment of the compound of formula (I) (or, more broadly speaking, of a RBM39 degrader) to (a), such as to an antibody of (a), is not to be particularly limited in any way, and accordingly, any feasible attachments that are apparent to the skilled person, are encompassed. Particularly preferred are attachments as shown in formulae (Ia) and following, as well as (Ib) and the following.

It is to be understood that the compound of formula (I) may be attached to (a), such as antibody in (a), directly, or it may be attached through a particular linker. Preferably, the compound of formula (I) is attached to (a) through a linker.

The linker moiety (or the linker), as referred to herein, is not meant to be particularly limited and any linker that is conceivable to the skilled person is considered encompassed by the present invention.

In the immunoconjugate of the present invention, particularly preferred is a linker comprising a moiety according to formula (II):

-L₁-L_{P}-L₂-X- (II)

which is defined in the following. Accordingly, and in other words, in one embodiment of the present invention, the immunoconjugate comprises a moiety according to formula (II):

-L₁-L_{P}-L₂-X- (II).

In formula (II), L₁ is C₂₋₂₀ alkylene, optionally substituted with one or more substituents selected from -CN, halogen, -OH, and -NH₂, and wherein one or more -CH₂- groups may independently be replaced by a group selected from -CH=CH-, -C=C-, -O-, -NR'-, -CO-, C₆₋₁₀ arylene, 5-6 membered heteroarylene, - (CH₂-O-CH₂)-, -(CH₂-CH₂-O)-, -CO-O-, -CO-NR'-, C₃-C₁₁ cycloalkylene, or 4-11 membered heterocycloalkylene, wherein R' is H or C₁-C₆ alkyl.

To the skilled person, it is immediately apparent that L₁ is a bivalent moiety, or in other words a moiety with two attachment points. Accordingly, it is to be understood that L₁ is attached to (a), in particular to the antibody of (a), and L₁ is attached to L_{P}.

Preferably, L₁ is C₂₋₁₂ alkylene, optionally substituted with one or more substituents selected from -CN, halogen, -OH, and -NH₂, and wherein one or more -CH₂- groups may independently be replaced by a group selected from -O-, -NR'-, -CO-, -(CH₂-O-CH₂)-, -(CH₂-CH₂-O)-, C₃-C₁₁ cycloalkylene, and 4-11 membered heterocycloalkylene, wherein R' is H or C₁-C₆ alkyl.

More preferably, L₁ is C₂₋₁₂ alkylene, wherein one or more -CH₂- groups may independently be replaced by a group selected from -CO-, -(CH₂-O-CH₂)-, -(CH₂-CH₂-O)-, and C₃-C₁₁ cycloalkylene.

Accordingly, different L₁ are conceivable to the skilled person. For example, L₁ is selected from:

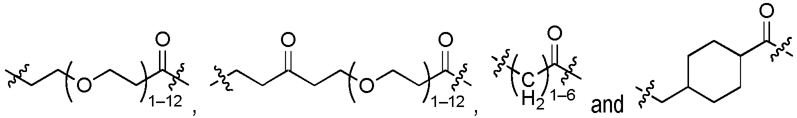

It is to be understood that, as L₁ is a bivalent moiety, and includes two attachment points, its directionality is to be defined. Accordingly, it is preferred that the right empty valence of L₁ as shown herein, is attached to L_{P}. It follows that the left empty valence of L₁ is preferably attached to (a), in particular to the antibody of (a).

In one embodiment, L₁ is 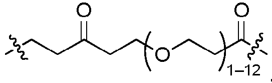 It is preferred that the right empty valence of L₁ as shown herein, is attached to L_{P}. It follows that the left empty valence of L₁ is preferably attached to (a), in particular to the antibody of (a).

In one embodiment, L₁ is 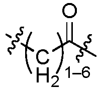 It is preferred that the right empty valence of L₁ as shown herein, is attached to L_{P}. It follows that the left empty valence of L₁ is preferably attached to (a), in particular to the antibody of (a).

In one embodiment, L₁ is 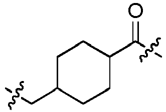 It is preferred that the right empty valence of L₁ as shown herein, is attached to L_{P}. It follows that the left empty valence of L₁ is preferably attached to (a), in particular to the antibody of (a).

It is however particularly preferred that in the immunoconjugate of the present invention, as defined herein, comprising the moiety of formula (II), the L₁ is 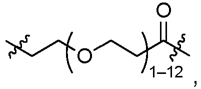 wherein the right empty valence of L₁ is attached to L_{P}.

More preferably, in said immunoconjugate, the L₁ is 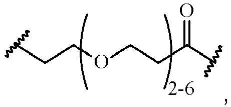 wherein the right empty valence of L₁ is attached to L_{P}.

Even more preferably, in said immunoconjugate, the L₁ is 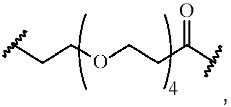 wherein the right empty valence of L₁ is attached to L_{P}.

As apparent to the skilled person, L₁ may also be a trivalent moiety, such as the following moiety: 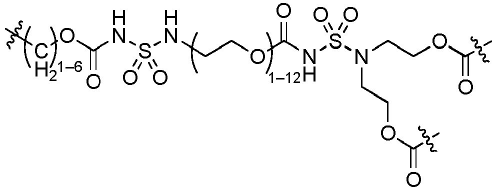 wherein the left side is attached to antibody, and two empty valences on the right side are attached to a different occurrence of L_{P}.

In formula (II), L_{P} is a bond or is a peptide linker which includes up to 5 amino acid residues, wherein the C-terminus of the peptide linker is connected to -NH- moiety of L₂ or to X. It is accordingly to be understood that the N-terminus of the peptide linker is connected to L₁. It is further preferred that L₁ comprises a -CO-moiety, through which it is attached to Lₚ. Accordingly, it is preferred that L₁ and L_{P} are connected with each other through an amide bond (-CO-NH-).

L_{P} is preferably a peptide linker which includes up to 5 amino acid residues. Accordingly, L_{P} may include 1, 2, 3, 4, or 5 amino acid residues. Preferably, L_{P} includes between 2 and 4 amino acid residues. Accordingly, L_{P} may include 2, 3 or 4 amino acid residues.

Suitable peptide linkers as L_{P} include -Val-Cit-, -cBu-Cit-, -Val-Ala-, -Glu-Val-Cit-, -Gly-Gly-Gly-, -Ala-Ala-Asn- and -Gly-Gly-Phe-Gly-, wherein the C-terminus of the peptide linker is connected to -NH- moiety of L₂ or to X, and wherein the N-terminus is connected to L₁.

As understood herein, -Val-Cit- is a peptide linker according to formula:

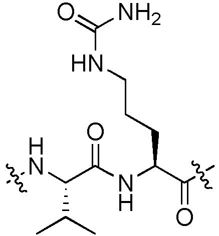

As understood herein, -cBu-Cit- includes a modified amino acid residue comprising a 1,1-cyclobutylene moiety. This peptide linker is according to formula:

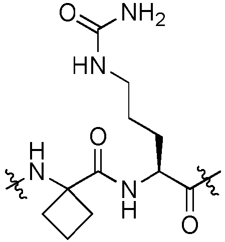

As understood herein, -Glu-Val-Cit- is a peptide linker according to formula:

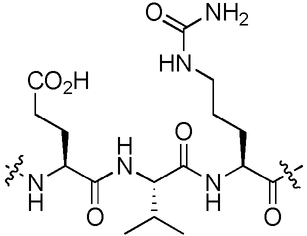

Thus, preferably L_{P} is selected from -Val-Cit-, -cBu-Cit-, -Val-Ala-, -Glu-Val-Cit-, -Gly-Gly-Gly-, -Ala-Ala-Asn- and -Gly-Gly-Phe-Gly-, wherein the C-terminus of the peptide linker is connected to -NH- moiety of L₂ or to X, and wherein the N-terminus is connected to L₁.

Particularly preferred L_{P} is -Gly-Gly-Phe-Gly-, wherein the C-terminus of the peptide linker is connected to -NH- moiety of L₂ or to X, and wherein the N-terminus is connected to L₁.

However, in one embodiment, the peptide linker L_{P} is -Val-Cit-, wherein the C-terminus of the peptide linker is connected to -NH- moiety of L₂ or to X, and wherein the N-terminus is connected to L₁.

Alternatively, in one embodiment, L_{P} is -Ala-Ala-Asn-, wherein the C-terminus of the peptide linker is connected to -NH- moiety of L₂ or to X, and wherein the N-terminus is connected to L₁.

In formula (II), L₂ is -NH-1,4-phenylene-CH₂- or a bond. Preferably, L₂ is -NH-1,4-phenylene-CH₂-. In other words, L₂ is preferably a moiety according to formula:

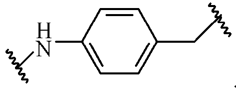

It is to be understood that L₂ is to be attached to L_{P} (or, if L_{P} is a bond, to L₁), through its -NH- group. Thus, it is to be understood that L₂ is preferably to be attached to L_{P} (or, if Lp is a bond, to L₁), through an amide bond.

In formula (II), X is selected from a bond, -O-, -O-CO-, -CO-O-, and -NH-, wherein X is attached to the compound of formula (I).

Preferably, X is -O-CO-, wherein the right empty valence of said -O-CO- is attached to the compound of formula (I). In this embodiment, is it particularly preferred that (b) the compound of formula (I) is present as a moiety according to formula (Ib). Thus, it this specific embodiment, wherein X is -O-CO-, the right empty valence of said -O-CO- is connected to the moiety of formula (Ib).

Alternatively, X is a bond. It is particularly preferred that (b), the compound of formula (I), is present as a moiety according to formula (Ia). Thus, in this specific embodiment, wherein X is a bond, the (b) is according to formula (Ia).

Different strategies for attachment to (a) or, more specifically, the antibody of (a), are contemplated. Particularly preferred in the context of the present invention is site-specific antibody conjugation via Cys-thiol reaction. In this context particularly preferred is maleimide-thiol conjugation, which allows for the attachment of payload-containing compounds to Cys residues in (a), and in particular in the antibody of (a). In the course of such reaction, the conjugate is formed comprising a moiety according to formula (Ila):

-Z-L₁-L_{P}-L₂-X- (Ila).

In formula (Ila), Z is 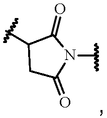 attached through its right empty valence to L₁, and through its left empty valence to a cysteine residue of the antibody.

Furthermore, in formula (Ila), L₁, L_{P}, L₂, and X are as defined in formula (II), including any specific embodiment of this formula or any preferred definition of formula (II).

Accordingly, in other words, preferably in the immunoconjugate of the present invention, the moiety of formula (II) is comprised in a moiety of formula (Ila):

-Z-L₁-L_{P}-L₂-X- (lla),

as defined herein.

The immunoconjugate may also be defined according to the process of its production, and in particular through substrates of the conjugation reaction and the reaction itself. Accordingly, in one preferred embodiment, the immunoconjugate of the present invention is obtainable in the reaction of the compound comprising a moiety of formula

Z₁-L₁-L_{P}-L₂-X- (IIb)

with -SH group of cysteine of the antibody.

In formula (IIb), Z₁ is a group that can react (i.e., is capable of reacting) with -SH of cysteine to form a covalent bond with said cysteine. Such group may be an unsaturated group, in particular unsaturated group including at least one double or triple bond. Such group may be a Michael acceptor, which is known to the skilled person to react with -SH group of cysteine. In this content, it is preferred that Z₁ is selected from 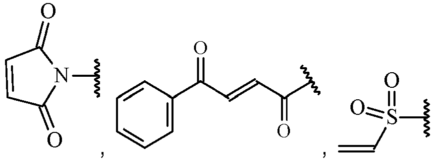 and

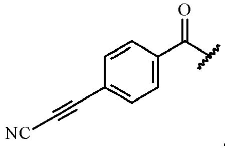

Alternatively, Z₁ may be a phosphonamidite-based group, i.e., a group selected from 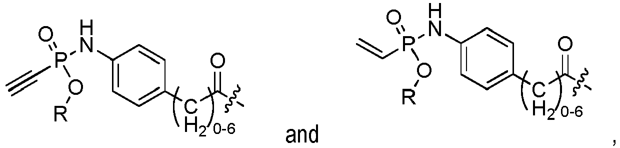 wherein R is -(CH₂CH₂O)₁₋₂₃-CH₂CH₂OH or -(CH₂CH₂O)₁₋₂₃-CH₂CF₃.

Preferably however, Z₁ is selected from 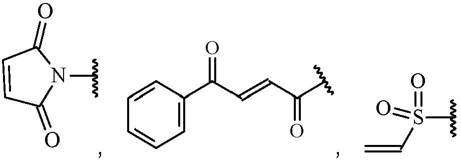 and

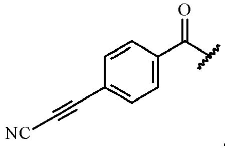

More preferably, in formula (Ilb), Z₁ is

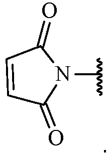

In formula (IIb), L₁, L_{P}, L₂, and X are as defined for the formula (II), including any specific embodiment of formula (II) and any preferred definition of formula (II).

Alternatively, in order to achieve homogenous labeling and obtain immunoconjugates with an average DAR 4, the compound of formula (I) (or, referring to more generally, RBM39 degrader) can be attached to the antibody using a bioconjugation protocol consisting of a controlled TCEP (tris(2-carboxyethyl)phosphine)-mediated antibody disulfide bond reduction followed by a site-specific bioconjugation via rebridging of disulfide of a compound comprising a moiety of formula (Ilc):

Z₂-L₁-L_{P}-L₂-X- (IIc).

In formula (IIc), Z₂ is a group that can react with two -SH of cysteine to form a covalent bond with each cysteine. Preferably, Z₂ is selected from 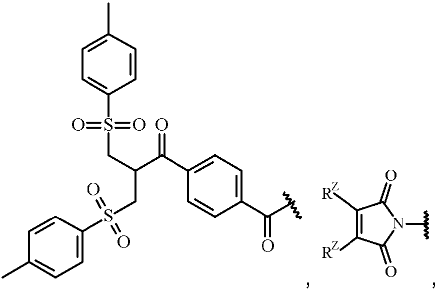 wherein each R^{Z} independently is Hal (such as Cl, Br or I) or -S-aryl (such as -S-phenyl), and 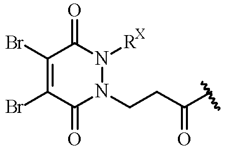 , wherein R^{X} is C₁₋₅ alkyl (such as methyl or ethyl), C₂₋₅ alkenyl or C₂₋₅ alkynyl (such as propargyl).

In formula (IIc), L₁, L_{P}, L₂, and X are as defined in formula (II), including any specific embodiment of formula (II) and any preferred definition of formula (II).

As however mentioned before, the strategy for linking the RBM39 degrader is not meant to be particularly limited and any particular strategy recognizable to the skilled person can be used in the immunoconjugates of the present invention.

For example, for the compounds being hydrophobic payloads, a conjugation relying on self-immolative linker bearing a moiety derived from glucuronic acid can be employed. Accordingly, such conjugate is released in the cell according to β-glucuronidase-based mechanism, followed by 1,6-elimination, which is summarized in the following scheme:

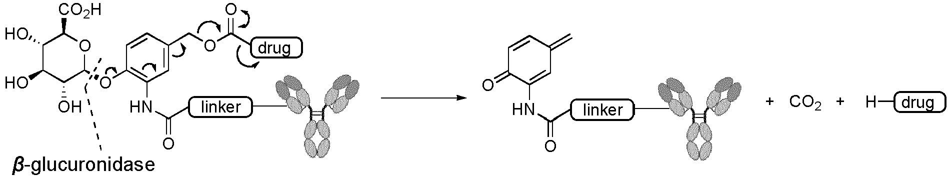

Accordingly, in one embodiment, the immunoconjugate of the invention includes the moiety of formula 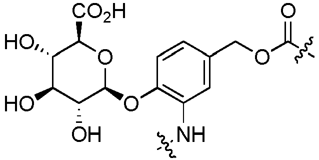 wherein the RBM39 degrader is attached to an empty valence of carbonyl group, and wherein the antibody is attached (directly or indirectly, for example via -Z-L₁-, as described herein) to an empty valence of -NH-group.

In one embodiment, the immunoconjugate of the invention includes the moiety of formula 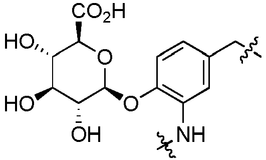 wherein the RBM39 degrader is attached to an empty valence of methylene group (preferably through nitrogen atom of a sulfonamide group), and wherein the antibody is attached (directly or indirectly, for example via -Z-L₁-, as described herein) to an empty valence of -NH- group.

Herein, the drug refers to a compound of formula (I), or more generally, an RBM39 degrader. Alternatively, a linker comprising a moiety derived from galactose may be used, which is released in the cell in a β-galactosidase-catalyzed reaction, as shown in the following:

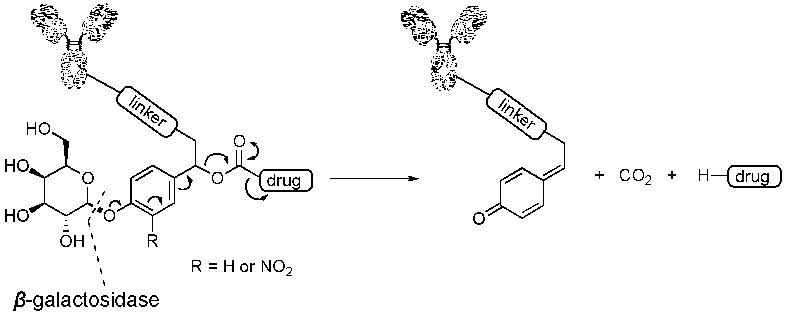

Accordingly, in one embodiment, the immunoconjugate of the invention includes the moiety of formula 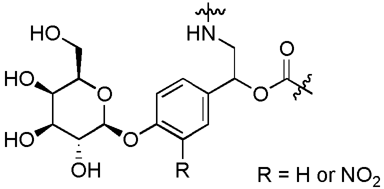 wherein the RBM39 degrader is attached to an empty valence of carbonyl group, and wherein the antibody is attached (directly or indirectly, for example via -Z-L₁-, as described herein) to an empty valence of -NH-group.

In one embodiment, the immunoconjugate of the invention includes the moiety of formula 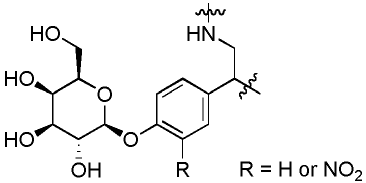 wherein the RBM39 degrader is attached to an empty valence of CH group (preferably through nitrogen atom of a sulfonamide group), and wherein the antibody is attached (directly or indirectly, for example via -Z-L₁-, as described herein) to an empty valence of -NH- group.

Encompassed by the present invention are further bioconjugation strategies, for example a strategy based on enzymatic glycan remodeling followed by metal-free click-chemistry attachment, as described in Wijdeven et al., 2002, mAbs, 14(1), e2078466, https://doi.org/10.1080/19420862.2022.2078466.

Further labeling strategies may be based on introducing particular sequences in the protein that can undergo further modification. The examples of such suitable sequences include aldehyde tag and sortase recognition motif.

The aldehyde tag is an artificial peptide tag recognized by the formylglycine-generating enzyme (FGE). A suitable example of an aldehyde tag is a tag according to sequence LCTPSR (SEQ ID NO.: 19), wherein upon FGE acting on said sequence, the cysteine residue is converted to formylglycine, which can be used in further attachment strategies.

The sortase recognition motif is according to sequence LPXTG (SEQ ID NO.: 20), wherein X can be any natural amino acid residue. The sortase enzyme, for example *Staphylococcus aureus* sortase, is a transpeptidase that attaches surface proteins to the cell wall; it cleaves between the Gly and Thr of the LPXTG motif and catalyses the formation of an amide bond between the carboxyl-group of threonine and the amino-group of the cell-wall peptidoglycan. As known to the skilled person, sortase recognition motif allows for attachment of further peptidic moieties, and can also be used for conjugating the compound of formula (I) to the antibody, to yield an immunoconjugate, as provided herein.

It is further apparent to the skilled person that labeling strategy based on transglutaminase can be used for attaching the metal chelator to the antibody. Transglutaminases catalyze an acyl-transfer reaction to the side chain of glutamine residues of their protein substrate. Depending on the acyl donor, this can result in an amide bond between the glutamine and a primary amine, crosslinking between two proteins via a side chain lysine of the donor protein or the deamidation of glutamine. For protein labelling purposes, the acyl-transfer reaction is preferred. While transglutaminases are specific for glutamine on the target protein, the flexibility in terms of the amine containing acyl-donor offers diverse possibilities for modification. In contrast to other protein-ligation strategies, the probe containing reactant is not required to be a peptide and can simply be an alkylamine or an oligoamine as long as it contains a primary amine. For example, the compound of formula (I) can be attached to a peptide comprising a peptide sequence suitable for attachment to the antibody via transglutaminase-catalyzed reaction. One example of such sequence is RAKAR sequence (SEQ ID NO.: 21). Further such sequences are disclosed in WO 2019/057772.

The antibody may also optionally include one or more non-canonical amino acids to be used for coupling of said antibody with another chemical entity. Said amino acids may include residues that would be reactive in addition reactions known to the skilled person as bioorthogonal chemistry approaches. Of these of particular importance is click chemistry, well known to the skilled person. Suitable examples of such residues include residues comprising azide moiety, or cyclooctyne moiety or a moiety being capable to perform an inverse-demand Diels-Alder cycloaddition reaction e.g., a trans-cyclooctene / tetrazine reaction pair. However, the invention is not meant to be limited to any of these examples, and other such residues known to the skilled person may also be used. The methods of producing antibodies (or fragments thereof, as applicable) comprising non-canonical amino acid residue(s) using recombinant methods are known to the skilled person.A further suitable example of biorthogonal chemistry approach is sulfur (VI) fluoride exchange chemistry.

The present invention is further not limited in any way with respect to the compound of formula (I)-antibody ratio (also referred to as drug-antibody ratio, i.e., DAR), and any obtainable ratio can be used in the immunoconjugate of the present invention. For example, the drug/antibody ratio may be between 1 and 8, preferably between 3 and 5, such as 3.0, 3.1, 3.2, .3.3, 3.4, 3.5, 3.6, 3.7, 3.8, 3.9, 4.0, 4.1, 4.2, 4.3, 4.4, 4.5, 4.6, 4.7, 4.8, 4.9 or 5.0. Preferably, said ratio is between 3.5 and 4.5. Particularly preferred is ratio of about 4.0, such as 4.0. Alternatively, it is preferred that the drug/antibody ratio is between 5 and 7, such as 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, or 7.0. Particularly preferred ratio is about 6.0, such as 6.0. However, the drug antibody ratio can also be between 7 and 8, such as 7.0, 7.1, 7.2, 7.3, 7.4, 7.5, 7.6, 7.7, 7.8, 7.9, or 8.0. Particularly preferred ratio is about 8.0, such as 8.0.

Particularly preferred immunoconjugate of the present invention comprises:
(a) dinutuximab beta antibody (ch14.18/CHO, Qarziba^{®}), i.e., antibody comprising a heavy chain comprising an amino acid sequence according to SEQ ID NO.: 15 and comprising a light chain comprising an amino acid sequence according to SEQ ID NO.: 16, and
(b) a moiety of formula (lb-2), as outlined in the following

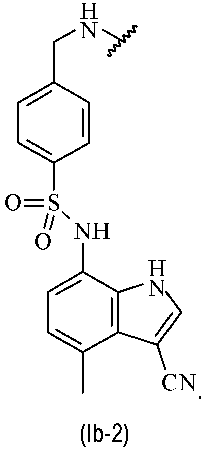

Preferably, the antibody further comprises a moiety according to formula (Ila):

-Z-L₁-L_{P}-L₂-X- (IIa).

wherein Z is 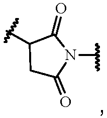 attached through its right empty valence to L₁, and through its left empty valence to a cysteine residue of the antibody;
L₁ is 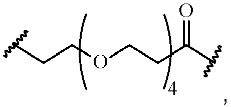 wherein the right empty valence of L₁ is attached to L_{P}, and the left empty valence is attached to Z;
L_{P} is -Gly-Gly-Phe-Gly-, wherein the C-terminus of the peptide linker is connected to -NH- moiety of L₂, and wherein the N-terminus is connected to L₁;
L₂ is a moiety according to formula 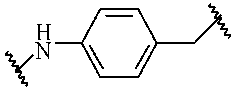
wherein L₂ is attached to L_{P} through its -NH- group;
X is -O-CO-, and the right empty valence of said -O-CO- is connected to (b). It is to be understood that the right empty valence of said -O-CO- is connected to the empty valence in (Ib-2) formula.

Particularly preferred immunoconjugate of the invention may also be defined as being obtainable in a reaction of a compound according to formula: 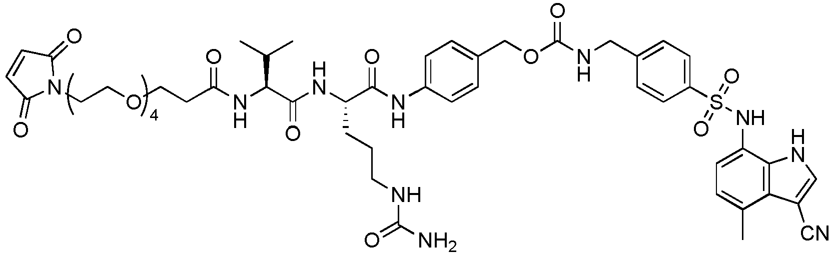 and dinutuximab beta antibody (ch14.18/CHO, Qarziba^{®}), i.e. antibody comprising a heavy chain comprising an amino acid sequence according to SEQ ID NO.: 15 and comprising a light chain comprising an amino acid sequence according to SEQ ID NO.: 16.

In one embodiment, the present invention relates to a compound of formula (III):

Z_{Z}-L₁-L_{P}-L₂-X-Y (III)

or a pharmaceutically acceptable salt thereof.

In formula (III), Z is Z₁ or Z₂. Preferably, Z is Z₁.

In formula (III), Z₁ is selected from 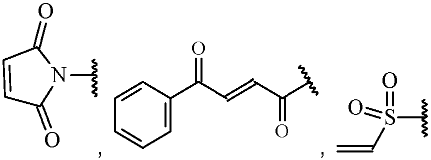 and

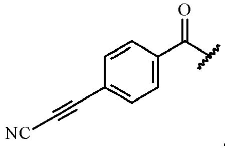

Alternatively, Z₁ may be a phosphonamidite-based group, i.e., a group selected from 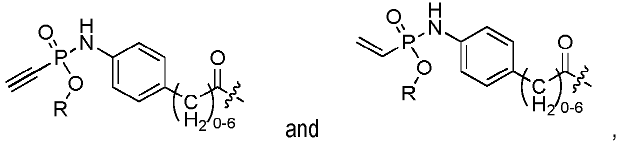 wherein R is -(CH₂CH₂O)₁₋₂₃-CH₂CH₂OH or -(CH₂CH₂O)₁₋₂₃-CH₂CF₃. It is to be understood that notation 0-6 refers to an integer from 0 to 6, i.e. any one of 0, 1, 2, 3, 4, 5, and 6. Similarly, notation 1 to 23 refers to an integer from 1 to 23, including any one of 1, 2, 3, 4, 5, 6, 7, 8, 9, 10, 11, 12, 13, 14, 15, 16, 17, 18, 19, 20, 21, 22, and 23.

Preferably, Z₁ is selected from 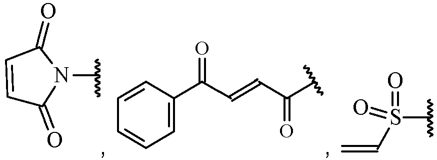 and

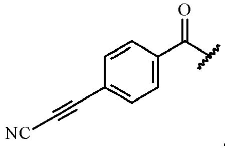

More preferably, Z₁ is

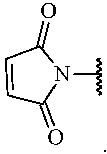

In formula (III), Z₂ is selected from 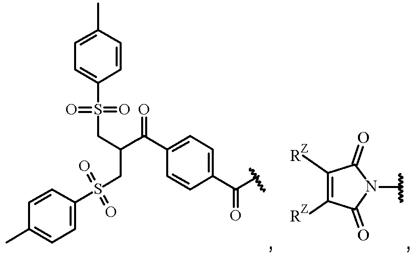 wherein each R^{Z} independently is Hal (such as Cl, Br or I) or -S-aryl (such as -S-phenyl), and 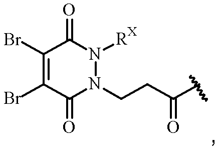 wherein R^{X} is C₁₋₅ alkyl (such as methyl or ethyl), C₂₋₅ alkenyl or C₂₋₅ alkynyl (such as propargyl).

In formula (III), L₁, L_{P}, L₂, and X are as defined for formula (II), including any specific embodiment of formula (II) and including any preferred definition of formula (II).

In formula (III), Y is the moiety of formula (Ia), including any specific embodiments and preferred definitions of this formula, or the moiety of formula (Ib), including any specific embodiments and preferred definitions of the formula.

The compound of formula (III) is useful for attaching the compound of formula (I) to the antibody.

The scope of the invention embraces all pharmaceutically acceptable salt forms of the compounds of formula (III) which may be formed, e.g., by protonation of an atom carrying an electron lone pair which is susceptible to protonation, such as an amino group, with an inorganic or organic acid, or as a salt of an acid group (such as a carboxylic acid group) with a physiologically acceptable cation. Exemplary base addition salts comprise, for example: alkali metal salts such as sodium or potassium salts; alkaline earth metal salts such as calcium or magnesium salts; zinc salts; ammonium salts; aliphatic amine salts such as trimethylamine, triethylamine, dicyclohexylamine, ethanolamine, diethanolamine, triethanolamine, procaine salts, meglumine salts, ethylenediamine salts, or choline salts; aralkyl amine salts such as N,N-dibenzylethylenediamine salts, benzathine salts, benethamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctylammonium salts or tetrabutylammonium salts; and basic amino acid salts such as arginine salts, lysine salts, or histidine salts. Exemplary acid addition salts comprise, for example: mineral acid salts such as hydrochloride, hydrobromide, hydroiodide, sulfate salts (such as, e.g., sulfate or hydrogensulfate salts), nitrate salts, phosphate salts (such as, e.g., phosphate, hydrogenphosphate, or dihydrogenphosphate salts), carbonate salts, hydrogencarbonate salts, perchlorate salts, borate salts, or thiocyanate salts; organic acid salts such as acetate, propionate, butyrate, pentanoate, hexanoate, heptanoate, octanoate, cyclopentanepropionate, decanoate, undecanoate, oleate, stearate, lactate, maleate, oxalate, fumarate, tartrate, malate, citrate, succinate, adipate, gluconate, glycolate, nicotinate, benzoate, salicylate, ascorbate, pamoate (embonate), camphorate, glucoheptanoate, or pivalate salts; sulfonate salts such as methanesulfonate (mesylate), ethanesulfonate (esylate), 2-hydroxyethanesulfonate (isethionate), benzenesulfonate (besylate), p-toluenesulfonate (tosylate), 2-naphthalenesulfonate (napsylate), 3-phenylsulfonate, or camphorsulfonate salts; glycerophosphate salts; and acidic amino acid salts such as aspartate or glutamate salts. Preferred pharmaceutically acceptable salts of the compounds of formula (III) include a hydrochloride salt, a hydrobromide salt, a mesylate salt, a sulfate salt, a tartrate salt, a fumarate salt, an acetate salt, a citrate salt, and a phosphate salt. A particularly preferred pharmaceutically acceptable salt of the compound of formula (III) is a hydrochloride salt. Accordingly, it is preferred that the compound of formula (III), including any one of the specific compounds of formula (III) described herein, is in the form of a hydrochloride salt, a hydrobromide salt, a mesylate salt, a sulfate salt, a tartrate salt, a fumarate salt, an acetate salt, a citrate salt, or a phosphate salt, and it is particularly preferred that the compound of formula (III) is in the form of a hydrochloride salt.

The present invention also specifically relates to the compound of formula (III), including any one of the specific compounds of formula (III) described herein, in non-salt form.

The following definitions apply throughout the present description and claims, unless explicitly indicated to the contrary.

The term "hydrogen" is herein used to refer to protium, deuterium and/or tritium, preferably to protium. Accordingly, the term "non-hydrogen atom" refers to any atoms that is not hydrogen, i.e. that is not protium, deuterium or tritium.

The term "hydrocarbon group" refers to a group consisting of carbon atoms and hydrogen atoms.

The term "alicyclic" is used in connection with cyclic groups and denotes that the corresponding cyclic group is non-aromatic.

As used herein, the term "alkyl" refers to a monovalent saturated acyclic (i.e., non-cyclic) hydrocarbon group which may be linear or branched. Accordingly, an "alkyl" group does not comprise any carbon-to-carbon double bond or any carbon-to-carbon triple bond. A "C₁₋₅ alkyl" denotes an alkyl group having 1 to 5 carbon atoms. Preferred exemplary alkyl groups are methyl, ethyl, propyl (e.g., n-propyl or isopropyl), or butyl (e.g., n-butyl, isobutyl, sec-butyl, or tert-butyl). Unless defined otherwise, the term "alkyl" preferably refers to C₁₋₄ alkyl, more preferably to methyl or ethyl, and even more preferably to methyl.

As used herein, the term "alkenyl" refers to a monovalent unsaturated acyclic hydrocarbon group which may be linear or branched and comprises one or more (e.g., one or two) carbon-to-carbon double bonds while it does not comprise any carbon-to-carbon triple bond. The term "C₂₋₅ alkenyl" denotes an alkenyl group having 2 to 5 carbon atoms. Preferred exemplary alkenyl groups are ethenyl, propenyl (e.g., prop-1-en-1-yl, prop-1-en-2-yl, or prop-2-en-1-yl), butenyl, butadienyl (e.g., buta-1,3-dien-1-yl or buta-1,3-dien-2-yl), pentenyl, or pentadienyl (e.g., isoprenyl). Unless defined otherwise, the term "alkenyl" preferably refers to C₂₋₄ alkenyl.

As used herein, the term "alkynyl" refers to a monovalent unsaturated acyclic hydrocarbon group which may be linear or branched and comprises one or more (e.g., one or two) carbon-to-carbon triple bonds and optionally one or more (e.g., one or two) carbon-to-carbon double bonds. The term "C₂₋₅ alkynyl" denotes an alkynyl group having 2 to 5 carbon atoms. Preferred exemplary alkynyl groups are ethynyl, propynyl (e.g., propargyl), or butynyl. Unless defined otherwise, the term "alkynyl" preferably refers to C₂₋₄ alkynyl.

As used herein, the term "alkylene" refers to an alkanediyl group, i.e. a divalent saturated acyclic hydrocarbon group which may be linear or branched. A "C₁₋₅ alkylene" denotes an alkylene group having 1 to 5 carbon atoms, and the term "C₀₋₃ alkylene" indicates that a covalent bond (corresponding to the option "C₀ alkylene") or a C₁₋₃ alkylene is present. Preferred exemplary alkylene groups are methylene (-CH₂-), ethylene (e.g., -CH₂-CH₂- or -CH(-CH₃)-), propylene (e.g., -CH₂-CH₂-CH₂-, -CH(-CH₂-CH₃)-, -CH₂-CH(-CH₃)-, or -CH(-CH₃)-CH₂-), or butylene (e.g., -CH₂-CH₂-CH₂-CH₂-). Unless defined otherwise, the term "alkylene" preferably refers to C₁₋₄ alkylene (including, in particular, linear C₁₋₄ alkylene), more preferably to methylene or ethylene, and even more preferably to methylene.

As used herein, the term "alkenylene" refers to an alkenediyl group, i.e. a divalent unsaturated acyclic hydrocarbon group which may be linear or branched and comprises one or more (e.g., one or two) carbon-to-carbon double bonds while it does not comprise any carbon-to-carbon triple bond. A "C₂₋₅ alkenylene" denotes an alkenylene group having 2 to 5 carbon atoms. Unless defined otherwise, the term "alkenylene" preferably refers to C₂₋₄ alkenylene (including, in particular, linear C₂₋₄ alkenylene).

As used herein, the term "alkynylene" refers to an alkynediyl group, i.e. a divalent unsaturated acyclic hydrocarbon group which may be linear or branched and comprises one or more (e.g., one or two) carbon-to-carbon triple bonds and optionally one or more (e.g., one or two) carbon-to-carbon double bonds. A "C₂₋₅ alkynylene" denotes an alkynylene group having 2 to 5 carbon atoms. Unless defined otherwise, the term "alkynylene" preferably refers to C₂₋₄ alkynylene (including, in particular, linear C₂₋₄ alkynylene).

As used herein, the term "carbocyclyl" refers to a hydrocarbon ring group, including monocyclic rings as well as bridged ring, spiro ring and/or fused ring systems (which may be composed, e.g., of two or three rings), wherein said ring group may be saturated, partially unsaturated (i.e., unsaturated but not aromatic) or aromatic. Unless defined otherwise, "carbocyclyl" preferably refers to aryl, cycloalkyl or cycloalkenyl.

As used herein, the term "heterocyclyl" refers to a ring group, including monocyclic rings as well as bridged ring, spiro ring and/or fused ring systems (which may be composed, e.g., of two or three rings), wherein said ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group may be saturated, partially unsaturated (i.e., unsaturated but not aromatic) or aromatic. For example, each heteroatom-containing ring comprised in said ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. Unless defined otherwise, "heterocyclyl" preferably refers to heteroaryl, heterocycloalkyl or heterocycloalkenyl.

As used herein, the term "aryl" refers to an aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Aryl" may, e.g., refer to phenyl, naphthyl, dialinyl (i.e., 1,2-dihydronaphthyl), tetralinyl (i.e., 1,2,3,4-tetrahydronaphthyl), indanyl, indenyl (e.g., 1H-indenyl), anthracenyl, phenanthrenyl, 9H-fluorenyl, or azulenyl. Unless defined otherwise, an "aryl" preferably has 6 to 14 ring atoms, more preferably 6 to 10 ring atoms, even more preferably refers to phenyl or naphthyl, and most preferably refers to phenyl.

As used herein, the term "arylene" refers to an aryl group, as defined herein above, but having two points of attachment, i.e. a divalent aromatic hydrocarbon ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic). "Arylene" may, e.g., refer to phenylene (e.g., phen-1,2-diyl, phen-1,3-diyl, or phen-1,4-diyl), naphthylene (e.g., naphthalen-1,2-diyl, naphthalen-1,3-diyl, naphthalen-1,4-diyl, naphthalen-1,5-diyl, naphthalen-1,6-diyl, naphthalen-1,7-diyl, naphthalen-2,3-diyl, naphthalen-2,5-diyl, naphthalen-2,6-diyl, naphthalen-2,7-diyl, or naphthalen-2,8-diyl), 1,2-dihydronaphthylene, 1,2,3,4-tetrahydronaphthylene, indanylene, indenylene, anthracenylene, phenanthrenylene, 9H-fluorenylene, or azulenylene. Unless defined otherwise, an "arylene" preferably has 6 to 14 ring atoms, more preferably 6 to 10 ring atoms, even more preferably refers to phenylene or naphthylene, and most preferably refers to phenylene (particularly phen-1,4-diyl).

As used herein, the term "heteroaryl" refers to an aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroaryl" may, e.g., refer to thienyl (i.e., thiophenyl), benzo[b]thienyl, naphtho[2,3-b]thienyl, thianthrenyl, furyl (i.e., furanyl), benzofuranyl, isobenzofuranyl, chromanyl, chromenyl (e.g., 2H-1-benzopyranyl or 4H-1-benzopyranyl), isochromenyl (e.g., 1H-2-benzopyranyl), chromonyl, xanthenyl, phenoxathiinyl, pyrrolyl (e.g., 1H-pyrrolyl), imidazolyl, pyrazolyl, pyridyl (i.e., pyridinyl; e.g., 2-pyridyl, 3-pyridyl, or 4-pyridyl), pyrazinyl, pyrimidinyl, pyridazinyl, indolyl (e.g., 3H-indolyl), isoindolyl, indazolyl, indolizinyl, purinyl, quinolyl, isoquinolyl, phthalazinyl, naphthyridinyl, quinoxalinyl, cinnolinyl, pteridinyl, carbazolyl, β-carbolinyl, phenanthridinyl, acridinyl, perimidinyl, phenanthrolinyl (e.g., [1,10]phenanthrolinyl, [1,7]phenanthrolinyl, or [4,7]phenanthrolinyl), phenazinyl, thiazolyl, isothiazolyl, phenothiazinyl, oxazolyl, isoxazolyl, oxadiazolyl (e.g., 1,2,4-oxadiazolyl, 1,2,5-oxadiazolyl (i.e., furazanyl), or 1,3,4-oxadiazolyl), thiadiazolyl (e.g., 1,2,4-thiadiazolyl, 1,2,5-thiadiazolyl, or 1,3,4-thiadiazolyl), phenoxazinyl, pyrazolo[1,5-a]pyrimidinyl (e.g., pyrazolo[1,5-a]pyrimidin-3-yl), 1,2-benzoisoxazol-3-yl, benzothiazolyl, benzothiadiazolyl, benzoxazolyl, benzisoxazolyl, benzimidazolyl, benzo[b]thiophenyl (i.e., benzothienyl), triazolyl (e.g., 1H-1,2,3-triazolyl, 2H-1,2,3-triazolyl, 1H-1,2,4-triazolyl, or 4H-1,2,4-triazolyl), benzotriazolyl, 1H-tetrazolyl, 2H-tetrazolyl, triazinyl (e.g., 1,2,3-triazinyl, 1,2,4-triazinyl, or 1,3,5-triazinyl), furo[2,3-c]pyridinyl, dihydrofuropyridinyl (e.g., 2,3-dihydrofuro[2,3-c]pyridinyl or 1,3-dihydrofuro[3,4-c]pyridinyl), imidazopyridinyl (e.g., imidazo[1,2-a]pyridinyl or imidazo[3,2-a]pyridinyl), quinazolinyl, thienopyridinyl, tetrahydrothienopyridinyl (e.g., 4,5,6,7-tetrahydrothieno[3,2-c]pyridinyl), dibenzofuranyl, 1,3-benzodioxolyl, benzodioxanyl (e.g., 1,3-benzodioxanyl or 1,4-benzodioxanyl), or coumarinyl. Unless defined otherwise, the term "heteroaryl" preferably refers to a 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroaryl" refers to a 5 or 6 membered monocyclic ring comprising one or more (e.g., one, two or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "heteroarylene" refers to a heteroaryl group, as defined herein above, but having two points of attachment, i.e. a divalent aromatic ring group, including monocyclic aromatic rings as well as bridged ring and/or fused ring systems containing at least one aromatic ring (e.g., ring systems composed of two or three fused rings, wherein at least one of these fused rings is aromatic; or bridged ring systems composed of two or three rings, wherein at least one of these bridged rings is aromatic), wherein said aromatic ring group comprises one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said aromatic ring group may contain one or two O atoms and/or one or two S atoms (which may optionally be oxidized) and/or one, two, three, or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heteroarylene" may, e.g., refer to thienylene (i.e., thiophenylene; e.g., thien-2,3-diyl, thien-2,4-diyl, or thien-2,5-diyl), benzo[b]thienylene, naphtho[2,3-b]thienylene, thianthrenylene, furylene (i.e., furanylene; e.g., furan-2,3-diyl, furan-2,4-diyl, or furan-2,5-diyl), benzofuranylene, isobenzofuranylene, chromanylene, chromenylene, isochromenylene, chromonylene, xanthenylene, phenoxathiinylene, pyrrolylene, imidazolylene, pyrazolylene, pyridylene (i.e., pyridinylene), pyrazinylene, pyrimidinylene, pyridazinylene, indolylene, isoindolylene, indazolylene, indolizinylene, purinylene, quinolylene, isoquinolylene, phthalazinylene, naphthyridinylene, quinoxalinylene, cinnolinylene, pteridinylene, carbazolylene, β-carbolinylene, phenanthridinylene, acridinylene, perimidinylene, phenanthrolinylene, phenazinylene, thiazolylene (e.g., thiazol-2,4-diyl, thiazol-2,5-diyl, or thiazol-4,5-diyl), isothiazolylene (e.g., isothiazol-3,4-diyl, isothiazol-3,5-diyl, or isothiazol-4,5-diyl), phenothiazinylene, oxazolylene (e.g., oxazol-2,4-diyl, oxazol-2,5-diyl, or oxazol-4,5-diyl), isoxazolylene (e.g., isoxazol-3,4-diyl, isoxazol-3,5-diyl, or isoxazol-4,5-diyl), oxadiazolylene (e.g., 1,2,4-oxadiazol-3,5-diyl, 1,2,5-oxadiazol-3,4-diyl, or 1,3,4-oxadiazol-2,5-diyl), thiadiazolylene (e.g., 1,2,4-thiadiazol-3,5-diyl, 1,2,5-thiadiazol-3,4-diyl, or 1,3,4-thiadiazol-2,5-diyl), phenoxazinylene, pyrazolo[1,5-a]pyrimidinylene, 1,2-benzoisoxazolylene, benzothiazolylene, benzothiadiazolylene, benzoxazolylene, benzisoxazolylene, benzimidazolylene, benzo[b]thiophenylene (i.e., benzothienylene), triazolylene (e.g., 1H-1,2,3-triazolylene, 2H-1,2,3-triazolylene, 1H-1,2,4-triazolylene, or 4H-1,2,4-triazolylene), benzotriazolylene, 1H-tetrazolylene, 2H-tetrazolylene, triazinylene (e.g., 1,2,3-triazinylene, 1,2,4-triazinylene, or 1,3,5-triazinylene), furo[2,3-c]pyridinylene, dihydrofuropyridinylene (e.g., 2,3-dihydrofuro[2,3-c]pyridinylene or 1,3-dihydrofuro[3,4-c]pyridinylene), imidazopyridinylene (e.g., imidazo[1,2-a]pyridinylene or imidazo[3,2-a]pyridinylene), quinazolinylene, thienopyridinylene, tetrahydrothienopyridinylene (e.g., 4,5,6,7-tetrahydrothieno[3,2-c]pyridinylene), dibenzofuranylene, 1,3-benzodioxolylene, benzodioxanylene (e.g., 1,3-benzodioxanylene or 1,4-benzodioxanylene), or coumarinylene. Unless defined otherwise, the term "heteroarylene" preferably refers to a divalent 5 to 14 membered (more preferably 5 to 10 membered) monocyclic ring or fused ring system comprising one or more (e.g., one, two, three or four) ring heteroatoms independently selected from O, S and N, wherein one or more S ring atoms (if present) and/or one or more N ring atoms (if present) are optionally oxidized, and wherein one or more carbon ring atoms are optionally oxidized; even more preferably, a "heteroarylene" refers to a divalent 5 or 6 membered monocyclic ring comprising one or more (e.g., one, two or three) ring heteroatoms independently selected from O, S, and N, wherein one or more S ring atoms (if present) and/or one or more N ring atoms (if present) are optionally oxidized, and wherein one or more carbon ring atoms are optionally oxidized. A "heteroarylene", including any of the specific heteroarylene groups described herein, may be attached through two carbon ring atoms, particularly through those two carbon ring atoms that have the greatest distance from one another (in terms of the number of ring atoms separating them by the shortest possible connection) within one single ring or within the entire ring system of the corresponding heteroarylene.

As used herein, the term "cycloalkyl" refers to a saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkyl" may, e.g., refer to cyclopropyl, cyclobutyl, cyclopentyl, cyclohexyl, cycloheptyl, decalinyl (i.e., decahydronaphthyl), or adamantyl. Unless defined otherwise, "cycloalkyl" preferably refers to a C₃₋₁₁ cycloalkyl, and more preferably refers to a C₃₋₇ cycloalkyl. A particularly preferred "cycloalkyl" is a monocyclic saturated hydrocarbon ring having 3 to 7 ring members (e.g., cyclopropyl or cyclohexyl). As used herein, the term "cycloalkylene" refers to a cycloalkyl group, as defined herein above, but having two points of attachment, i.e. a divalent saturated hydrocarbon ring group, including monocyclic rings as well as bridged ring, spiro ring and/or fused ring systems (which may be composed, e.g., of two or three rings; such as, e.g., a fused ring system composed of two or three fused rings). "Cycloalkylene" may, e.g., refer to cyclopropylene (e.g., cyclopropan-1,1-diyl or cyclopropan-1,2-diyl), cyclobutylene (e.g., cyclobutan-1,1-diyl, cyclobutan-1,2-diyl, or cyclobutan-1,3-diyl), cyclopentylene (e.g., cyclopentan-1,1-diyl, cyclopentan-1,2-diyl, or cyclopentan-1,3-diyl), cyclohexylene (e.g., cyclohexan-1,1-diyl, cyclohexan-1,2-diyl, cyclohexan-1,3-diyl, or cyclohexan-1,4-diyl), cycloheptylene, decalinylene (i.e., decahydronaphthylene), or adamantylene. Unless defined otherwise, "cycloalkylene" preferably refers to a C₃₋₁₁ cycloalkylene, and more preferably refers to a C₃₋₇ cycloalkylene. A particularly preferred "cycloalkylene" is a divalent monocyclic saturated hydrocarbon ring having 3 to 7 ring members (e.g., cyclopropylene or cyclohexylene).

As used herein, the term "heterocycloalkyl" refers to a saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkyl" may, e.g., refer to aziridinyl, azetidinyl, pyrrolidinyl, imidazolidinyl, pyrazolidinyl, piperidinyl, piperazinyl, azepanyl, diazepanyl (e.g., 1,4-diazepanyl), oxazolidinyl, isoxazolidinyl, thiazolidinyl, isothiazolidinyl, morpholinyl (e.g., morpholin-4-yl), thiomorpholinyl (e.g., thiomorpholin-4-yl), oxazepanyl, oxiranyl, oxetanyl, tetrahydrofuranyl, 1,3-dioxolanyl, tetrahydropyranyl, 1,4-dioxanyl, oxepanyl, thiiranyl, thietanyl, tetrahydrothiophenyl (i.e., thiolanyl), 1,3-dithiolanyl, thianyl, 1,1-dioxothianyl, thiepanyl, decahydroquinolinyl, decahydroisoquinolinyl, or 2-oxa-5-aza-bicyclo[2.2.1]hept-5-yl. Unless defined otherwise, "heterocycloalkyl" preferably refers to a 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkyl" refers to a 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "heterocycloalkylene" refers to a heterocycloalkyl group, as defined herein above, but having two points of attachment, i.e. a divalent saturated ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, and further wherein one or more carbon ring atoms may optionally be oxidized (i.e., to form an oxo group). For example, each heteroatom-containing ring comprised in said saturated ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkylene" may, e.g., refer to aziridinylene, azetidinylene, pyrrolidinylene, imidazolidinylene, pyrazolidinylene, piperidinylene, piperazinylene, azepanylene, diazepanylene (e.g., 1,4-diazepanylene), oxazolidinylene, isoxazolidinylene, thiazolidinylene, isothiazolidinylene, morpholinylene, thiomorpholinylene, oxazepanylene, oxiranylene, oxetanylene, tetrahydrofuranylene, 1,3-dioxolanylene, tetrahydropyranylene, 1,4-dioxanylene, oxepanylene, thiiranylene, thietanylene, tetrahydrothiophenylene (i.e., thiolanylene), 1,3-dithiolanylene, thianylene, 1,1-dioxothianylene, thiepanylene, decahydroquinolinylene, decahydroisoquinolinylene, or 2-oxa-5-aza-bicyclo[2.2.1]hept-5-ylene. Unless defined otherwise, "heterocycloalkylene" preferably refers to a divalent 3 to 11 membered saturated ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, and wherein one or more carbon ring atoms are optionally oxidized; more preferably, "heterocycloalkylene" refers to a divalent 5 to 7 membered saturated monocyclic ring group containing one or more (e.g., one, two, or three) ring heteroatoms independently selected from O, S and N, wherein one or more S ring atoms (if present) and/or one or more N ring atoms (if present) are optionally oxidized, and wherein one or more carbon ring atoms are optionally oxidized.

As used herein, the term "cycloalkenyl" refers to an unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond. "Cycloalkenyl" may, e.g., refer to cyclopropenyl, cyclobutenyl, cyclopentenyl, cyclohexenyl, cyclohexadienyl, cycloheptenyl, or cycloheptadienyl. Unless defined otherwise, "cycloalkenyl" preferably refers to a C₃₋₁₁ cycloalkenyl, and more preferably refers to a C₃₋₇ cycloalkenyl. A particularly preferred "cycloalkenyl" is a monocyclic unsaturated alicyclic hydrocarbon ring having 3 to 7 ring members and containing one or more (e.g., one or two; preferably one) carbon-to-carbon double bonds.

As used herein, the term "cycloalkenylene" refers to a cycloalkenyl group, as defined hereinabove, but having two points of attachment, i.e. a divalent unsaturated alicyclic (non-aromatic) hydrocarbon ring group, including monocyclic rings as well as bridged ring, spiro ring and/or fused ring systems (which may be composed, e.g., of two or three rings; such as, e.g., a fused ring system composed of two or three fused rings), wherein said hydrocarbon ring group comprises one or more (e.g., one or two) carbon-to-carbon double bonds and does not comprise any carbon-to-carbon triple bond.

As used herein, the term "heterocycloalkenyl" refers to an unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring. "Heterocycloalkenyl" may, e.g., refer to imidazolinyl (e.g., 2-imidazolinyl (i.e., 4,5-dihydro-1H-imidazolyl), 3-imidazolinyl, or 4-imidazolinyl), tetrahydropyridinyl (e.g., 1,2,3,6-tetrahydropyridinyl), dihydropyridinyl (e.g., 1,2-dihydropyridinyl or 2,3-dihydropyridinyl), pyranyl (e.g., 2H-pyranyl or 4H-pyranyl), thiopyranyl (e.g., 2H-thiopyranyl or 4H-thiopyranyl), dihydropyranyl, dihydrofuranyl, dihydropyrazolyl, dihydropyrazinyl, dihydroisoindolyl, octahydroquinolinyl (e.g., 1,2,3,4,4a,5,6,7-octahydroquinolinyl), or octahydroisoquinolinyl (e.g., 1,2,3,4,5,6,7,8-octahydroisoquinolinyl). Unless defined otherwise, "heterocycloalkenyl" preferably refers to a 3 to 11 membered unsaturated alicyclic ring group, which is a monocyclic ring or a fused ring system (e.g., a fused ring system composed of two fused rings), wherein said ring group contains one or more (e.g., one, two, three, or four) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms; more preferably, "heterocycloalkenyl" refers to a 5 to 7 membered monocyclic unsaturated non-aromatic ring group containing one or more (e.g., one, two, or three) ring heteroatoms independently selected from O, S and N, wherein one or more S ring atoms (if present) and/or one or more N ring atoms (if present) are optionally oxidized, wherein one or more carbon ring atoms are optionally oxidized, and wherein said ring group comprises at least one double bond between adjacent ring atoms and does not comprise any triple bond between adjacent ring atoms.

As used herein, the term "heterocycloalkenylene" refers to a heterocycloalkenyl group, as defined hereinabove, as defined hereinabove, but having two points of attachment, i.e. a divalent unsaturated alicyclic (non-aromatic) ring group, including monocyclic rings as well as bridged ring, spiro ring and/or fused ring systems (which may be composed, e.g., of two or three rings; such as, e.g., a fused ring system composed of two or three fused rings), wherein said ring group contains one or more (such as, e.g., one, two, three, or four) ring heteroatoms independently selected from O, S and N, and the remaining ring atoms are carbon atoms, wherein one or more S ring atoms (if present) and/or one or more N ring atoms (if present) may optionally be oxidized, wherein one or more carbon ring atoms may optionally be oxidized (i.e., to form an oxo group), and further wherein said ring group comprises at least one double bond between adjacent ring atoms and does not comprise any triple bond between adjacent ring atoms. For example, each heteroatom-containing ring comprised in said unsaturated alicyclic ring group may contain one or two O atoms and/or one or two S atoms (which may optionally be oxidized) and/or one, two, three or four N atoms (which may optionally be oxidized), provided that the total number of heteroatoms in the corresponding heteroatom-containing ring is 1 to 4 and that there is at least one carbon ring atom (which may optionally be oxidized) in the corresponding heteroatom-containing ring.

As used herein, the term "halogen" refers to fluoro (-F), chloro (-CI), bromo (-Br), or iodo (-I). As it is to be understood for the skilled person, the terms "halogen" and "halo" may be used interchangeably.

As used herein, the term "haloalkyl" refers to an alkyl group substituted with one or more (preferably 1 to 6, more preferably 1 to 3) halogen atoms which are selected independently from fluoro, chloro, bromo and iodo, and are preferably all fluoro atoms. It will be understood that the maximum number of halogen atoms is limited by the number of available attachment sites and, thus, depends on the number of carbon atoms comprised in the alkyl moiety of the haloalkyl group. "Haloalkyl" may, e.g., refer to -CF₃, -CHF₂, -CH₂F, -CF₂-CH₃, -CH₂-CF₃, -CH₂-CHF₂, -CH₂-CF₂-CH₃, -CH₂-CF₂-CF₃, or -CH(CF₃)₂. A particularly preferred "haloalkyl" group is -CF₃.

The terms "bond" and "covalent bond" are used herein synonymously, unless explicitly indicated otherwise or contradicted by context.

As used herein, the terms "optional", "optionally" and "may" denote that the indicated feature may be present but can also be absent. Whenever the term "optional", "optionally" or "may" is used, the present invention specifically relates to both possibilities, i.e., that the corresponding feature is present or, alternatively, that the corresponding feature is absent. For example, the expression "X is optionally substituted with Y" (or "X may be substituted with Y") means that X is either substituted with Y or is unsubstituted. Likewise, if a component of a composition is indicated to be "optional", the invention specifically relates to both possibilities, i.e., that the corresponding component is present (contained in the composition) or that the corresponding component is absent from the composition.

Various groups are referred to as being "optionally substituted" in this specification. Generally, these groups may carry one or more substituents, such as, e.g., one, two, three or four substituents. It will be understood that the maximum number of substituents is limited by the number of attachment sites available on the substituted moiety. Unless defined otherwise, the "optionally substituted" groups referred to in this specification carry preferably not more than two substituents and may, in particular, carry only one substituent. Moreover, unless defined otherwise, it is preferred that the optional substituents are absent, i.e. that the corresponding groups are unsubstituted.

A skilled person will appreciate that the substituent groups comprised in the compounds of the present invention may be attached to the remainder of the respective compound via a number of different positions of the corresponding specific substituent group. Unless defined otherwise, the preferred attachment positions for the various specific substituent groups are as illustrated in the examples.

As used herein, unless explicitly indicated otherwise or contradicted by context, the terms "a", "an" and "the" are used interchangeably with "one or more" and "at least one". Thus, for example, a composition comprising "a" compound of formula (I) can be interpreted as referring to a composition comprising "one or more" compounds of formula (I).

As understood herein, the term "peptide linker" refers preferably to a bivalent moiety obtained from a peptide, as defined herein, through attachment to -CO- moiety of its C-terminal amino acid residue and attachment to -NH- moiety of its N-terminal amino acid residue.

As understood herein, the term "peptide" refers to a polymer of two or more amino acid residues linked via amide bonds that are formed between an amino group of one amino acid residue and a carboxylic acid group of another amino acid residue. The amino acid residues comprised in the peptide or protein, which are also referred to as amino acids, may be selected from the 20 standard proteinogenic a amino acids (i.e., Ala, Arg, Asn, Asp, Cys, Glu, Gln, Gly, His, Ile, Leu, Lys, Met, Phe, Pro, Ser, Thr, Trp, Tyr, and Val) but also from non-proteinogenic and/or non-standard a amino acids (such as, e.g., ornithine, citrulline, homolysine, pyrrolysine, 4 hydroxyproline, a methylalanine (i.e., 2 aminoisobutyric acid), norvaline, norleucine, terleucine (tert-leucine), labionin, 2,4-diamino-3-hydroxybutyric acid (2,4-diamino-3-hydroxybutyrate), 2,4-diaminobutyrate, 5-hydroxylysine, 3-hydroxyarginine, 2,3-didehydroarginine or an alanine or glycine that is substituted at the side chain with a cyclic group such as, e.g., cyclopentylalanine, cyclohexylalanine, phenylalanine, naphthylalanine, pyridylalanine, thienylalanine, cyclohexylglycine, or phenylglycine) as well as β amino acids (e.g., β alanine), γ-amino acids (e.g., γ-aminobutyric acid, isoglutamine, or statine) and δ-amino acids. Preferably, the amino acid residues comprised in the peptide or protein are selected from a amino acids, more preferably from the 20 standard proteinogenic a amino acids (which can be present as the L isomer or the D-isomer, and are preferably all present as the L isomer). The peptide may be unmodified or may be modified, e.g., at its N terminus, at its C terminus and/or at a functional group in the side chain of any of its amino acid residues (particularly at the side chain functional group of one or more Lys, His, Ser, Thr, Tyr, Cys, Asp, Glu, and/or Arg residues). Such modifications may include, e.g., the attachment of any of the protecting groups described for the corresponding functional groups in: Wuts PG & Greene TW, Greene's protective groups in organic synthesis, John Wiley & Sons, 2006. Such modifications may also include the covalent attachment of one or more polyethylene glycol (PEG) chains (forming a PEGylated peptide), the glycosylation and/or the acylation with one or more fatty acids (e.g., one or more C8-30 alkanoic or alkenoic acids; forming a fatty acid acylated peptide or protein). Moreover, such modified peptides or proteins may also include peptidomimetics, provided that they contain at least two amino acids that are linked via an amide bond (formed between an amino group of one amino acid and a carboxyl group of another amino acid). The amino acid residues comprised in the peptide or protein may, e.g., be present as a linear molecular chain (forming a linear peptide) or may form one or more rings (corresponding to a cyclic peptide). Unless indicated to the contrary, the peptides as referred to herein are linear peptides of less than 40 residues, comprising amino acid residues selected from the 20 standard proteinogenic a amino acids, each present as L isomer. Preferably, side chains of amino acid residues are not modified. Preferably, N-terminus of the peptide is either not modified or is acetylated, and/or C-terminus of the peptide is either not modified or is amidated. Acetylation is preferably understood herein as a modification of the amino group, preferably a main chain amino group NH₂-, into CH₃CONH- moiety. Amidation is preferably understood herein as a modification of the carboxylic group, preferably a main chain carboxylic group -COOH, into a -CONH₂ moiety.

As used herein, the term "amino acid" refers, in particular, to any one of the 20 standard proteinogenic α-amino acids (i.e., Ala, Arg, Asn, Asp, Cys, Glu, Gln, Gly, His, Ile, Leu, Lys, Met, Phe, Pro, Ser, Thr, Trp, Tyr, and Val) but also to non-proteinogenic and/or non-standard α-amino acids (such as, e.g., ornithine, citrulline, homolysine, pyrrolysine, 4-hydroxyproline, α-methylalanine (i.e., 2-aminoisobutyric acid), norvaline, norleucine, terleucine (tert-leucine), labionin, or an alanine or glycine that is substituted at the side chain with a cyclic group such as, e.g., cyclopentylalanine, cyclohexylalanine, phenylalanine, naphthylalanine, pyridylalanine, thienylalanine, cyclohexylglycine, or phenylglycine) as well as β-amino acids (e.g., β-alanine), γ-amino acids (e.g., γ-aminobutyric acid, isoglutamine, or statine) and/or δ-amino acids as well as any other compound comprising at least one carboxylic acid group and at least one amino group. Unless defined otherwise, an "amino acid" preferably refers to an α-amino acid, more preferably to any one of the 20 standard proteinogenic α-amino acids (which can be present as the L-isomer or the D-isomer, and are preferably present as the L-isomer).

The terms "peptide", "polypeptide" and "protein" may be used interchangeably. Moreover, unless explicitly indicated otherwise or contradicted by context, any reference to a sequence (or partial sequence) from a defined amino acid sequence (e.g., defined by its SEQ ID) is to be understood as relating to a continuous sequence (or a continuous partial sequence) from the corresponding defined sequence. Thus, for example, any reference to a partial sequence of X to Y amino acid residues from a larger amino acid sequence defined by its SEQ ID is to be understood as relating to a continuous (uninterrupted) sequence of X to Y amino acid residues from the defined larger amino acid sequence.

The term "% sequence identity", as used herein, e.g., in connection with amino acid sequences of proteins/peptides and/or in connection with nucleotide sequences of nucleic acid molecules, describes the number of matches of identical amino acid residues (or nucleotides) of two or more aligned sequences as compared to the number of amino acid residues (or nucleotides) making up the overall length of the compared sequences (or the overall compared portions thereof). Using an alignment of two or more sequences or subsequences, the percentage of amino acid residues (or nucleotides) that are the same may be determined when the (sub)sequences are compared and aligned for maximum correspondence over a window of comparison, or over a designated region as measured using a sequence comparison algorithm as known in the art, or when manually aligned and visually inspected. Examples of algorithms that can be used for determining sequence identity include, in particular, those based on the NCBI BLAST algorithm (Altschul SF et al., Nucleic Acids Res, 1997, 25(17): 3389-402, doi: 10.1093/nar/25.17.3389), CLUSTALW (Thompson JD et al., Nucleic Acids Res, 1994, 22(22): 4673-80, doi: 10.1093/nar/22.22.4673), or FASTA (Pearson WR et al., Proc Natl Acad Sci USA, 1988, 85(8): 2444-8, doi: 10.1073/pnas.85.8.2444). Although the FASTA algorithm typically does not consider internal non-matching deletions or additions in sequences, i.e. gaps, in its calculation, this can be corrected manually to avoid an overestimation of the % sequence identity. CLUSTALW, however, does take sequence gaps into account in its identity calculations.

Furthermore, it is to be understood that wherever a numerical range is provided/described herein, all values and subranges encompassed by the respective numerical range are specifically provided by the invention. Accordingly, the present invention specifically and individually relates to each value that falls within a numerical range described herein, as well as each and any subrange encompassed by a numerical range described herein.

As used herein, the term "about" preferably refers to ±10% of the indicated numerical value, more preferably to ±5% of the indicated numerical value, and in particular to the exact numerical value indicated. If the term "about" is used in connection with the endpoints of a range, it preferably refers to the range from the lower endpoint -10% of its indicated numerical value to the upper endpoint +10% of its indicated numerical value, more preferably to the range from of the lower endpoint -5% to the upper endpoint +5%, and even more preferably to the range defined by the exact numerical values of the lower endpoint and the upper endpoint. If the term "about" is used in connection with the endpoint of an open-ended range, it preferably refers to the corresponding range starting from the lower endpoint -10% or from the upper endpoint +10%, more preferably to the range starting from the lower endpoint -5% or from the upper endpoint +5%, and even more preferably to the open-ended range defined by the exact numerical value of the corresponding endpoint. If the term "about" is used in connection with a parameter that is quantified in integers, such as the number of nucleotides in a given nucleic acid or the number of amino acid residues in a given peptide or protein, the numbers corresponding to ±10% or ±5% of the indicated numerical value are to be rounded to the nearest integer (using the tie-breaking rule "round half up"). Thus, for example, the expression "about 25 amino acid residues" preferably refers to the range of 23 to 28 amino acid residues, more preferably to the range of 24 to 26 amino acid residues, and even more preferably refers to the specific value of 25 amino acid residues.

As used herein, the term "comprising" (or "comprise", "comprises", "contain", "contains", or "containing"), unless explicitly indicated otherwise or contradicted by context, has the meaning of "containing, inter alia", i.e., "containing, among further optional elements, ...". In addition, this term also includes the narrower meanings of "consisting essentially of" and "consisting of". For example, the term "A comprising B and C" has the meaning of "A containing, inter alia, B and C", wherein A may contain further optional elements (e.g., "A containing B, C and D" would also be encompassed), but this term also includes (i.e., also provides a specific disclosure of) the meaning of "A consisting essentially of B and C" and the meaning of "A consisting of B and C" (i.e., no other components than B and C are comprised in A).

As used herein, the term "having" (or "has" or "have"), particularly when used in connection with an amino acid sequence or a nucleotide sequence, means "comprising". In addition, the term "having" also includes the preferred meaning "consisting of". Accordingly, an expression such as, e.g., "a VH domain having the sequence..." indicates that the VH domain "comprises" the indicated sequence, and that it preferably "consists of" the indicated sequence.

Unless specifically indicated otherwise or contradicted by context, all properties and parameters (including functional parameters) referred to herein are preferably to be determined (and, likewise, all assays and tests referred to herein are preferably to be conducted) at standard conditions, particularly at standard ambient temperature and pressure conditions, e.g., at a temperature of 25°C (298.15 K) and at an absolute pressure of 101.325 kPa (1 atm). Assays determining a biological activity (e.g., an enzymatic activity) can also be conducted at a temperature of 37°C.

Moreover, unless indicated otherwise, any reference to a database, an industry standard, a pharmacopeia, or a manufacturer's manual refers to the corresponding latest version that was available either at the filing date or at the earliest priority date (which may also be referred to as the earliest filing date) of the present specification, preferably the latest version that was available at the earliest priority date of the present specification.

All amino acid sequences referred to herein are also described in a concurrently filed sequence listing, which is incorporated herein by reference in its entirety. In the case of conflict between any sequence specified in the present disclosure and the corresponding sequence specified in the accompanying sequence listing, the present invention specifically and individually relates to each one of the respective sequences.

It is to be understood that the present invention specifically and individually relates to each and every combination of features and embodiments described herein, including any combination of general and/or preferred features/embodiments. Thus, in accordance with the present invention, each such combination is specifically envisaged herein.

In a further embodiment, the present invention relates to a pharmaceutical composition comprising the immunoconjugate of the present invention, and a pharmaceutically acceptable carrier

Pharmaceutical formulations of an immunoconjugate of the present invention as described herein are prepared by mixing such immunoconjugate having the desired degree of purity with one or more optional pharmaceutically acceptable carriers *(*Remington's Pharmaceutical Sciences 16th edition, Osol, A. Ed. (1980)), in the form of lyophilized formulations or aqueous solutions. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA or GLDA; sugars such as sucrose, mannitol, trehalose or sorbitol; osmo-protectants like ectoin; salt-forming counter-ions such as sodium; metal complexes (e.g. Zn protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one aspect, a sHASEGP is combined with one or more additional glycosaminoglycanases such as chondroitinases.

The term "pharmaceutical formulation" or "pharmaceutical composition" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject. A pharmaceutically acceptable carrier includes, but is not limited to, a buffer, excipient, stabilizer, or preservative.

Exemplary lyophilized immunoconjugate formulations are described in US Patent No. 6,267,958. Aqueous immunoconjugate formulations include those described in US Patent No. 6,171,586 and WO 2006/044908, the latter formulations including a histidine-acetate buffer.

The formulation herein may also contain more than one active ingredient as necessary for the particular indication being treated, preferably those with complementary activities that do not adversely affect each other.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the antibody or immunoconjugate, which matrices are in the form of shaped articles, e.g. films, or microcapsules.

The formulations to be used for *in vivo* administration are generally sterile. Sterility may be readily accomplished, e.g., by filtration through sterile filtration membranes.

In one embodiment, the present invention relates to the immunoconjugate of the present invention or pharmaceutical composition of the present invention for use as a medicament. In other words, the present invention relates to the immunoconjugate of the present invention or pharmaceutical composition of the present invention for use in therapy. It is to be understood that the immunoconjugate or the pharmaceutical compositions of the present invention can be used in the treatment of a disease or a disorder.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, immunoconjugates of the invention are used to delay development of a disease or to slow the progression of a disease.

An immunoconjugate of the invention (and any additional therapeutic agent) can be administered by any suitable means, including parenteral, intrapulmonary, and intranasal, and, if desired for local treatment, intralesional, intrauterine or intravesical administration. Parenteral infusions include intramuscular, intravenous, intraarterial, intraperitoneal, or subcutaneous administration. Dosing can be by any suitable route, e.g. by injections, such as intravenous or subcutaneous injections, depending in part on whether the administration is brief or chronic. Various dosing schedules including but not limited to single or multiple administrations over various time-points, bolus administration, and pulse infusion are contemplated herein.

Immunoconjugates of the invention would be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The immunoconjugate need not be, but is optionally formulated with one or more agents currently used to prevent or treat the disorder in question. The effective amount of such other agents depends on the amount of immunoconjugate present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of an immunoconjugate of the invention (when used alone or in combination with one or more other additional therapeutic agents) will depend on the type of disease to be treated, the type of immunoconjugate, the severity and course of the disease, whether the immunoconjugate is administered for preventive or therapeutic purposes, previous therapy, the patient's clinical history and response to the immunoconjugate, and the discretion of the attending physician. The immunoconjugate is suitably administered to the patient at one time or over a series of treatments. Depending on the type and severity of the disease, about 1 µg/kg to 15 mg/kg (e.g. 0.1 mg/kg-10 mg/kg) of immunoconjugate can be an initial candidate dosage for administration to the patient, whether, for example, by one or more separate administrations, or by continuous infusion. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. One exemplary dosage of the immunoconjugate would be in the range from about 0.05 mg/kg to about 10 mg/kg. Thus, one or more doses of about 0.5 mg/kg, 2.0 mg/kg, 4.0 mg/kg or 10 mg/kg (or any combination thereof) may be administered to the patient. Such doses may be administered intermittently, e.g. every week or every three weeks (e.g. such that the patient receives from about two to about twenty, or e.g. about six doses of the antibody). An initial higher loading dose, followed by one or more lower doses may be administered. However, other dosage regimens may be useful. The progress of this therapy is easily monitored by conventional techniques and assays.

The immunoconjugates of the present invention are particularly useful in the treatment or prevention of cancer. Accordingly, the present invention provides the immunoconjugate of the present invention for use in the treatment or prevention of cancer. Likewise, in one embodiment, the present invention relates to use of the immunoconjugate of the present invention in the manufacture of a medicament for the treatment of prevention of cancer. In a further embodiment, the present invention relates to a method of treating cancer, the method comprising a step of administering the immunoconjugate of the present invention to a subject in need thereof. It is to be understood that preferably a therapeutically effective amount of said immunoconjugate is to be administered.

Preferably, the cancer is characterized by upregulation of RBM39. Exemplary cancers characterized by upregulation of RBM39 are as disclosed herein. For example such cancers include cancer selected from neuroblastoma, Ewing sarcoma, osteosarcoma, AML, rhabdomyosarcoma, melanoma, breast cancer, triple negative breast cancer, lung cancer, colorectal cancer, ovarian cancer, multiple myeloma, hepatocellular carcinoma, pancreatic cancer, brain cancer, medulloblastoma, and ovarian cancer. Preferably, cancer is selected from neuroblastoma, Ewing sarcoma, osteosarcoma, rhabdomyosarcoma, melanoma, acute myeloid leukemia, breast cancer, lung cancer, ovarian cancer and colorectal cancer.

It is particularly preferred, within the scope of the present invention, that the cancer to be treated is neuroblastoma. More preferably, the neuroblastoma is a pediatric neuroblastoma. As further disclosed herein, said neuroblastoma, preferably said pediatric neuroblastoma, is characterized by presence of GD2 displayed on cancer cell surface.

Another potential strategy for improving the efficacy of the immunoconjugates of the present invention, in particular GD2-targeted therapy that should be considered is the combination with EZH2 or HDAC inhibitors. It has been shown that treatment with EZH2 or HDAC inhibitors may induce or upregulate the expression of GD2 disialoganglioside TAA in neuroblastoma (and other types of cancer) leading to enhanced outcomes of the anti-GD2 antibody-based immunotherapy. Examples of inhibitors that can be used for these purposes include GSK126 or tazemetostat as EZH2i and vorinostat, entinostat or panabinostat as HDACi.

Accordingly, in one embodiment of the present invention, the immunoconjugate or the pharmaceutical composition is to be co-administered with an EZH2 inhibitor (such as GSK126 or tazemetostat) or with an HDAC inhibitor (such as vorinostat, entinostat or panabinostat).

The efficacy of the immunoconjugates of the present invention, in particular of the GD2-targeted immunoconjugates of the invention, can be further accomplished by their co-administration with DNA damage response inhibitors, e.g., ATM/ATRi (gartisertib, berzosertib), DNA-PKi (AZD-7648, nedisertib), PARPi (olaparib), immune checkpoint inhibitors, e.g., anti-PD-1 (nivolumab), anti-CD47 (magrolimab) or combined anti-TIGIT & anti-PD-L1 therapy (tiragolumab, atezolizumab).

Further embodiments of the invention are illustrated in the following numbered items:
1. An immunoconjugate comprising
   a) an antibody binding to a tumor antigen displayed on the surface of a cancer cell; and
   b) a compound according to formula (I): 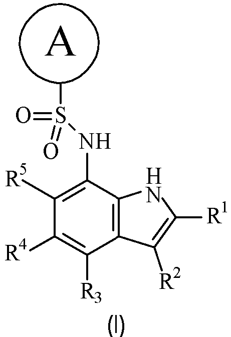
   wherein
   R¹ is selected from -H, C₁₋₅ alkyl, -OH, -CN and -NO₂;
   R² is selected from -H, halogen, C₁₋₅ alkyl, C₁₋₅ haloalkyl, -OH, -CN, -NO₂, -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
   R³ is selected from -H, halogen, C₁₋₅ alkyl, aryl, -OH, -O(C₁₋₅ alkyl), -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
   R⁴ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
   R⁵ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl); and
   A is a phenyl or five- or six-membered heteroaryl, optionally substituted with one or more groups selected from R^{S}; and
   each R^{S} is independently selected from halogen, C₁₋₅ alkyl, C₁₋₅ haloalkyl, -OH, -O(C₁₋₅ alkyl), -(C₁₋₅ alkylene)-OH, -CN, -NH₂, -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -O-(C₁₋₅ alkylene)-NH₂, -O-(C₂₋₅ alkylene)-NH(C₁₋₅ alkyl), -O-(C₂₋₅ alkylene)-N(C₁₋₅ alkyl)(C₁₋₅ alkyl), -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and - SO₂N(C₁₋₅ alkyl)(C₁₋₅ alkyl).
2. The immunoconjugate of item 1, wherein the antibody is a monoclonal antibody, a chimeric antibody, a recombinant antibody, an antigen-binding fragment of a recombinant antibody, a single chain antibody, a humanized antibody, a bispecific antibody, a multi-specific antibody, a single-domain antibody, or an antibody construct.
3. The immunoconjugate of item 1 or 2, wherein the cancer cell is selected from a neuroblastoma cell, an Ewing sarcoma cell, an osteosarcoma cell, a leukemia cell (such as AML cell), a rhabdomyosarcoma cell, a melanoma cell, a breast cancer cell, a triple negative breast cancer cell, a lung cancer cell, a colorectal cancer cell (such as colorectal adenoma cell), an ovarian cancer cell, a multiple myeloma cell, a hepatocellular carcinoma cell, pancreatic cancer cell, brain cancer cell (such as glioma cell), a medulloblastoma cell, prostate cancer cell, bladder cancer cell, urethral cancer cell, cervical cancer cell, a soft tissue sarcoma cell and a lymphoma cell.
4. The immunoconjugate of item 3, wherein the cancer cell is selected from a neuroblastoma cell, an Ewing sarcoma cell, an osteosarcoma cell, a rhabdomyosarcoma cell, a melanoma cell and a triple negative breast cancer cell.
5. The immunoconjugate of item 1 or 2, wherein the tumor antigen is selected from GD2, *O*-acetyl-GD2 (such as 7-*O*-acetyl-GD2 or 9-*O*-acetyl-GD2), B7-H3, GPC2, ALK, HER2, HER3, Trop-2, CD33, CLL-1, c-KIT, CD123, EGFR, FR-α, MUC16 and STEAP1.
6. The immunoconjugate of item 5, wherein the tumor antigen is selected from GD2, O-acetyl-GD2, B7-H3, GPC2, and ALK.
7. The immunoconjugate of item 5 or 6, wherein the tumor antigen is GD2.
8. The immunoconjugate of item 7, wherein (a) is dinutuximab, dinutuximab beta or naxitamab.
9. The immunoconjugate of item 7, wherein the antibody further binds to B7-H3 or to CD3.
10. The immunoconjugate of any one of items 1 to 9, wherein the antibody is characterized by a point mutation K322A and/or wherein the antibody is a deglycosylated antibody.
11. The immunoconjugate of any one of items 1 to 10, wherein the immunoconjugate comprises a moiety according to formula (II):

   -L₁-L_{P}-L₂-X- (II)

   wherein
   L₁ is C₂₋₂₀ alkylene, optionally substituted with one or more substituents selected from -CN, halogen, -OH, and -NH₂, and wherein one or more -CH₂- groups may independently be replaced by a group selected from -CH=CH-, -C=C-, -O-, -NR'-, -CO-, C₆₋₁₀ arylene, 5-6 membered heteroarylene, - (CH₂-O-CH₂)-, -(CH₂-CH₂-O)-, -CO-O-, -CO-NR'-, C₃-C₁₁ cycloalkylene, or 4-11 membered heterocycloalkylene, wherein R' is H or C₁-C₆ alkyl;
   L_{P} is a bond or is a peptide linker which includes up to 5 amino acid residues, wherein the C-terminus of the peptide linker is connected to -NH- moiety of L₂ or to X,
   L₂ is -NH-1,4-phenylene-CH₂- or is a bond, and
   X is selected from a bond, -O-, -O-CO-, -CO-O-, and -NH-,
   wherein X is attached to the compound of formula (I).
12. The immunoconjugate of item 11, wherein L₁ is C₂₋₁₂ alkylene, optionally substituted with one or more substituents selected from -CN, halogen, -OH, and -NH₂, and wherein one or more -CH₂-groups may independently be replaced by a group selected from -O-, -NR'-, -CO-, -(CH₂-O-CH₂)-, -(CH₂-CH₂-O)-, C₃-C₁₁ cycloalkylene, and 4-11 membered heterocycloalkylene, wherein R' is H or C₁-C₆ alkyl.
13. The immunoconjugate of item 11 or 12, wherein L₁ is selected from: 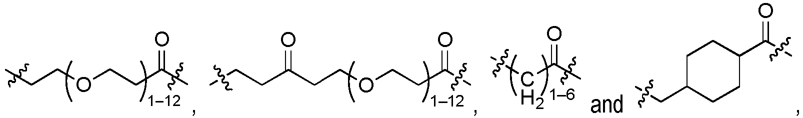 wherein the right empty valence of L₁ is attached to L_{P}.
14. The immunoconjugate of item 13, wherein the L₁ is: 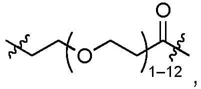
   preferably wherein the L₁ is 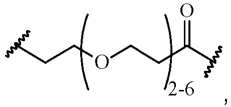
   more preferably wherein the L₁ is 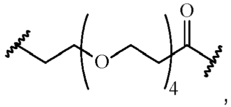
   wherein the right empty valence of L₁ is attached to L_{P}.
15. The immunoconjugate of any one of items 11 to 14, wherein L_{P} is selected from -Val-Cit-, -cBu-Cit-, -Val-Ala-, -Glu-Val-Cit-, -Gly-Gly-Gly-, -Ala-Ala-Asn- and -Gly-Gly-Phe-Gly-, wherein the C-terminus of the peptide linker is connected to -NH- moiety of L₂ or to X, and wherein the N-terminus is connected to L₁.
16. The immunoconjugate of item 15, wherein L_{P} is -Gly-Gly-Phe-Gly-, wherein the C-terminus of the peptide linker is connected to -NH- moiety of L₂ or to X, and wherein the N-terminus is connected to L₁.
17. The immunoconjugate of any one of items 11 to 14, wherein the peptide linker is -Val-Cit-, wherein the C-terminus of the peptide linker is connected to -NH- moiety of L₂ or to X, and wherein the N-terminus is connected to L₁.
18. The immunoconjugate of any one of items 11 to 17, wherein X is -O-CO-, wherein the right empty valence of said -O-CO- is attached to the compound of formula (I).
19. The immunoconjugate of any one of items 11 to 17, wherein X is a bond.
20. The immunoconjugate of any one of items 11 to 19, wherein the moiety of formula (II) is comprised in a moiety of formula (Ila):

   -Z-L₁-L_{P}-L₂-X- (IIa)

   wherein Z is 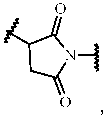 attached through its right empty valence to L₁, and through its left empty valence to a cysteine residue of the antibody, and wherein L₁, L_{P}, L₂, and X are as defined in any one of items 11 to 19.
21. The immunoconjugate of any one of items 1 to 20, wherein R¹ is -H or -OH, preferably wherein R¹ is -H.
22. The immunoconjugate of any one of items 1 to 21, wherein R² is selected from -H, halogen, -OH, - CN, -CONH₂, -CONH(C₁₋₅ alkyl), and -CON(C₁₋₅ alkyl)(C₁₋₅ alkyl), preferably wherein R² is -H, halogen or -CN, more preferably wherein R² is -CN.
23. The immunoconjugate of any one of items 1 to 22, wherein R³ is selected from -H, halogen, and C₁₋₅ alkyl, preferably wherein R³ is selected from -H and C₁₋₅ alkyl (such as methyl).
24. The immunoconjugate of any one of items 1 to 23, wherein R⁴ is selected from -H and halogen, preferably wherein R⁴ is -H.
25. The immunoconjugate of any one of items 1 to 24, wherein R⁵ is selected from -H and halogen, preferably wherein R⁵ is -H.
26. The immunoconjugate of any one of items 1 to 25, wherein A is phenyl optionally substituted with one or more groups selected from -H, halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl), preferably a phenyl substituted with methyl or a phenyl substituted with -CN.
27. The immunoconjugate of any one of items 1 to 20, wherein the compound of formula (I) is selected from: 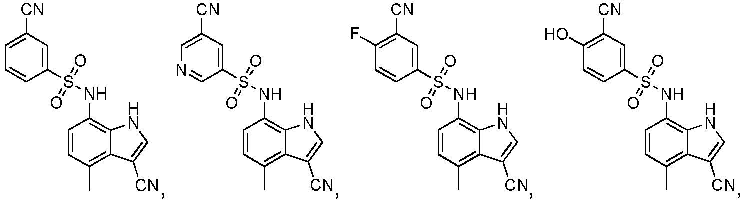 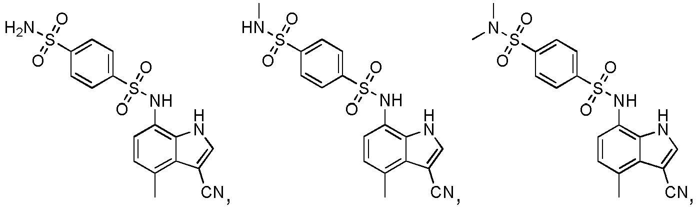 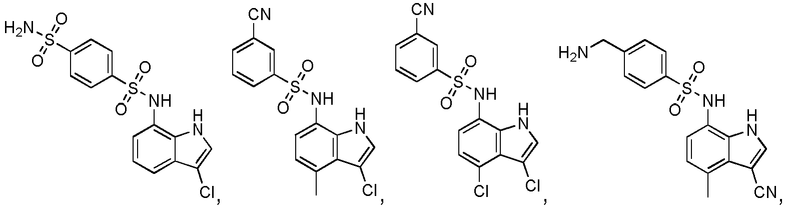 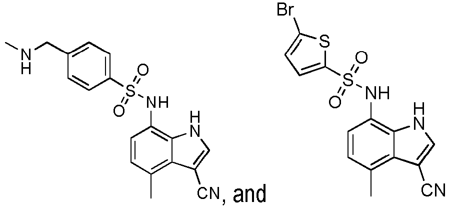
28. The immunoconjugate of any one of items 1 to 27, wherein the compound of formula (I) is present in the immunoconjugate as a moiety of formula (Ia): 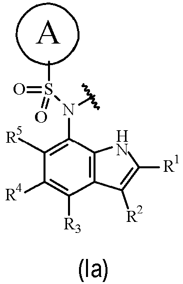 wherein R¹, R², R³, R⁴, R⁵ and A are as defined in any one of items 1 to 27, preferably wherein X is a bond.
29. The immunoconjugate of item 28, wherein the moiety of formula (Ia) is a moiety of formula (la-1): 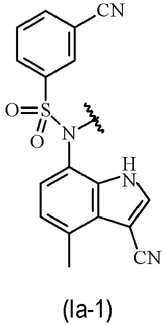
30. The immunoconjugate of any one of items 1 to 27, wherein the compound of formula (I) is present in the immunoconjugate as a moiety of formula (lb): 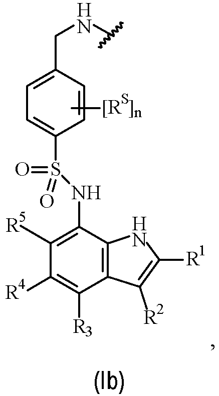 preferably 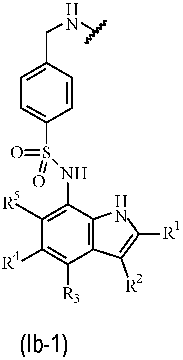 wherein R¹, R², R³, R⁴, and R⁵ are as defined in any one of items 1 to 27, preferably wherein X is -O-CO-, wherein the right empty valence of said -O-CO- is connected to the moiety of formula (Ib), wherein R^{S} is selected from halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl), and wherein n is an integer from 0 to 4.
31. The immunoconjugate of item 30, wherein the moiety of formula (lb) is a moiety of formula (lb-2): 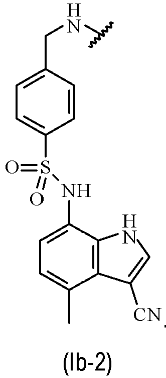
32. The immunoconjugate of any one of items 1 to 31, characterized by DAR in the range from 1 to 8.
33. A pharmaceutical composition comprising the immunoconjugate of any one of items 1 to 32, and a pharmaceutically acceptable carrier.
34. The immunoconjugate of any one of items 1 to 32 or a pharmaceutical composition of item 33 for use as a medicament.
35. The immunoconjugate of any one of items 1 to 32 or a pharmaceutical composition of item 33 for use in the treatment or prevention of cancer.
36. The immunoconjugate for use or the pharmaceutical composition for use of item 35, wherein the cancer is characterized by upregulation of RBM39.
37. The immunoconjugate for use or the pharmaceutical composition for use of item 35 or 36, wherein the cancer is selected from neuroblastoma, Ewing sarcoma, osteosarcoma, rhabdomyosarcoma, melanoma, acute myeloid leukemia, breast cancer, lung cancer, ovarian cancer and colorectal cancer.
38. The immunoconjugate for use or the pharmaceutical composition for use of item 35, wherein the cancer is neuroblastoma, preferably a pediatric neuroblastoma.
39. The immunoconjugate for use or the pharmaceutical composition for use of any one of items 34 to 37, wherein the immunoconjugate or the pharmaceutical composition is to be co-administered with an EZH2 inhibitor (such as GSK126 or tazemetostat) or with an HDAC inhibitor (such as vorinostat, entinostat or panabinostat).
40. A compound of formula:

   Z₁-L₁-L_{P}-L₂-X-Y (III)

   or a pharmaceutically acceptable salt thereof, wherein
   Zz is Z₁ or Z₂,
   Z₁ is selected from 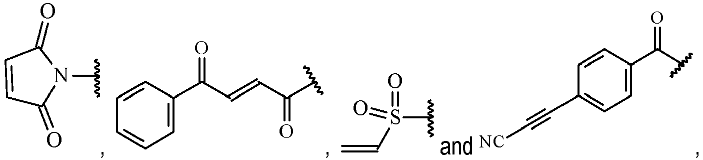 or Z₁ is a group selected from 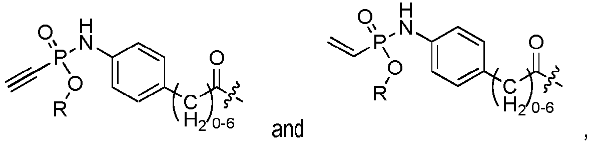
   wherein R is -(CH₂CH₂O)₁₋₂₃-CH₂CH₂OH or -(CH₂CH₂O)₁₋₂₃-CH₂CF₃;
   Z₂ is selected from 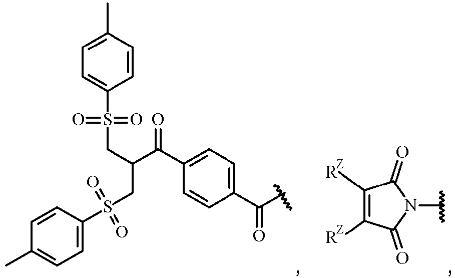 wherein each R^{Z} independently is Hal (such as CI, Br or I) or -S-aryl (such as -S-phenyl), and 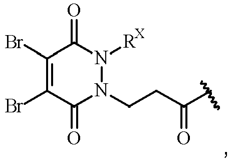 wherein R^{X} is C₁₋₅ alkyl (such as methyl or ethyl), C₂₋₅ alkenyl or C₂₋₅ alkynyl (such as propargyl);
   L₁, L_{P}, L₂, and X are as defined in any one of items 11 to 19; and
   Y is the moiety of formula (Ia) as defined in claim 28 or 29 or the moiety of formula (Ib) as defined in item 30 or 31.

The invention is illustrated by the following examples. These are not meant to limit the scope of protection in any way, which is defined by the hereto appended claims, but serve merely illustrative purposes.

### Examples

MYCN-amplified high-risk neuroblastoma was selected as a proof-of-concept model disease with upregulation of RBM39 and sensitivity towards the pharmacological depletion of the RBM39 oncoprotein using molecular glue degraders. Moreover, clinically approved antibody-based immunotherapy targeting GD2 disialoganglioside is available in the form of anti-GD2 antibody dinutuximab beta.⁹

Results presented in Figure 3 show that the cellular internalization rates of the dinutuximab beta (ch14.18/CHO, tradename Qarziba) antibody to some extent correlate with the surface present GD2 and support the use of the dinutuximab beta as a delivery vehicle for small molecule drugs and payloads selectively to GD2+ neuroblastoma and potentially other cancer cells. Viable neuroblastoma cells were selected using a FACS gating strategy comprising of FSC-A/SSC-A selection of neuroblastoma cells, FSC-A/FSC-H exclusion of doublets and finally selection of FSC-A/PI- viable neuroblastoma cells. For the antibody internalization, median fluorescence intensity of Cy5 was determined for every sample and plotted as fold change relative to the vehicle-treated control. For the surface GD2 expression, geometric mean fluorescence intensity was determined for every sample, BD Quantibrite^{™} Phycoerythrin (PE) Fluorescence Quantitation Kit (BD Biosciences 340495) were analyzed according to the manufacturer's instructions with every run. This was used to generate a standard curve from which the PE geometric mean for each sample was converted into the number of antibodies bound per cell (ABC) representing surface antigen expression.

Results of the screen presented in Figure 4, together with the literature evidence for the DCAF-15-mediated RBM39 dependency in *MYCN*-amplified high-risk neuroblastoma lead to the selection of E7820-based RBM39 molecular glues as payloads for the anti-GD2 DACs. Other hits and potential DAC degrader payloads are highlighted in the following Table 3, below.

**Table 3. In vitro biological activity (EC₅₀ [nM]) of the RBM39 molecular glue degraders obtained from the degrader payload screen in neuroblastoma cell lins.**

| | **E7820** | indisulam | dCeMM1 | tasisulam |
|---|---|---|---|---|
| | 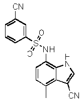 | 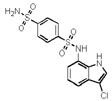 | 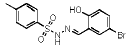 | 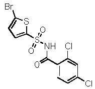 |
| IMR-32 | 57.9 | 89.0 | >1000 | >1000 |
| STA-NB-8 | 25.4 | 47.2 | 451.0 | 896.9 |
| CLB-Ma | 38.4 | 69.4 | 731.4 | >1000 |
| CHLA-90 | >1000 | >1000 | >1000 | >1000 |
| SK-N-MM | 650.9 | 900.5 | >1000 | >1000 |
| STA-NB-1.2 | 26.1 | 60.4 | 484.7 | >1000 |
| STA-NB-6 | 147.9 | 366.6 | >1000 | >1000 |
| STA-NB-10 | 101.5 | 274.2 | >1000 | >1000 |
| SK-N-SH | 101.3 | 238.7 | >1000 | >1000 |
| KELLY | 285.9 | 602.3 | >1000 | >1000 |

Accordingly, the first generation anti-GD2-RBM39 immunoconjugates were prepared based on maleimide-containing chemical modified, as outlined in Figure 5. An alternative strategy for labeling the Cys residues is shown in Figure 6.

*In vitro* anti-cancer activity of the first generation anti-GD2/RBM39 DACs in (a) IMR-32 (GD2+) and (b) SK-N-SH (GD2) neuroblastoma cell line evaluated by CellTiter-Blue^{®} cell viability assay after 72 h treatment is shown in Figure 7.

In a GD2+ neuroblastoma cell line IMR-32, a remarkable and unexpected increase in the potency of the anti-GD2/RBM39 DAC compared to the small molecule RBM39 degrader or dinutuximab beta antibody alone was observed (Figure 7a). The results showed that once linked to the anti-GD2 dinutuximab beta antibody, anti-cancer activity of the RBM39 molecular glue degrader was significantly enhanced depending on the average DAR of the specific anti-GD2 DAC. Around 15-fold higher neuroblastoma cell killing activity was observed when IMR-32 cells were treated with DAC1 DAR ~4 whilst DAC2 with an average DAR ~6 showed even higher, -30-fold increase in the anti-cancer potency. This is attributed to the increased stability of the small molecule drug and more efficient drug delivery mediated through the antibody internalization which consequentially leads to increase of the intracellular drug concentration in the GD2+ neuroblastoma cells.

Importantly, the anti-cancer activity was completely diminished in the GD2, SK-N-SH, neuroblastoma cell line highlighting the specificity of anti-GD2/RBM39 DAC towards GD2+ cancer cells (Figure 7b). These results also show that recognition of the GD2 TAA, cellular internalization of the DAC and subsequent degrader payload release upon linker cleavage are critical for the anti-cancer activity of RBM39 TPD delivered by anti-GD2 antibodies. In such way, better control of the biological activity of the RBM39 degraders and not only cancer tissue but specific cancer cell specificity can be achieved which could potentially lead to larger therapeutic index with lower adverse side effects of this investigational class of drugs.

Altogether, these experiments demonstrated that combining the TPD of RBM39 oncoprotein and antibody-mediated drug delivery in the form of anti-GD2 DACs leads to higher anti-cancer activity in *MYCN*-amplified neuroblastoma cells and reduced side effects due to minimized exposure of the non-targeted cells to the biological activity of the RBM39 molecular glue degrader. Therefore, chemical linking of the RBM39 degrader drug to the antibody is crucial for this exceptional anti-cancer potency increase, control of the biological activity and safety which is what this invention aims to explore. It is further noted that the activity of the immunoconjugate of the invention against different cell lines correlates well with expression of GD2 on their surface, as shown in Figure 8.

*In vitro* anti-cancer activity of the first generation anti-GD2/RBM39 DAC or DB antibody in 22 h co-culture ADCC assay with freshly isolated human HD NK cells in GD2+ **(a)** IMR-32 and **(b)** STA-NB-1.2 neuroblastoma cell lines evaluated by flow cytometry-based assay is shown in Figure 9.

Results of co-culture experiments with isolated human NK cells presented in Figure 9 show that the anti-GD2/RBM39 DAC is capable of exerting its anti-cancer activity not only through the delivery of the RBM39 degrader payload but also through a secondary cell killing mechanism, such as ADCC. Increased ratio of immune effector cells, in this case NK cells freshly isolated from the blood of healthy donors (HDs) leads to higher specific lysis of GD2+ neuroblastoma cells through the antibody-dependent cytokine release. At various E:T (effector to target) ratio, a surprisingly significant and unexpected (-15%) contribution to the cytotoxic activity is observed by anti-GD2/RBM39 DAC-treated cells compared to the anti-GD2 dinutuximab beta antibody alone treatment conditions. This effect is completely absent in condition in which no effector cells were present confirming the importance of the NK cells in the induction of the ADCC. Further, more encouraging evidence for the extraordinary anti-cancer activity of the anti-GD2/RBM39 DACs, can be provided by additional experiments using NK cells isolated from different donors or whole peripheral blood mononuclear cells (PBMCs) which in addition to NK cells contain other immune effector cells, e.g., T cells, monocytes or dendritic cells that can elicit and further enhance anti-tumor immune responses.

The observed synergistic effects of the ADCC mediated by the anti-GD2 antibody and RBM39 TPD which leads to superior cancer cell cytotoxic activity is a novel and unexplored therapeutic approach which is only possible through defined chemical linkage of the anti-GD2 antibody and a RBM39 molecular glue degrader described in this invention. More detailed description of the mechanism and hypothesis for this exceptional synergistic effect is illustrated in the Figure 10.

Anti-GD2/RBM39 DACs can exert their cytotoxic activity by triggering CDC or ADCC mediated by NK cells or by other immune effector cells, e.g., monocytes or granulocytes which causes lysis of the tumor cells. Activation of the NK cells also leads to the secretion of cytokines such as interferon gamma (INF-y) that not only further stimulates anti-tumor immune responses but also upregulates MHC class I antigen presentation which is downregulated in neuroblastoma and contributes to the tumor immune evasion. On the other hand, anti-GD2/RBM39 DAC can be internalized upon the recognition of the GD2 TAA which subsequently leads to the chemical linker cleavage and release of the RBM39 degrader payload. Recruitment of RBM39 to DCAF15 leads to the proteasomal degradation of RBM39 RNA splicing factor, alterations in RNA splicing and neuroblastoma cell death. Moreover, it has been previously shown that such RNA splicing alterations result in the production of neoantigens which are then presented by tumor MHC class I molecules and enhance tumor immune recognition and killing by T cells. Such mechanism can be particularly beneficial for increasing anti-tumor immune responses against immunologically 'cold' (pediatric) tumors characterized with low tumor mutational burden. Therefore, by turning 'cold' tumors into 'hot' ones, the (anti-GD2/) RBM39 DAC biotherapeutic platform can potentially increase the efficacy of the antibody-based immunotherapy, address tumor immune escape and improve the clinical outcomes for not only high-risk neuroblastoma but also for other cancer patients.

This hypothesis is partially supported by the findings described in recently posted bioRxiv preprint (reference: RBM39 degrader invigorates natural killer cells to eradicate neuroblastoma despite cancer cell plasticity, doi: https://doi.org/10.1101/2024.03.21.586157). Authors report that treatment with RBM39 molecular glue degrader indisulam induced inflammatory tumor microenvironment and enhanced anticancer activity of NK cells (and possibly other accessory and effector immune cells) in a *in vivo* transgenic *TH-MYCN*/*ALK^{F1178L}* mouse model of high-risk neuroblastoma. Moreover, the combination of indisulam and anti-GD2 antibody-based immunotherapy resulted in a complete and durable response in the preclinical immune competent mouse model indicating that such combination could be highly effective in treating high-risk neuroblastoma. However, it is likely that the RBM39 degrader drug dose required to achieve such effects in human patients needs to be higher than recommended dose and might be associated with prohibitive toxicity (reference: E7820, an anti-cancer sulfonamide, degrades RBM39 in patients with splicing factor mutant myeloid malignancies: a phase II clinical trial, doi: 10.1038/s41375-023-02050-4). Therapeutic strategy based on antibody-mediated delivery could potentially be the solution addressing the limited clinical efficacy of RBM39 degraders and whilst the above mentioned bioRxiv preprint appears to be completely silent of such approach, the present invention relating to RBM39 degrader-based immunoconjugates provides unexpected improvements in efficacy, safety and tolerability of the combination of (anti-GD2) antibody-based immunotherapy and RBM39 degraders allowing the realization of the full translational potential of RBM39 molecular glue degrader therapeutics. This might be especially relevant for the treatment of solid tumors and hematologic malignancies, where patients have relapsed or refractory disease that is resistant to standard therapy.

### Materials and methods

Expired lots of dinutuximab beta antibody (ch14.18/CHO, Qarziba^{®}) used in this study were obtained from the Institutional pharmacy (APO) at AKH in Vienna.

### Neuroblastoma cell lines

Neuroblastoma cell lines used in this study were grown in a humidified incubator at 37 °C under 5% CO₂ with 90% humidity and split when reaching 80-90% confluency using Accutase^{®} cell detachment solution (BioLegend 423201). SK-N-SH, IMR-32, CLB-Ma, Kelly, SK-N-BE(2), SK-N-MM, STA-NB-1.2, STA-NB-6, STA-NB-8, STA-NB-10 neuroblastoma cell lines were grown in RPMI 1640 Medium, GlutaMax^{™} Supplement (Gibco 61870010), supplemented with 10% heat-inactivated FBS (non-USA origin, Sigma-Aldrich F9665), 27 mM HEPES (Gibco 15630080), 1 mM sodium pyruvate (Gibco 11360070) and penicillin (90 units/ml)-streptomycin (90 µg/ml) (Gibco 15140122). CHLA-90 neuroblastoma cell lines was grown in Iscove's Modified Dulbecco's Medium (with L-glutamine and Phenol Red, without α-thioglycerol and 2-mercaptoethanol, Gibco 21980032), supplemented with 10% heat-inactivated FBS (non-USA origin, Sigma-Aldrich F9665), 27 mM HEPES (Gibco 15630080), 1 mM sodium pyruvate (Gibco 11360070), penicillin (80 units/ml)-streptomycin (80 µg/ml) (Gibco 15140122) and 1× insulin-transferrin-selenium basal medium supplement (Gibco 41400045). Before each seeding, cell count was determined using a hemocytometer.

### Flow cytometry analysis

Flow cytometry analysis of the stained cells was performed using BD LSRFortessa^{™} flow cytometer. Experiments performed in 96-well plates were analyzed using BD^{™} High Throughput Sampler (HTS) connected BD LSRFortessa^{™} flow cytometer. Cell staining was performed on ice, with respective fluorophore-modified antibodies or fluorescent dyes diluted in BD Pharmigen^{™} Stain Buffer (FBS) (BD Biosciences 554656).

### Surface GD2 disialoganglioside quantification

Neuroblastoma cells were detached from the tissue culture flasks using Accutase^{®} cell detachment solution (BioLegend 423201), broken into single-cell suspensions and counted using a hemocytometer. 100 000 cells/tube were transferred into FACS tubes, washed with BD Pharmigen^{™} Stain Buffer (FBS) and incubated for 30 min on ice with 1:200 PE Mouse Anti-Human Disialoganglioside GD2 (Clone 14.G2α, BD Biosciences 562100) antibody, in triplicates. Cells were washed with BD Pharmigen^{™} Stain Buffer (FBS), stained for 15 min on ice with 1:100 propidium iodide solution (BioLegend, 421301) and analyzed on the BD LSRFortessa^{™} flow cytometer. Data analysis was performed using FlowJo^{™} software and graphs were created using GraphPad Prism 9 software.

### Dinutuximab beta internalization

Neuroblastoma cells were seeded in flat-bottom 96-well plates, 50000 cells/well in 100 µl of the respective cell growth medium. Plates were incubated at 37 °C for 24 h. Plates were spun down (350xg for 5 min), cell medium was aspirated and replaced with fresh 100 µl of respective cell growth medium containing 10 µg/ml of dinutuximab beta-pHrodo^{™} Deep Red (in triplicates) which was prepared using pHrodo^{™} Deep Red Antibody kit (Invitrogen, P35356) according to the manufacturer's instructions. Plates were incubated at 37 °C for 4 h. Cells were transferred to 96-well U-bottom plates, washed with BD Pharmigen^{™} Stain Buffer (FBS) (BD Biosciences 554656) and stained for 15 min on ice with 1:100 propidium iodide solution (BioLegend, 421301). Cells were analyzed using BD^{™} High Throughput Sampler (HTS) connected to the BD LSRFortessa^{™} flow cytometer. Data analysis was performed using FlowJo^{™} software and graphs were created using GraphPad Prism 9 software.

### Degrader payload screen

Neuroblastoma cells were seeded in flat-bottom 96-well plates, 10000 cells/well in 100 µl of the respective cell growth medium. Plates were incubated at 37 °C for 24 h. Small molecule degraders were added in 100 µl of the respective cell growth medium (prepared from 10 mM DMSO stock solutions, 4 compounds/plate, 1 nM-1 µM final concentrations, 0.25% final DMSO concentration, in triplicates). Plates were incubated at 37 °C for 72 h. To access the cell viability, cell medium was aspirated and replaced with 120 µl of the respective cell growth medium mixed in 5:1 ratio with CellTiter-Blue^{®} reagent (Promega, G8081). Plates were incubated at 37 °C for 4-6 h. Fluorescence (Ex/Em 0 560/590 nm) was recorded on EnSpire Multimode Plate Reader (PerkinElmer^{®}). Data were analyzed and graphs created using Graph Pad Prism 9 software.

Subsequent cytotoxic validation assays were performed in a similar fashion with accordingly modified drug concentration ranges and incubation times.

### Dinutuximab beta bioconjugation

To 100 µl of the 25 µM (3.8 mg/ml) antibody solution in reaction buffer (100 mM NaPi, 5 mM EDTA, pH 8.0) from the previous step, 2.5 µl of TCEP (10 mM in 100 mM NaPi, 5 mM EDTA, pH 8.0, freshly prepared, 10 equiv.) solution was added. Reaction mixture was vortexed and then incubated at 37 °C on a Thermo-Shaker for 30 min with shaking (300 rpm). After this, small molecules were removed and reduced antibody was exchanged to 100 mM NaPi, 5 mM EDTA, pH 8.0 buffer using Zeba^{™} Spin Desalting Column 7K MWCO (Thermo Scientific, 89883, 0.5 ml column, spinning at 4 °C). To 100 µl of the freshly reduced antibody solution in reaction buffer (100 mM NaPi, 5 mM EDTA, pH 8.0) from the previous reduction step, 10 µl of the respective maleimide-linker-drug compound (5 mM in DMSO, 20 equiv., -10% DMSO final concentration) solution was added. Reaction mixture was vortexed and then incubated at 37 °C on a Thermo-Shaker for 30 min with shaking (300 rpm). After this, small molecules were removed and Ab was exchanged to 100 mM NaPi, 5 mM EDTA, pH 8.0 buffer using Zeba^{™} Spin Desalting Column 7K MWCO (Thermo Scientific, 89883, 0.5 ml column, spinning at 4 °C). The yield of the bioconjugation reaction was determined using Bradford Bio-Rad Protein Assay (Bio-Rad, 500-0006) according to the manufacturer's instructions.

### HR LC-MS characterization of DACs

LC-MS analysis of the antibody modification reactions was performed on a Waters SYNAPT G2-Si mass spectrometer operated in a resolution mode and coupled to an Dionex nano HPLC system using an Waters XBridge Protein BEH C4 column and a step gradient 12-40-72% of MeCN. Total mass spectra were reconstructed from the ion series using the MaxEnt1 algorithm preinstalled on MassLynx software (v. 4.1 from Waters) according to the manufacturer's instructions.

### In vitro anti-cancer activity

Neuroblastoma cells were seeded in flat-bottom 96-well plates, 10000 cells/well in 100 µl of the respective cell growth medium. Plates were incubated at 37 °C for 24 h. Dinutuximab beta antibody, degrader-antibody conjugate or small molecule degrader were added in 100 µl of the respective cell growth medium (prepared from DPBS for antibody/conjugate or 10 mM DMSO stock solutions for the small molecule degraders, 0.5 nM-500 nM final concentrations, 0.25% final DMSO concentration, in triplicates). Plates were incubated at 37 °C for 66-72 h. To access the cell viability, cell medium was aspirated and replaced with 120 µl of the respective cell growth medium mixed in 5:1 ratio with CellTiter-Blue^{®} reagent (Promega, G8081). Plates were incubated at 37 °C for 4-6 h. Fluorescence (Ex/Em 0 560/590 nm) was recorded on EnSpire Multimode Plate Reader (PerkinElmer^{®}). Data were analyzed and graphs created using Graph Pad Prism 9 software.

### ADCC assay

Neuroblastoma cells were collected using accutase and counted. 6-10x10⁶ of neuroblastoma cells were washed with DPBS (2x10 ml, spun down at 300xg for 5 min) to remove residual serum proteins and taken up in DPBS (2 ml). 1 µl of the Violet Proliferation Dye 450 (BD 562158, 1 mM DMSO stock, 0.5 µM VPD450 dye final concentration, 0.05% DMSO final concentration) was added. Cells were incubated in a water bath at 37 °C for 15 min (mixed well every 5 min). Blocking was performed by addition of respective cell growth medium (10 ml), cells were washed with the respective cell growth medium (1×10 ml) and resuspended in the respective cell growth medium. Cells were counted again and seeded in flat-bottom 96-well plates, 50000 cells/well in 100 µl of the respective cell growth medium. Plates were incubated at 37 °C for 24 h. Plates were spun down (300xg for 5 min) and dinutuximab beta or DAC were added in 50 µl the respective cell growth medium (0-2.5 µg/ml final concentration, in triplicates) followed by the addition of 50 µl of the respective cell growth medium containing freshly isolated HD human NK cells (using RosetteSep^{™} Human NK Cell Enrichment Cocktail, STEMCELL Technologies 15065) in E:T ratios 2:1, 1:1, 1:2, 1:4 or 1:8 (in triplicates). Plates incubated at 37 °C for 20-24 h. Cells were transferred to 96-well U-bottom plates, washed with BD Pharmigen^{™} Stain Buffer (FBS) (BD Biosciences 554656) and stained for 5 min on ice with 1:100 7-AAD viability staining solution (50 µg/ml, Invitrogen 00-6993-50). Cells were analyzed using BD^{™} High Throughput Sampler (HTS) connected to the BD LSRFortessa^{™} flow cytometer. Data analysis was performed using FlowJo^{™} software and graphs were created using Graph Pad Prism 9 software.

### Organic synthesis

### General remarks

All synthetic reaction using anhydrous solvents were performed under argon atmosphere in heat gun-dried glassware unless stated otherwise. All the reagents and solvents were purchased from commercial suppliers and used as received. High-performance flash chromatography (HPFC) purifications were performed on on Biotage Selekt apparatus over Biotage Sfar Silica HC D column cartridges for normal-phase HPFC or Biotage Sfar Silica C18 D column cartridges for reversed-phase high-performance flash chromatography (RP-HPFC) employing Merck silica gel (Kieselgel 60, 63-200 µm). Monitoring of reactions was performed using pre-coated TLC Silica gel 60 F254 plates. Compounds were detected using shortwave (254 nm) or longwave (365 nm) UV lamp, eventually by staining with an indicated solution prepared by known procedures. NMR spectra were recorded on Bruker AV Neo 500, AV III 600 and AV III HD 700 instruments), in CDCl₃ (TMS was used as internal standard), MeOH-*d₄* (referenced to the residual solvent signal) acetone-*d₆* (referenced to the residual solvent signal) or DMSO-*d₆* (referenced to the residual solvent signal). Chemical shifts are given in ppm (δ-scale), coupling constants (J) in Hz and the NMR peak multiplicities are denoted as follows: s, singlet; d, doublet; dd, doublet of doublets: t, triplet; m, multiplet; br s, broad singlet. Low resolution mass spectra were measured on Bruker amaZon speed ETD instrument using electrospray ionization (ESI) while high resolution (ESI-TOF) analyses were performed on Bruker timsTOF flex instrument.

### Synthesis of the AMe-deCN-E7820 RBM39 degrader payload (JKW-48)

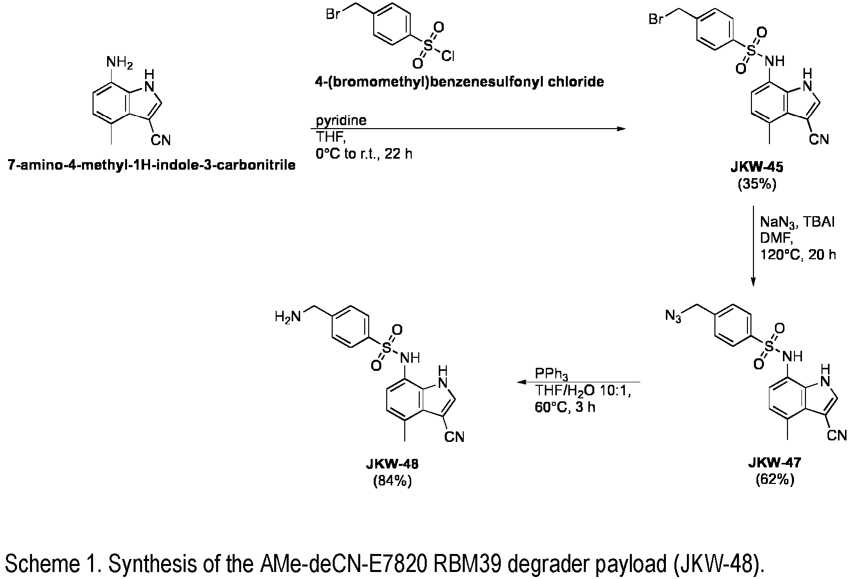

### 4-(Bromomethyl)-N-(3-cyano-4-methyl-1H-indol-7-yl)benzenesulfonamide (JKW-45)

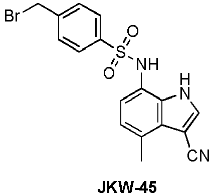

Pyridine (194 µl, 2.40 mmol) was added to an ice-cooled solution of 7-amino-4-methyl-1H-indole-3-carbonitrile (137 mg, 0.80 mmol) in 3 ml of anhydrous THF under an argon atmosphere. The resulting reaction mixture was stirred with cooling for 10 min, then 4-(bromomethyl)benzenesulfonyl chloride (324 mg, 1.20 mmol) was added in 3 ml of anhydrous THF. The resulting reaction mixture was stirred with cooling for 10 min and then it was allowed to warm to r.t. and stirred at r.t. for 22 h. Reaction mixture was then diluted with EtOAc (30 ml) and washed with dH₂O (20 ml). Aq. phase was extracted with EtOAc (20 ml), combined org. layers were dried over anhydrous MgSO₄, solvents were removed under reduced pressure and the crude product was purified using HPFC (silica column, 0→60% EtOAc in hexane). The title compound JKW-45 (113 mg, 0.28 mmol, 35%) was obtained as a reddish solid. ¹H NMR (500.0 MHz, DMSO-*d₆*) δ 11.92 (s, 1H), 9.97 (s, 1H), 8.17 (m, 1H), 7.71-7.67 (m, 2H), 7.59-7.57 (m, 2H), 6.78 (d, *J* = 7.6 Hz, 1H), 6.57-6.55 (m, 1H), 4.80 (s, 1H), 4.72 (s, 1H), 2.56 (s, 3H).

HR MS (ESI) for C₁₇H₁₅BrN₃O₂S [M+H]+: calcd. 404.0063; found 404.0062.

### 4-(Azidomethyl)-N-(3-cyano-4-methyl-1H-indol-7-yl)benzenesulfonamide (JKW-47)

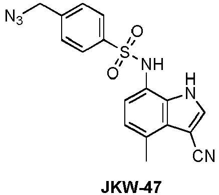

A reaction mixture consisting of JKW-45 (104 mg, 0.26 mmol), NaN₃ (85 mg, 1.31 mmol) and TBAI (20 mg, 0.05 mmol) in 2 ml of anhydrous DMF under an argon atmosphere was stirred at 120 °C, 22 h. Reaction mixture was then diluted with EtOAc (25 ml) and washed with dH₂O (25 ml). Org. phase was dried over anhydrous MgSO₄, solvents were removed under reduced pressure and the crude product was purified using HPFC (silica column, 0→100% EtOAc in hexane). The title compound JKW-47 (59 mg, 0.16 mmol, 62%) was obtained as a yellowish solid.

¹H NMR (500.0 MHz, CDCl₃) δ 9.80 (s, 1H), 7.79 (d, *J* = 2.9 Hz, 1H), 7.63 (d, *J*= 8.4 Hz, 2H), 7.37 (d, *J* = 8.4 Hz, 2H), 6.75 (dd, *J* = 7.7, 1.0 Hz, 1H), 6.70 (s, 1H), 6.37 (d, *J =* 7.7 Hz, 1H), 4.42 (s, 2H), 2.72 (s, 3H).

HR MS (ESI) for C₁₇H₁₅N₆O₂S [M+H]+: calcd. 367.0972; found 367.0974.

### 4-(Aminomethyl)-N-(3-cyano-4-methyl-1H-indol-7-yl)benzenesulfonamide (JKW-48)

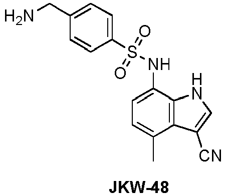

A reaction mixture consisting of JKW-47 (52 mg, 0.14 mmol) and PPh₃ (56 mg, 0.21 mmol) in 1 ml of THF/dH₂O mixture (10:1) under an argon atmosphere was stirred at 60 °C for 3 h. Reaction mixture was then concentrated under reduced pressure, residue was diluted with EtOAc (20 ml) and washed with dH₂O (20 ml). Org. phase was dried over anhydrous MgSO₄, solvents were removed under reduced pressure and the crude product was purified using HPFC (silica column, 0->10% MeOH in DCM). The title compound JKW-48 (40 mg, 0.12 mmol, 84%) was obtained as a brownish solid.

¹H NMR (500.0 MHz, MeOH-*d₄*) δ 7.93 (s, 1H), 7.63 (d, J= 8.4 Hz, 2H), 7.38 (d, J= 8.4 Hz, 2H), 6.73 (dd, *J* = 7.7, 1.0 Hz, 1H), 6.49 (d, *J* = 7.7 Hz, 1H), 4.52 (s, 2H), 2.65 (d, *J* = 0.8 Hz, 3H).

HR MS (ESI) for C₁₇H₁₇N₄O₂S [M +H]+: calcd. 341.1067; found 341.1068.

### Synthesis of the chemical linker-modified AMe-deCN-E7820 RBM39 degrader payload (JKW-68)

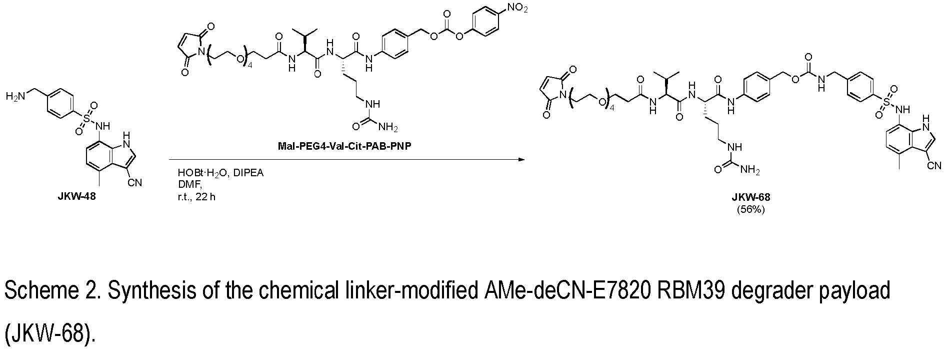

### 4-((17S,20S)-1-(2,5-Dioxo-2,5-dihydro-1H-pyrrol-1-yl)-17-isopropyl-15,18-dioxo-20-(3-ureidopropyl)-3,6,9,12-tetraoxa-16,19-diazahenicosan-21-amido)benzyl (4-(N-(3-cyano-4-methyl-1H-indol-7-yl)sulfamoyl)benzyl)carbamate (JKW-68)

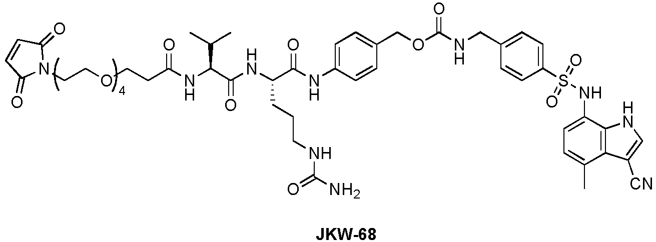

DIPEA (7 µl, 0.04 mmol) was added to a solution of Mal-PEG4-Val-Cit-PAB-PNP (17.5 mg, 0.02 mmol), JKW-48 (7 mg, 0.02 mmol) and HOBt·H₂O (6.2 mg, 0.04 mmol) in 0.5 ml of anhydrous DMF. The resulting reaction mixture was stirred at r.t. for 22 h. Solvents were then removed under reduced pressure (co-evaporated with toluene 3×7 ml) and the crude product was purified using reversed-phase high-performance flash chromatography (RP-HPFC) (C18 column, 10→100% MeCN in H₂O). The title compound JKW-68 (12 mg, 0.01 mmol, 56%) was obtained as an off-white solid.

HR MS (ESI) for C₅₁H₆₅N₁₀O₁₄S [M+H]⁺: calcd. 1073.4397; found 1073.4395.

### Synthesis of the chemical linker-modified MMAE payload (JKW-8)

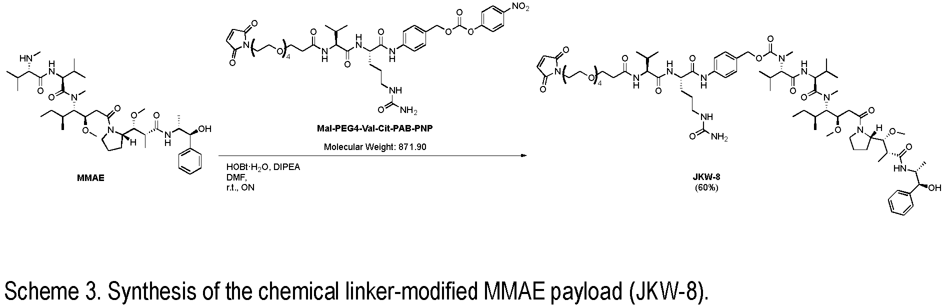

### 4-((17S,20S)-1-(2,5-Dioxo-2,5-dihydro-1H-pyrrol-1-yl)-17-isopropyl-15,18-dioxo-20-(3-ureidopropyl)-3,6,9, 12-tetraoxa-16, 19-diazahenicosan-21-amido)benzyl ((S)-1-(((S)-1-(((3R,4S,5S)-1-((S)-2-((1R,2R)-3-(((1S,2R)-1-hydroxy-1-phenylpropan-2-yl)amino)-1-methoxy-2-methyl-3-oxopropyl)pyrrolidin-1-yl)-3-methoxy-5-methyl-1-oxoheptan-4-yl)(methyl)amino)-3-methyl-1-oxobutan-2-yl)amino)-3-methyl-1-oxobutan-2-yl)(methyl)carbamate (JKW-8)

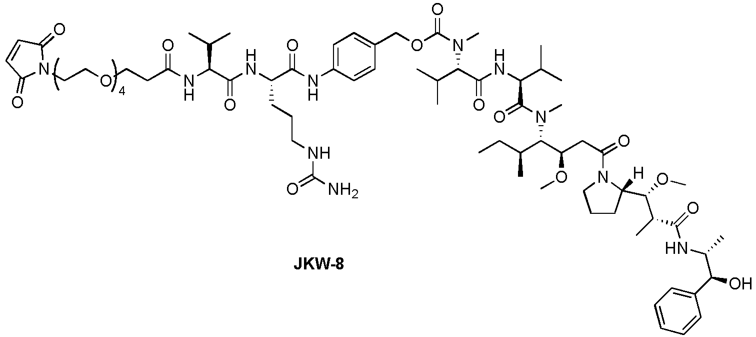

DIPEA (6 µl, 0.034 mmol) was added to a solution of Mal-PEG4-Val-Cit-PAB-PNP (13.5 mg, 0.015 mmol), MMAE (11 mg, 0.015 mmol) and HOBt·H₂O (5 mg, 0.033 mmol) in 0.5 ml of anhydrous DMF. The resulting reaction mixture was stirred at r.t. for 24 h. Solvents were then removed under reduced pressure (co-evaporated with toluene 2x6 ml) and the crude product was purified using RP-HPFC (C18 column, 10→50% MeCN in H₂O). The title compound JKW-8 (13 mg, 0.009 mmol, 60%) was obtained as a white solid.

HR MS (ESI) for C₇₃H₁₁₆N₁₁O₁₉ [M+H]⁺: calcd 1450.8443; found 1450.8446.

### Synthesis of the chemical linker-modified Exatecan payload (JKW-130)

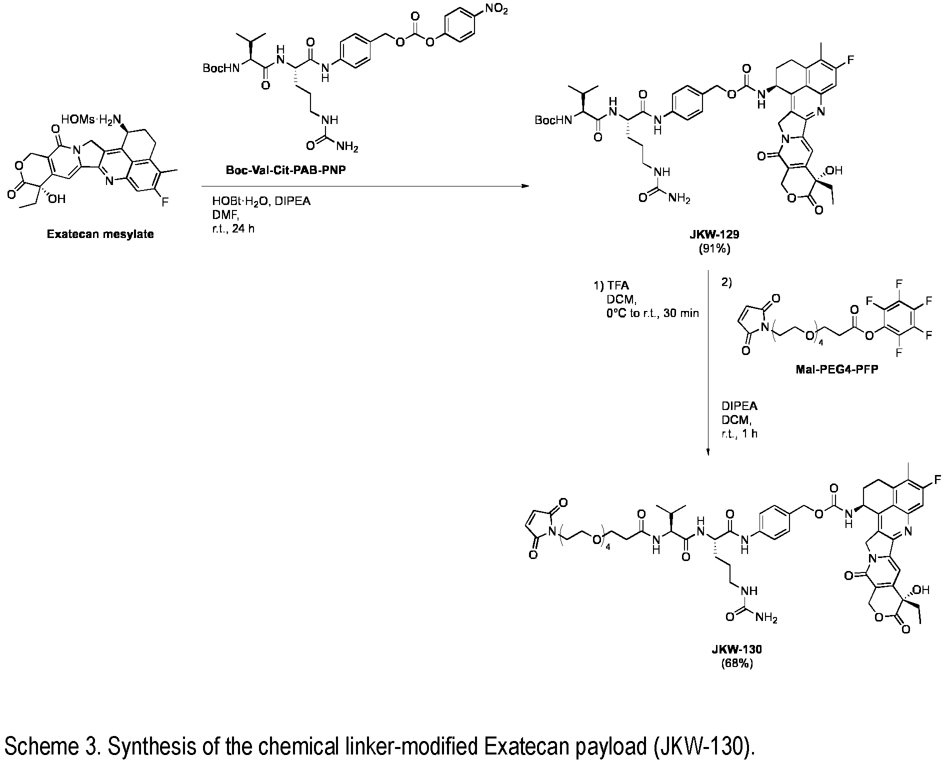

### 4-((S)-2-((S)-2-((tert-butoxycarbonyl)amino)-3-methylbutanamido)-5-ureidopentanamido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (JKW-129)

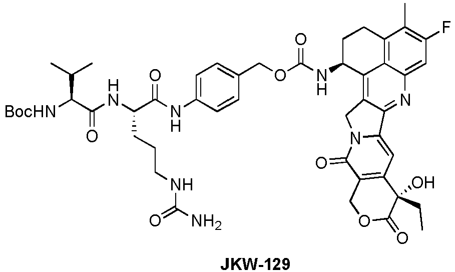

DIPEA (25 µl, 0.15 mmol) was added to a suspension of Boc-Val-Cit-PAB-PNP (30 mg, 0.05 mmol), Exatecan mesylate (25 mg, 0.05 mmol) and HOBt·H₂O (15 mg, 0.10 mmol) in 0.5 ml DMF under argon atmosphere. The resulting reaction mixture was stirred at r.t. for 24 h. Solvents were then removed under reduced pressure (co-evaporated with toluene 3×20 ml) and the crude product was purified using HPFC (sillica column, 0→10% MeOH in DCM). The title compound JKW-129 (43 mg, 0.04 mmol, 91%) was obtained as a yellow solid.

HR MS (ESI) for C₄₈H₅₇FN₈O₁₁Na [M+Na]⁺: calcd 963.4023; found 963.4026.

### 4-((17S,20S)-1-(2,5-dioxo-2,5-dihydro-1H-pyrrol-1-yl)-17-isopropyl-15,18-dioxo-20-(3-ureidopropyl)-3,6,9,12-tetraoxa-16,19-diazahenicosan-21-amido)benzyl ((1S,9S)-9-ethyl-5-fluoro-9-hydroxy-4-methyl-10,13-dioxo-2,3,9,10,13,15-hexahydro-1H,12H-benzo[de]pyrano[3',4':6,7]indolizino[1,2-b]quinolin-1-yl)carbamate (JKW-130)

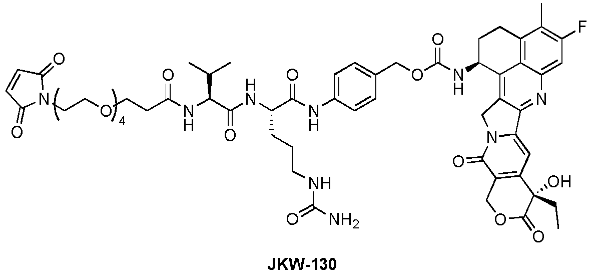

TFA (0.5 ml) was added to an ice-cooled suspension of JKW-129 (41 mg, 0.04 mmol) in 2 ml of anhydrous DCM under an argon atmosphere. The resulting reaction mixture was allowed to warm to r.t. and stirred at r.t. for 30 min. Solvents were then removed under reduced pressure (co-evaporated with methanol 3×2 ml). Crude product and Mal-PEG4-PFP (25 mg, 0.05 mmol) were dissolved in 0.5 ml DMF under an argon atmosphere and DIPEA (23 µl, 0.13 mmol) was added. The resulting reaction mixture was stirred at r.t. for 1 h. Solvents were then removed under reduced pressure (co-evaporated with toluene 3x10 ml) and the crude product was purified using reversed-phase high-performance flash chromatography (RP-HPFC) (C18 column, 10→50% MeCN in H₂O). The title compound JKW-130 (32 mg, 0.03 mmol, 68%) was obtained as a yellowish solid.

HR MS (ESI) for C₅₈H₇₁FN₉O₁₆Na [M+H]+: calcd 1168.4997; found 1168.4994.

### Synthesis of the chemical linker-modified E7820 RBM39 degrader payload (JKW-162)

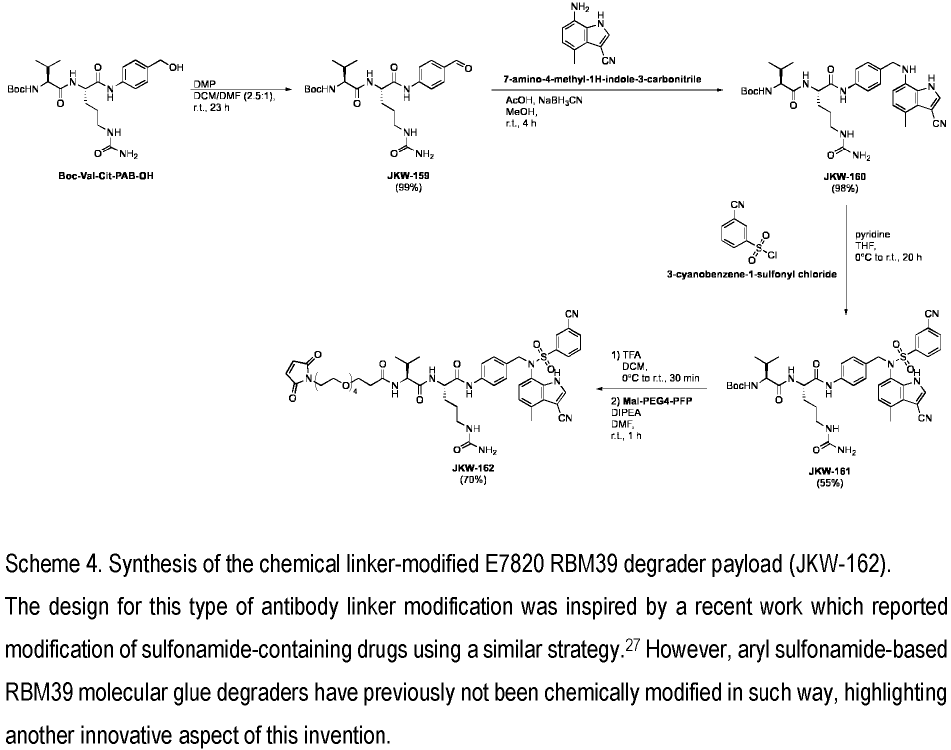

### tert-butyl ((S)-1-(((S)-1-((4-formylphenyl)amino)-1-oxo-5-ureidopentan-2-yl)amino)-3-methyl-1-oxobutan-2-yl)carbamate (JKW-159)

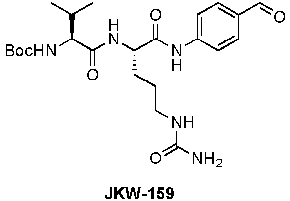

A reaction mixture consisting of Boc-Val-Cit-PAB-OH (120 mg, 0.25 mmol) and Dess-Martin Periodinane (253 mg, 0.60 mmol) in 7 ml of DCM/DMF (2.5:1) under an argon atmosphere was stirred at r.t. for 23 h. The reaction mixture was quenched with addition of dH₂O (2 ml) and Na₂S₂O₃ (300 mg), and stirred at r.t. for 30 min. After that, the reaction mixture was concentrated under reduced pressure and the crude product was purified using HPFC (sillica column, 0->15% MeOH in DCM). The title compound JKW-159 (118 mg, 0.25 mmol, 99%) was obtained as a white solid.

LR MS (ESI) for C₂₃H₃₅N₅O₆Na [M+Na]⁺: calcd 500.25; found 500.28.

### tert-butyl ((S)-1-(((S)-1-((4-(((3-cyano-4-methyl-1H-indol-7-yl)amino)methyl)phenyl)amino)-1 -oxo-5-ureidopentan-2-yl)amino)-3-methyl-1-oxobutan-2-yl)carbamate (JKW-160)

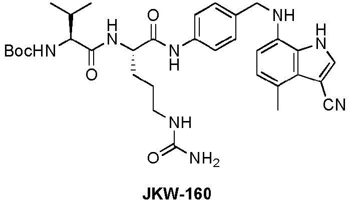

A solution of JKW-159 (120 mg, 0.25 mmol) and 7-amino-4-methyl-1H-indole-3-carbonitrile (43 mg, 0.25 mmol) in 5 ml of anhydrous MeOH and 300 µl of glacial acetic acid (5% final concentration) was stirred at r.t. for 1 h under an argon atmosphere. NaBH₃CN (24 mg, 0.38 mmol) was then added and the reaction mixture was stirred at r.t. for 3 h. Solvents were removed under reduced pressure and the crude product was purified using HPFC (sillica column, 0->15% MeOH in DCM). The title compound JKW-160 (155 mg, 0.24 mmol, 98%) was obtained as a yellowish solid.

HR MS (ESI) for C₃₃H₄₄N₈O₅Na [M+Na]⁺: calcd 655.3327; found 655.3318.

### tert-butyl ((S)-1-(((S)-1-((4-(((3-cyano-N-(3-cyano-4-methyl-1H-indol-7-yl)phenyl)sulfonamido)methyl)phenyl)amino)-1-oxo-5-ureidopentan-2-yl)amino)-3-methyl-1-oxobutan-2-yl)carbamate (JKW-161)

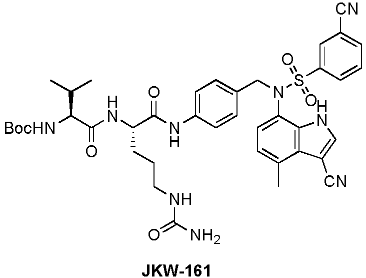

Pyridine (29 µl, 0.36 mmol) was added to an ice-cooled solution of JKW-160 (76 mg, 0.12 mmol) in 1 ml of anhydrous THF under an argon atmosphere. The resulting reaction mixture was stirred with cooling for 10 min, then 3-cyanobenzene-1-sulfonyl chloride (36 mg, 0.18 mmol) was added in 0.5 ml of anhydrous THF. The resulting reaction mixture was stirred with cooling for 10 min and then it was allowed to warm to r.t. and stirred at r.t. for 20 h. Solvents were removed under reduced pressure and the crude product was purified using HPFC (sillica column, 0→20% MeOH in DCM). The title compound JKW-161 (53 mg, 0.07 mmol, 55%) was obtained as an off-white solid.

LR MS (ESI) for C₄₀H₄₇N₉O₇SNa [M+Na]⁺: calcd 820.32; found 820.34.

### N-((S)-1-(((S)-1-((4-(((3-cyano-N-(3-cyano-4-methyl-1H-indol-7-yl)phenyl)sulfonamido)methyl)phenyl)amino)-1-oxo-5-ureidopentan-2-yl)amino)-3-methyl-1-oxobutan-2-yl)-1-(2,5-dioxo-2,5-dihydro-1H-pyrrol-1-yl)-3,6,9,12-tetraoxapentadecan-15-amide (JKW-162)

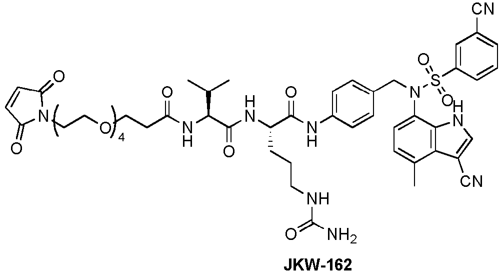

TFA (0.5 ml) was added to an ice-cooled suspension of JKW-161 (50 mg, 0.06 mmol) in 2 ml of anhydrous DCM under an argon atmosphere. The resulting reaction mixture was allowed to warm to r.t. and stirred at r.t. for 30 min. Solvents were then removed under reduced pressure (co-evaporated with methanol 3x2 ml). Crude product and Mal-PEG4-PFP (30 mg, 0.06 mmol) were dissolved in 0.5 ml DMF under an argon atmosphere and DIPEA (32 µl, 0.13 mmol) was added. The resulting reaction mixture was stirred at r.t. for 1 h. Solvents were then removed under reduced pressure (co-evaporated with toluene 3x10 ml) and the crude product was purified using reversed-phase high-performance flash chromatography (RP-HPFC)

(C18 column, 10→50% MeCN in H₂O). The title compound JKW-162 (43 mg, 0.04 mmol, 70%) was obtained as an off-white solid.

HR MS (ESI) for C₅₀H₆₁N₁₀O₁₂S [M+H]⁺: calcd 1025.4186; found 1025.4179.

### References

1) C. Xu et al., RNA-binding protein 39: a promising therapeutic target for cancer. Cell Death Discov. 7, 214 (2021).
2) T. Han et al., Anticancer sulfonamides target splicing by inducing RBM39 degradation via recruitment to DCAF15. Science 356, eaal3755 (2017).
3) T. Uehara et al., Selective degradation of splicing factor CAPERα by anticancer sulfonamides. Nat. Chem. Biol. 13, 675-680 (2017).
4) S. Singh et al., Targeting the spliceosome through RBM39 degradation results in exceptional responses in high-risk neuroblastoma models. Sci. Adv. 7, eabj5405 (2021).
5) A. Nijhuis et al., Indisulam targets RNA splicing and metabolism to serve as a therapeutic strategy for high-risk neuroblastoma. Nat. Commun. 13, 1380 (2022).
6) R. Ladenstein et al., Investigation of the Role of Dinutuximab Beta-Based Immunotherapy in the SIOPEN High-Risk Neuroblastoma 1 Trial (HR-NBL1). Cancers 12, 309 (2020).
7) A. L. Yu et al., Anti-GD2 antibody with GM-CSF, interleukin-2, and isotretinoin for neuroblastoma. N. Engl. J. Med. 363, 1324-1334 (2010).
8) R. Ladenstein et al., Interleukin 2 with anti-GD2 antibody ch14.18/CHO (dinutuximab beta) in patients with high-risk neuroblastoma (HR-NBL1/SIOPEN): a multicentre, randomised, phase 3 trial. Lancet. Oncol., 19, 1617-1629 (2018).
9) H. N. Lode et al., Long-term, continuous infusion of single-agent dinutuximab beta for relapsed/refractory neuroblastoma: an open-label, single-arm, Phase 2 study. Br. J. Cancer 129, 1780-1786 (2023).
10) F. Del Bufalo et al., GD2-CART01 for Relapsed or Refractory High-Risk neuroblastoma. N. Engl. J. Med., 388, 1284-1295 (2023).
11) A. Heczey et al., Anti-GD2 CAR-NKT cells in relapsed or refractory neuroblastoma: updated phase 1 trial interim results. Nat. Med. 29, 1379-1388 (2023).
12) F. Zirngibl et al., GD2-directed bispecific trifunctional antibody outperforms dinutuximab beta in a murine model for aggressive metastasized neuroblastoma. J. Immunother. Cancer 9, e002923 (2021).
13) R. Ladenstein et al., Ch14.18 antibody produced in CHO cells in relapsed or refractory Stage 4 neuroblastoma patients: A SIOPEN Phase 1 study. MAbs 5, 801-809 (2013).
14) H. N. Lode et al., Targeted Therapy with a Novel Enediyene Antibiotic Calicheamicin ϑI1 Effectively Suppresses Growth and Dissemination of Liver Metastases in a Syngeneic Model of Murine Neuroblastoma. Cancer Res. 58, 2925-2928 (1998).
15) D. V. Kalinovsky et al., Therapeutic efficacy of antibody-drug conjugates targeting GD2- positive tumors. J. Immunother. Cancer 10, e004646 (2022).
16) R. Sano et al., An antibody-drug conjugate directed to the ALK receptor demonstrates efficacy in preclinical models of neuroblastoma. Sci. Transl. Med. 11, eaau9732 (2019).
17) K. R. Bosse et al., Identification of GPC2 as an oncoprotein and Candidate Immunotherapeutic Target in High-Risk Neuroblastoma. Cancer Cell 32, 295-309 (2017).
18) S. Raman et al., A GPC2 antibody-drug conjugate is efficacious against neuroblastoma and small-cell lung cancer via binding a conformational epitope. Cell Rep. Med. 2, 100344 (2021)
19) S. Buongervino et al., Antibody-Drug Conjugate Efficacy in Neuroblastoma: Role of Payload, Resistance Mechanisms, Target Density, and Antibody Internalization. Mol. Cancer Ther. 20, 2228-2239 (2021)
20) N. M. Kendersky et al., The B7-H3-Targeting Antibody-Drug Conjugate m276-SL-PBD Is Potently Effective Against Pediatric Cancer Preclinical Solid Tumor Models. Clin. Cancer Res. 27, 2938-2946 (2021).
21) Z. Fu et al., Peripheral neuropathy associated with monomethyl auristatin E-based antibody-drug conjugates. iScience 26, 107778 (2023).
22) R. Zhang et al., Systematic pan-cancer analysis identifies RBM39 as an immunological and prognostic biomarker. J. Cell Mol. Med.26, 4859-4871 (2022).
23) S. X. Lu et al., Pharmacologic modulation of RNA splicing enhances anti-tumor immunity. Cell 184, 4032-4032 (2021).
14) X. Du et al., Structural Basis and Kinetic Pathway of RBM39 Recruitment to DCAF15 by a Sulfonamide Molecular Glue E7820. Structure, 27, 1625-1633 (2019)
25) T. B. Faust et al., Structural complementarity facilitates E7820- mediated degradation of RBM39 by DCAF15 Nat. Chem. Biol., 16, 7-14 (2020).
26) D. E. Bussiere et al., Structural basis of indisulam-mediated RBM39 recruitment to DCAF15 E3 ligase complex. Nat. Chem. Biol., 16, 15-23 (2020).
27) J. H. Kostyo et al., Sulfonamide Prodrugs with a Two-Stage Release Mechanism for the Efficient Delivery of the TLR4 Antagonist TAK-242. ACS Med. Chem. Lett., 14, 110-115 (2023).

## Claims

1. An immunoconjugate comprising
a) an antibody binding to a tumor antigen displayed on the surface of a cancer cell; and
b) a compound according to formula (I): 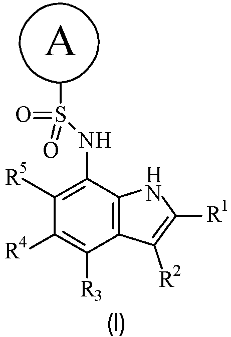 wherein
R¹ is selected from -H, C₁₋₅ alkyl, -OH, -CN and -NO₂;
R² is selected from -H, halogen, C₁₋₅ alkyl, C₁₋₅ haloalkyl, -OH, -CN, -NO₂, -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
R³ is selected from -H, halogen, C₁₋₅ alkyl, aryl, -OH, -O(C₁₋₅ alkyl), -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
R⁴ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl);
R⁵ is selected from -H, halogen, C₁₋₅ alkyl, aryl, -CN, -NO₂, -NH₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl); and
A is a phenyl or five- or six-membered heteroaryl, optionally substituted with one or more groups selected from R^{S}; and
each R^{S} is independently selected from halogen, C₁₋₅ alkyl, C₁₋₅ haloalkyl, -OH, -O(C₁₋₅ alkyl), -(C₁₋₅ alkylene)-OH, -CN, -NH₂, -(C₁₋₅ alkylene)-NH₂, -(C₁₋₅ alkylene)-NH(C₁₋₅ alkyl), -(C₁₋₅ alkylene)-N(C₁₋₅ alkyl)(C₁₋₅ alkyl), -0-(C₁₋₅ alkylene)-NH₂, -O-(C₂₋₅ alkylene)-NH(C₁₋₅ alkyl), -O-(C₂₋₅ alkylene)-N(C₁₋₅ alkyl)(C₁₋₅ alkyl), -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, -SO₂NH(C₁₋₅ alkyl) and - SO₂N(C₁₋₅ alkyl)(C₁₋₅ alkyl).

2. The immunoconjugate of claim 1, wherein the cancer cell is selected from a neuroblastoma cell, an Ewing sarcoma cell, an osteosarcoma cell, a leukemia cell (such as AML cell), a rhabdomyosarcoma cell, a melanoma cell, a breast cancer cell, a triple negative breast cancer cell, a lung cancer cell, a colorectal cancer cell (such as colorectal adenoma cell), an ovarian cancer cell, a multiple myeloma cell, a hepatocellular carcinoma cell, pancreatic cancer cell, brain cancer cell (such as glioma cell), a medulloblastoma cell, prostate cancer cell, bladder cancer cell, urethral cancer cell, cervical cancer cell, a soft tissue sarcoma cell and a lymphoma cell,
preferably wherein the cancer cell is selected from a neuroblastoma cell, an Ewing sarcoma cell, an osteosarcoma cell, a rhabdomyosarcoma cell, a melanoma cell and a triple negative breast cancer cell.

3. The immunoconjugate of claim 1 or 2, wherein the tumor antigen is selected from GD2, O-acetyl-GD2 (such as 7-O-acetyl-GD2 or 9-O-acetyl-GD2), B7-H3, GPC2, ALK, HER2, HER3, Trop-2, CD33, CLL-1, c-KIT, CD123, EGFR, FR-α, MUC16 and STEAP1,
preferably wherein the tumor antigen is selected from GD2, O-acetyl-GD2, B7-H3, GPC2, and ALK.

4. The immunoconjugate of claim 3, wherein the tumor antigen is GD2.

5. The immunoconjugate of claim 4, wherein (a) is dinutuximab, dinutuximab beta or naxitamab.

6. The immunoconjugate of any one of claims 1 to 5, wherein the immunoconjugate comprises a moiety according to formula (II):
-L₁-L_{P}-L₂-X- (II)
wherein
L₁ is C₂₋₂₀ alkylene, optionally substituted with one or more substituents selected from -CN, halogen, -OH, and -NH₂, and wherein one or more -CH₂- groups may independently be replaced by a group selected from -CH=CH-, -C=C-, -O-, -NR'-, -CO-, C₆₋₁₀ arylene, 5-6 membered heteroarylene, - (CH₂-O-CH₂)-, -(CH₂-CH₂-O)-, -CO-O-, -CO-NR'-, C₃-C₁₁ cycloalkylene, or 4-11 membered heterocycloalkylene, wherein R' is H or C₁-C₆ alkyl;
L_{P} is a bond or is a peptide linker which includes up to 5 amino acid residues, wherein the C-terminus of the peptide linker is connected to -NH- moiety of L₂ or to X,
L₂ is -NH-1,4-phenylene-CH₂- or is a bond, and
X is selected from a bond, -O-, -O-CO-, -CO-O-, and -NH-,
wherein X is attached to the compound of formula (I).

7. The immunoconjugate of claim 6, wherein L₁ is C₂₋₁₂ alkylene, optionally substituted with one or more substituents selected from -CN, halogen, -OH, and -NH₂, and wherein one or more -CH₂-groups may independently be replaced by a group selected from -O-, -NR'-, -CO-, -(CH₂-O-CH₂)-, -(CH₂-CH₂-O)-, C₃-C₁₁ cycloalkylene, and 4-11 membered heterocycloalkylene, wherein R' is H or C₁-C₆ alkyl,
preferably wherein L₁ is selected from: 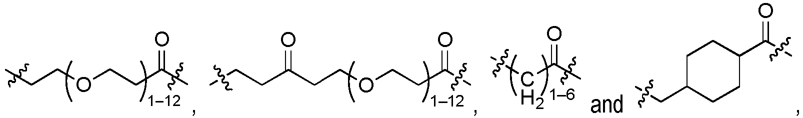
wherein the right empty valence of L₁ is attached to L_{P}, more preferably wherein the L₁ is: 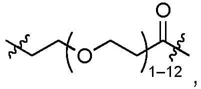
even preferably wherein the L₁ is 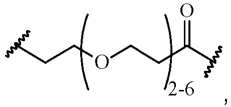 again more preferably wherein the L₁ is 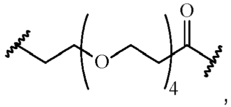
wherein the right empty valence of L₁ is attached to L_{P}, and/or
wherein L_{P} is selected from -Val-Cit-, -cBu-Cit-, -Val-Ala-, -Glu-Val-Cit-, -Gly-Gly-Gly-, -Ala-Ala-Asn- and -Gly-Gly-Phe-Gly-, wherein the C-terminus of the peptide linker is connected to -NH- moiety of L₂ or to X, and wherein the N-terminus is connected to L₁,
preferably wherein L_{P} is -Gly-Gly-Phe-Gly-, wherein the C-terminus of the peptide linker is connected to -NH- moiety of L₂ or to X, and wherein the N-terminus is connected to L₁,
or wherein the peptide linker is -Val-Cit-, wherein the C-terminus of the peptide linker is connected to -NH- moiety of L₂ or to X, and wherein the N-terminus is connected to L₁, and/or
wherein X is -O-CO-, wherein the right empty valence of said -O-CO- is attached to the compound of formula (I),
preferably wherein X is a bond.

8. The immunoconjugate of claim 6 or 7, wherein the moiety of formula (II) is comprised in a moiety of formula (Ila):
-Z-L₁-L_{P}-L₂-X- (IIa)
wherein Z is 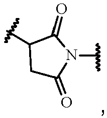 attached through its right empty valence to L₁, and through its left empty valence to a cysteine residue of the antibody, and
wherein L₁, L_{P}, L₂, and X are as defined in claim 6 or 7.

9. The immunoconjugate of any one of claims 1 to 8, wherein R¹ is -H or -OH, preferably wherein R¹ is -H,
and/or
wherein R² is selected from -H, halogen, -OH, -CN, -CONH₂, -CONH(C₁₋₅ alkyl), and -CON(C₁₋₅ alkyl)(C₁₋₅ alkyl), preferably wherein R² is -H, halogen or -CN, more preferably wherein R² is -CN, and/or
wherein R³ is selected from -H, halogen, and C₁₋₅ alkyl, preferably wherein R³ is selected from -H and C₁₋₅ alkyl (such as methyl),
and/or
wherein R⁴ is selected from -H and halogen, preferably wherein R⁴ is -H,
and/or
wherein R⁵ is selected from -H and halogen, preferably wherein R⁵ is -H,
and/or
wherein A is phenyl optionally substituted with one or more groups selected from -H, halogen, C₁₋₅ alkyl, C₁₋₅ haloalkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), -CON(C₁₋₅ alkyl)(C₁₋₅ alkyl), -SO₂NH₂, - SO₂NH(C₁₋₅ alkyl) and -SO₂N(C₁₋₅ alkyl)(C₁₋₅ alkyl), preferably a phenyl substituted with methyl or a phenyl substituted with -CN.

10. The immunoconjugate of any one of claims 1 to 8, wherein the compound of formula (I) is selected from:

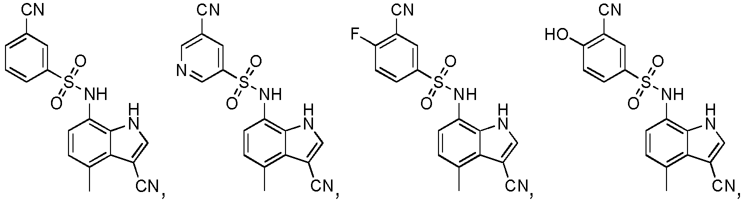

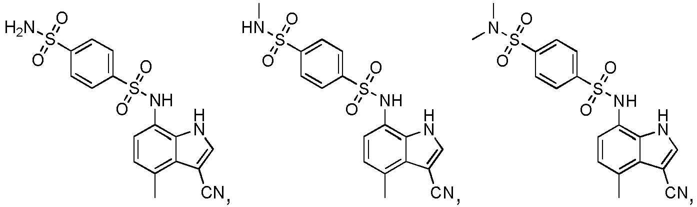

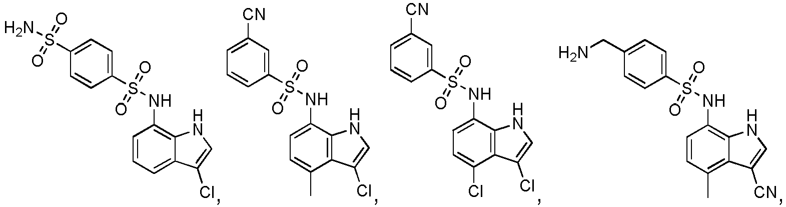

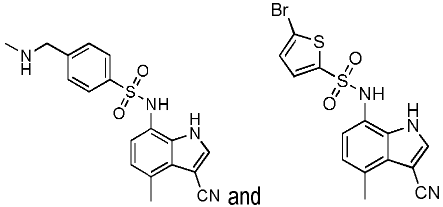

11. The immunoconjugate of any one of claims 1 to 10, wherein the compound of formula (I) is present in the immunoconjugate as a moiety of formula (Ia): 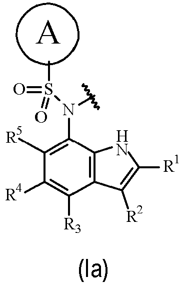
wherein R¹, R², R³, R⁴, R⁵ and A are as defined in any one of claims 1 to 10, preferably wherein X is a bond,
preferably wherein the moiety of formula (Ia) is a moiety of formula (la-1): 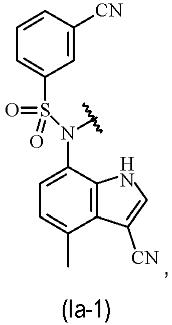 or
wherein the compound of formula (I) is present in the immunoconjugate as a moiety of formula (Ib): 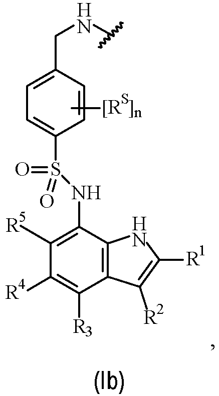
preferably 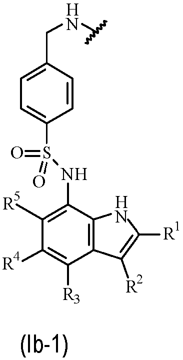
wherein R¹, R², R³, R⁴, and R⁵ are as defined in any one of claims 1 to 10, preferably wherein X is -O-CO-, wherein the right empty valence of said -O-CO- is connected to the moiety of formula (Ib), wherein R^{S} is selected from halogen, C₁₋₅ alkyl, -OH, -O(C₁₋₅ alkyl), -CN, -NO₂, -NH(C₁₋₅ alkyl), -N(C₁₋₅ alkyl)(C₁₋₅ alkyl), -CHO, -COOH, -COO(C₁₋₅ alkyl), -CONH₂, -CONH(C₁₋₅ alkyl), - CON(C₁₋₅ alkyl)(C₁₋₅ alkyl), -SO₂NH₂, -SO₂NH(C₁₋₅ alkyl) and -SO₂N(C₁₋₅ alkyl)(C₁₋₅ alkyl), and wherein n is an integer from 0 to 4,
preferably wherein the moiety of formula (lb) is a moiety of formula (Ib-2):

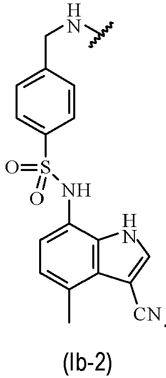

12. A pharmaceutical composition comprising the immunoconjugate of any one of claims 1 to 11, and a pharmaceutically acceptable carrier.

13. The immunoconjugate of any one of claims 1 to 11 or a pharmaceutical composition of claim 12 for use as a medicament.

14. The immunoconjugate of any one of claims 1 to 11 or a pharmaceutical composition of claim 12 for use in the treatment or prevention of cancer,
preferably wherein the cancer is **characterized by** upregulation of RBM39,
more preferably wherein the cancer is selected from neuroblastoma, Ewing sarcoma, osteosarcoma, rhabdomyosarcoma, melanoma, acute myeloid leukemia, breast cancer, lung cancer, ovarian cancer and colorectal cancer,
or
wherein the cancer is neuroblastoma, preferably a pediatric neuroblastoma,
optionally wherein the immunoconjugate or the pharmaceutical composition is to be co-administered with an EZH2 inhibitor (such as GSK126 or tazemetostat) or with an HDAC inhibitor (such as vorinostat, entinostat or panabinostat).

15. A compound of formula:
Z_{Z}-L₁-L_{P}-L₂-X-Y (III)
or a pharmaceutically acceptable salt thereof, wherein
Z_{Z} is Z₁ or Z₂,
Z₁ is selected from 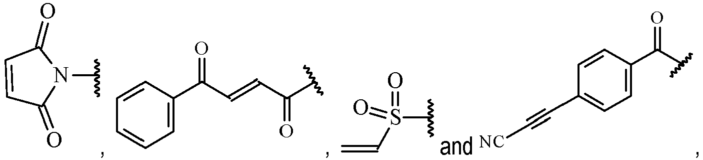
or Z₁ is a group selected from 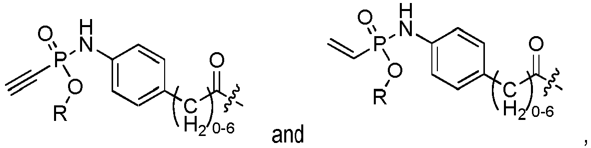
wherein R is -(CH₂CH₂O)₁₋₂₃-CH₂CH₂OH or -(CH₂CH_{2O})₁₋₂₃-CH₂CF₃;
Z₂ is selected from 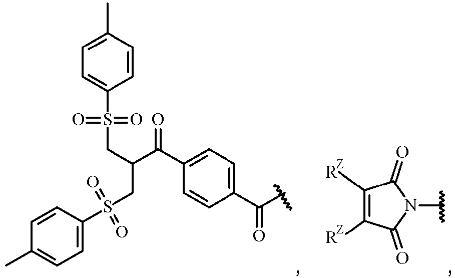
wherein each R^{Z} independently is Hal (such as CI, Br or I) or -S-aryl (such as -S-phenyl), and 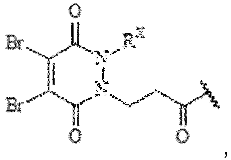
wherein R^{X} is C₁₋₅ alkyl (such as methyl or ethyl), C₂₋₅ alkenyl or C₂₋₅ alkynyl (such as propargyl);
L₁, L_{P}, L₂, and X are as defined in claim 6 or 7; and
Y is the moiety of formula (Ia) or the moiety of formula (Ib) as defined in claim 11.
